# EUROPEAN PATENT APPLICATION

(11) **EP 1 895 008 A2**
(43) Date of publication of application: **05.03.2008**
(21) Application number: 07014821.8
(22) Date of filing: 03.08.2000
(51) Int. Cl.: C12N 15/54, C12N 15/60, C12N 9/12, C12N 9/88, C12P 23/00

(54) **Isoprenoid biosynthesis**

(30) Priority: 04.08.1999 DE 19936663; 21.09.1999 DE 19945175; 21.09.1999 DE 19945174; 11.10.1999 DE 19948887; 05.11.1999 DE 19953309; 28.04.2000 DE 10020996
(62) Divisional of application: 00949452.7
(71) Applicant: Bacher, Adelbert, 85748 Garching (DE); Zenk, Meinhart H., Saint Louis, MO 63141 (US)
(72) Inventor: Bacher, Adelbert, 85748 Garching (DE); Zenk, Meinhart H., St. Louis, MO 63110 (DE); Eisenreich, Wolfgang, 85354 Freising (DE); Fellermeier, Monika, 80639 München (DE); Fischer, Markus, 80337 München (DE); Hecht, Stefan, 83043 Bad Aibling (DE); Herz, Stefan, 80636 München (DE); Kis, Klaus, 8032 Zürich (CH); Lüttgen, Holger, 14558 Nuthetal OT Nudow (DE); Rohdich, Felix, 85406 Zolling (DE); Sagner, Silvia, 80798 München (DE); Schuhr, Chrisoph A., 51065 Köln (DE); Wungsintaweekul, Juraithip c/o Dep.of Pharmacognosy and Pharmaceutical Botany, Songkhla, 90112 (TH)
(74) Representative: Blodig, Wolfgang

(57) **Abstract**

The present invention relates to enzymatic activity involved in isoprenoid biosynthesis as well as to inhibitors, notably herbicides, for enzymes in the biosynthesis of isoprenoids. More specifically, the present invention relates to screening methods for detecting such inhibitors, and to enzymatically active proteins for performing said methods as well as purified-isolated DNA coding for such proteins. Moreover, the present invention relates to novel inhibitors detectable by said screening methods as well as compositions and processes for inhibiting the synthesis of isoprenoids and for controlling the growth of organisms based on said inhibitors. The invention relates also to the development of inhibitor-resistant plant enzymes and plants, plant tissues, plant seeds and plant cells.

## Description

### FIELD OF THE INVENTION

The present invention relates to isoprenoid biosynthesis and notably to genes, enzymes and intermediates involved in isoprenoid biosynthesis as well as to inhibitors, notably herbicides, for enzymes in the biosynthesis of isoprenoids. More specifically, the present invention relates to screening methods for detecting such inhibitors, and to enzymatically active proteins for performing said methods as well as purified isolated DNA coding for such proteins. Moreover, the present invention relates to novel inhibitors detectable by said screening methods as well as compositions and processes for inhibiting the synthesis of isoprenoids and for controlling the growth of organisms based on said inhibitors. The invention relates also to the development of inhibitor-resistant plant enzymes and plants, plant tissues, plant seeds and plant cells.

### BACKGROUND OF THE INVENTION

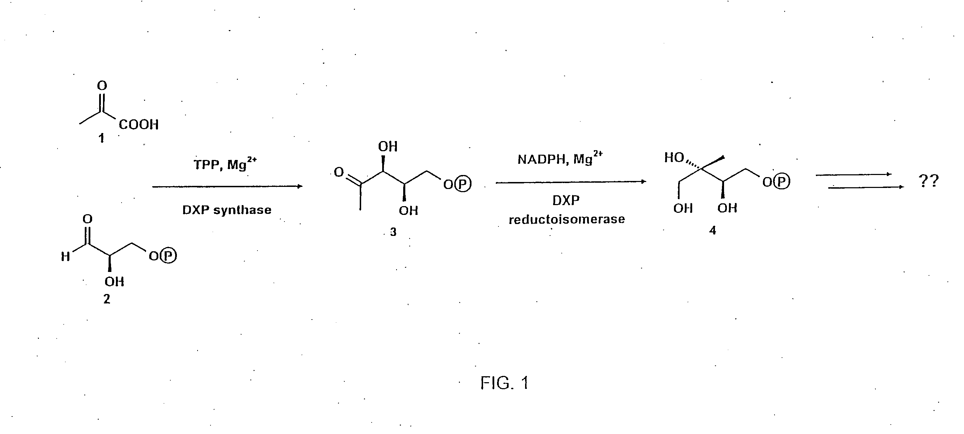

By the classical research of Bloch, Cornforth, Lynen and coworkers, isopentenyl pyrophosphate (IPP) and dimethylallyl pyrophosphate (DMAPP) have become established as key intermediates in the biosynthesis of isoprenoids via mevalonic acid. Bacteria, plants and the protist *Plasmodium falciparum* synthesize isoprenoids by an alternative pathway via 1-deoxy-D-xylulose 5-phosphate. This non-mevalonate pathway has so far only been partially explored (Fig. 1), but its absence in animals makes it an ideal target for pesticidal or medical purposes. Moreover, the idiosyncratic nature of the reactions in this pathway reduces the risk of cross-inhibitions with other, notably mammalian enzymes. For a better understanding of these aspects of the invention, the pathway shall be briefly explained. It begins with a condensation of pyruvate (1) with glycerolaldehyde 3-phosphate (2) to 1-deoxy-D-xylulose 5-phosphate (DXP) (3). Subsequently, DXP is converted to 2C-methyl-D-erythritol 4-phosphate (4) by a two-step reaction comprising a rearrangement and a reduction. The subsequent steps to isoprenoids have so far not been explored, but it may be assumed that the pathway includes intermediates of the type IPP and DMAPP. In any event, this pathway and notably these subsequent enzymatic steps are here determined to be ideal targets in screening chemical libraries for inhibitors.

The non-mevalonate pathway (alternative isoprenoid pathway), with which the present invention is concerned, generates the basic isoprenoid C₅-compounds (IPP and/or DMAPP or an equivalent compound) from which all higher isoprenoids derive by downstream biosynthetic pathways. Higher isoprenoids are called terpenoids. Therefore, any inhibitor of an enzyme in the non-mevalonate pathway is at the same time an inhibitor of all subsequent isoprenoid pathways, i.e. an inhibitor of the terpenoid pathways.

Wherever a phosphorylated compound or a carboxylic acid compound is mentioned it may exist as a free acid or as a salt with at least one proton replaced by ammonium or a metal ion or an organic cation. The metal ion may be an alkali metal ion or an alkaline earth metal ion. The organic cation may be derived from an amine. It may be a sulfonium ion, a guanidinium ion or a heteroaromatic ion. Such a phosphorylated compound, when in an aqueous solution, will exist in an equilibrium dissociation state.

### SUMMARY OF THE INVENTION

It is a first object of the invention to provide functional enzymes which operate in the alternative isoprenoid pathway downstream from 2C-methyl-D-erythritol 4-phosphate. This object has been achieved by the proteins of claims 1 to 13.

It is a further object of the invention to provide purified isolated nucleic acid, notably DNA coding for an enzyme in accordance with one of items (1) to (14) or a vector containing such DNA. This object has been achieved by the subject matter of items (15) to (18).

It is another object of the invention to provide methods for screening a chemical library for inhibitors of an enzyme in the alternative isoprenoid pathway downstream from 2C-methyl-D-erythritol 4-phosphate. This object has been achieved by the screening methods of items (19) to (30).

It is a further object of the invention to provide intermediates in the alternative isoprenoid pathway, which are the products of the enzymes in accordance with claims 1 to 14. This object is achieved by the chemical compounds in accordance with one of items (31), (34), (36), (39) and by the methods of preparation in accordance with one of items (32), (35), (37), (40). It is a further object of the invention to provide a use of said intermediate as a substrate for screening for inhibition of the isoprenoid pathway. This object is achieved in accordance with one of items (33), (38), (41).

It is a further object of the invention to provide a method for identifying inhibitor-resistant variants of an enzyme in the alternative isoprenoid pathway as well as nucleic acids and DNA vectors encoding said variants as well as cells and seeds of plants harboring such vector, as well as a method for conferring inhibitor resistance to plants and a corresponding method of weed control. These objects are achieved in accordance with items (42) to (51).

It is a further object of the invention to provide novel inhibitors of enzymes in the alternative isoprenoid biosynthesis downstream from 2C-methyl-D-erythritol 4-phosphate, compositions of such inhibitors and methods of *in vivo* inhibiting the biosynthesis of isoprenoids. These objects are achieved in accordance with items (52) to (54).

It is a further object of the invention to provide an economical process for efficiently producing the intermediates from readily available starting materials.

This problem is solved by the process of item (55). Preferred embodiments are defined in items (56) to (61). The process can be used for producing the desired product with any desired isotopic labeling.

Thus, the invention provides:
(1) A protein, in a form that is enzymatically functional for the conversion of cytidine triphosphate and 2C-methyl-D-erythritol 4-phosphate into 4-diphosphocytidyl 2C-methyl-D-erythritol in the presence of magnesium ions.
(2) The protein according to (1), wherein it has a sequence selected from the set of sequences coded by *ygbP* in the *E.coli* genome or the set of functional homologs thereof.
(3) A protein in a form that is enzymatically functional for the conversion of 4-diphosphocytidyl-2C-methyl-D-erythritol in the presence of manganese ions.
(4) The protein according to item (3), wherein it has a sequence selected from the set of sequences coded by *ygbB* in the *E.coli* genome or the set of functional homologs thereof.
(5) A protein in a form that is enzymatically functional for the conversion of 4-diphosphocytidyl-2C-methyl-D-erythritol and adenosine triphosphate into 4-diphosphocytidyl-2C-methyl-D-erythritol 2-phosphate in the presence of a magnesium salt.
(6) A protein according to item (5), characterized in that it has a sequence selected from the set of sequences coded by *YchB* in the *E. coli* genome or the set of functional homologs thereof.
(7) A protein in a form that is enzymatically functional for the conversion of 4-disphosphocytidyl-2C-methyl-D-erythritol 2-phosphate into 2C-methyl-D-erythritol 2,4-cyclopyrophosphate and CMP.
(8) The protein according to item (7), characterized in that is has a sequence selected from the set of sequences coded by the gene *YgbB* in the *E.coli* genome or by genes orthologous to *YgbB.*
(9) A protein in a form that is enzymatically bifunctional, having an N-terminal domain with the function of synthesis of diphosphocytidyl-2C-methyl-D-erythritol from cytidine triphosphate and 2C-methyl-D-erythritol 4-phosphate and a C-terminal domain with the function of the conversion of 4-diphosphocytidyl-2C-methyl-D-erythritol.
(10) The protein according to one of items (1) to (8), whereby it is a plant enzyme.
(11) The protein according to item (10) whereby it is an Arabidopsis thaliana enzyme or a Lycopersicum esculentum enzyme.
(12) The protein according to one of items (1) to (9) whereby it is a bacterial enzyme.
(13) The protein according to one of items (1) to (8) whereby it is a protozoal enzyme.
(14) The protein according to one of items (12) or (13), whereby it is a protein of an organism selected from *Escherichia coli, Haemophilus, Bacillus subtilis, Synechocystis, Mycobacterium, Aquifex aeolicus, Chlamydia, Thermotoga maritima, Helicobacter, Treponema pallidum, Borellia burgdorfferi, Salmonella, Yersinia, Actinobacillus, Vibrio cholerae, Shewanella putrefaciens, Pasteurella multocida, Pseudomonas aeruginosa, Neisseria, Bordetella, Thiobacillus ferrooxidans, Deinococcus radiodurans, Clostridium acetobutylicum, Chlorobium tepidum, Porphyromonas gingivalis, Enterococccus faecalis, Streptococcus, Staphylococcus aureus, Rhodobacter capsulatus, Caulobacter crescentus, Campylobacter jejunis, Plasmodium falciparum.*
(15) Purified isolated nucleic acid encoding a protein in accordance with one of items (1) to (14) and optionally comprising introns.
(16) A DNA expression vector comprising the nucleic acid sequence of item (15).
(17) A cell comprising the vector of item (16), wherein said cell is selected from the group consisting of bacterial, protozoal, fungal, plant, insect, and mammalian cells.
(18) A seed comprising a plant cell as defined in item (17).
(19) Use of a protein according to one of items (1) to (14) for screening a chemical library for inhibitors of the biosynthesis of isoprenoids.
(20) A method for screening chemical libraries for the presence or absence of inhibition of the biosynthesis of isoprenoids by blocking the synthesis of 4-diphosphocytidyl-2C-methyl-D-erythritol from cytidine triphosphate and 2C-methyl-D-erythritol 4-phosphate in accordance with the following steps:
   (a) preparing an aqueous mixture comprising an enzyme according to item (1), 2C-methyl-D-erythritol 4-phosphate, or a source therefore, cytidine triphosphate and a divalent metal salt;
   (b) reacting said mixture for a predetermined period of time at a predetermined temperature;
   (c) detecting the level of conversion to 4-diphosphocytidyl-2C-methyl-D-erythritol,
   (d) repeating steps (a) to (c) in the presence of a test sample of a chemical library,
   (e) determining the presence or absence of inhibition in step (d) by ascertaining whether or not the detected level is lower in the presence of the test sample.
(21) The method according to item (20), wherein step (c) is carried out by measuring the level of formation of pyrophosphate or of 4-diphospho-cytidyl-2C-methyl-D-erythritol or by measuring the consumption of cytidyl triphosphate or 2C-methyl-D-erythritol 4-phosphate.
(22) The method according to item (20), wherein the divalent metal salt is a magnesium salt.
(23) A method for screening chemical libraries for the presence or absence of inhibition of the biosynthesis of isoprenoids by blocking the conversion of 4-diphosphocytidyl-2C-methyl-D-erythritol in accordance with the following steps:
   (a) preparing an aqueous mixture comprising an enzyme according to item (3), 4-diphosphocytidyl-2C-methyl-D-erythritol or a source therefore and a divalent metal salt;
   (b) reacting said mixture for a predetermined period of time at a predetermined temperature;
   (c) detecting the level of conversion to 4-diphosphocytidyl-2C-methyl-D-erythritol;
   (d) repeating steps (a) to (c) in the presence of a test sample of a chemical library;
   (e) determining the presence or absence of inhibition in step (d) by ascertaining whether or not the detected level is higher in the presence of the test sample.
(24) The method according to item (23), wherein step (c) is carried out by measuring the level of formation of 2C-methyl-D-erythritol 3,4-cyclophosphate or CMP or by measuring the consumption of 4-diphosphocytidyl-2C-methyl-D-erythritol.
(25) The method according to item (23), wherein the divalent metal salt is a salt of divalent manganese.
(26) A method for screening chemical libraries for the presence or absence of inhibition of the biosynthesis of isoprenoids by blocking the synthesis of 4-diphosphocytidyl-2C-methyl-D-erythritol 2-phosphate from 4-diphosphocytdiyl-2C-methyl-D-erythritol and adenosine triphosphate in accordance with the following steps:
   (a) preparing an aqueous mixture comprising an enzyme according to item (5), 4-diphosphocytidyl-2C-methyl-D-erythritol or a source therefore, aadenosine triphosphate and a divalent metal salt;
   (b) reacting said mixture for a predetermined preriod of time at a predetermined temperature;
   (c) detecting the level of conversion to 4-diphosphocytidyl-2C-methyl-D-erythritol,
   (d) repeating steps (a) to (c) in the presence of a test sample of a chemical library,
   (e) determining the presence or absence of inhibition in step (d) by ascertaining whether or not the in step (d) by ascertaining whether or not the detected level is lower in the presence of the test sample.
(27) The method according to item (26), wherein step (c) is carried out by measuring the level of formation of 4-diphosphocytidyl-2C-methyl-D-erythritol 2-phosphate or of pyrophosphate or by measuring the consumption of 4-diphosphocytidyl-2C-methyl-D-erythritol or of adenosine triphosphate.
(28) The method according to item (26), wherein the divalent metal salt is a magnesium salt.
(29) A method for screening chemical libraries for the presence or absence of inhibition of the biosynthesis of isoprenoids by blocking the synthesis of 2C-methyl-D-erythritol 2,4-cyclopyrophosphate in accordance with the following steps:
   (a) preparing an aqueous mixture comprising an enzyme according to item (7) and 4-diphosphocytidyl-2C-methyl-D-erythritol 2-phosphate or a source therefore
   (b) reacting said mixture for a predetermined period of time at a predetermined temperature;
   (c) detecting the level of conversion to 2C-methyl-D-erythritol 2,4-cyclopyrophosphate;
   (d) repeating steps (a) to (c) in the presence of a test sample of a chemical library,
   (e) determining the presence or absence of inhibition in step (d) by ascertaining whether or not the detected level is lower in the presence of the test sample.
(30) The method according to item (29), wherein step (c) is carried out by measuring the level of formation of 2C-methyl-D-erythritol 2,4-cyclopyrophosphate or of cytidyl monophosphate or by measuring the level of consumption of 4-diphosphocytidyl-2C-methyl-D-erythritol 2-phosphate.
(31) Optionally isotope-labelled 4-diphosphocytidyl-2C-methyl-D-erythritol, or a salt thereof.
(32) A process for forming 4-diphosphocytidyl-2C-methyl-D-erythritol or a salt thereof by reacting cytidine triphosphate and 2C-methyl-D-erythritol 4-phosphate in the presence of a protein according to items (1) or (2) and a divalent metal salt.
(33) Use of 4-diphosphocytidyl-2C-methyl-D-erythritol or a salt thereof for screening for inhibitors of the biosynthesis of isoprenoids.
(34) Optionally isotope-labelled 2C-methyl-D-erythritol 3,4-cyclophosphate or a salt thereof.
(35) A process for forming 2C-methyl-D-erythritol 3,4-cyclophosphate or a salt thereof by reacting 4-diphosphocytidine-2C-methyl-D-erythritol in the presence of a protein according to item (4) and of a divalent metal salt.
(36) Optionally isotope-labelled 4-diphosphocytidyl-2C-methyl-D-erythritol 2-phosphate or a salt thereof.
(37) A process for forming 4-diphosphocytidyl-2C-methyl-D-erythritol 2-phosphate or a salt thereof by reacting 4-diphosphocytidyl-2C-methyl-D-erythritol with adenosine triphosphate in the presence of a protein according to items (5) or (6) and a divalent metal salt.
(38) Use of 4-diphosphocytidyl-2C-methyl-D-erythritol 2-phosphate or a salt thereof for screening for inhibitors of the biosynthesis of isoprenoids.
(39) Optionally isotope-labelled 2C-methyl-D-erythritol 2,4-cyclopyrophosphate or a salt thereof.
(40) A process for forming 2C-methyl-D-erythritol 2,4-cyclopyrophosphate or a salt thereof by reacting 4-diphosphocytidyl-2C-methyl-D-erythritol 2-phosphate with a protein according to one of items (7) or (8).
(41) Use of 2C-methyl-D-erythritol 2,4-cyclopyrophosphate or a salt thereof for screening for inhibitors of the biosynthesis of isoprenoids.
(42) A method for identifying inhibitor-resistant variants of the plant proteins of items (10) or (11) said method comprising:
   (a) providing a population of cells expressing one of said plant proteins;
   (b) mutagenizing said population of cells;
   (c) contacting said mutagenized population of cells with an herbicide, under conditions inhibitory for the functional effectiveness of one of said proteins of non-mutagenized cells;
   (d) recovering cells resistant to the inhibitory effects of said herbicide and
   (e) isolating and optionally sequencing nucleic acid encoding herbicide-resistant proteins from said recovered cells to provide and identify herbicide-resistant protein variants.
(43) A variant of protein of one of items (1) to (8), wherein said protein is herbicide-resistant.
(44) The variant protein as defined in item (43), wherein said variant protein, when expressed in a cell that requires the functional activity of said protein for viability, exhibits
   (i) catalytic activity alone sufficient to maintain the viability of a cell in which it is expressed; or catalytic activity in combination with any herbicide-resistant variant protein also expressed in the cell, which may be the same as or different than the first variant protein, sufficient to maintain the viability of a cell in which it is expressed; and
   (ii) catalytic activity that is more resistant to the herbicide than is wild type protein.
(45) The variant protein as defined in item (43), wherein said protein is derived from *Arabidopsis thaliana.*
(46) A nucleic acid encoding a variant protein as defined in item (43).
(47) A DNA vector comprising a nucleic acid as defined in item (46).
(48) A cell comprising DNA vector as defined in item (47), wherein said cell is selected from the group consisting of bacterial, fungal, plant, insect, and mammalian cells.
(49) A seed comprising a plant cell as defined in item (48).
(50) A method for conferring herbicide-resistance on a plant, said method comprising introducing into said plant a nucleic acid encoding an herbicide-resistant protein variant as defined in item (43) under conditions in which said nucleic acid is expressed in said plant.
(51) A method for weed control comprising cultivating a crop containing herbicide-resistant gen having a nucleic acid as defined in item (46) in the presence of a week-controlling effective amount of said herbicide.
(52) Inhibitor for the biosynthesis of isoprenoids selected from the group of chemical compounds which show enzyme inhibition in accordance with the screening method according to one of items (20), (23), (26) or (29).
(53) Composition for the inhibition of the biosynthesis of isoprenoids in plants, bacteria or protozoa comprising a chemical compound showing inhibition in the screening method according to one of items (20), (23), (26) or (29). in an amount suitable for inhibition.
(54) A method for the inhibition of the biosynthesis of isoprenoids in plants, bacteria or protozoa by treatment with an inhibitor according to item (52) or a composition in accordance with item (53) in an amount suitable for inhibition.
(55) A comprehensive process for preparing intermediates in the isoprenoid pathway downstream from 2C-methyl-D-erythritol 4-phosphate characterized by the following steps:
   (a) reacting dihydroxyacetone phosphate and sodium pyruvate in the presence of a magnesium salt, thiamine pyrophosphate, triose phosphate isomerase and 1-deoxy-D-xylulose 5-phosphate synthase to produce 1-deoxy-D-xylulose 5-phosphate;
   (b) reacting the reaction mixture obtained in step (a) with glucose and NADP⁺ in the presence of an Mg²⁺ or Mn²⁺ salt, glucose dehydrogenase and 1-deoxy-D-xylulose 5-phosphate reductoisomerase to produce 2C-methyl-D-erythritol 4-phosphate;
   (c) reacting the reaction mixture obtained in step (b) with cytidyl triphosphate, the protein of one of items (1) or (2) and a divalent metal salt to produce 4-diphosphocytidyl-2C-methyl-D-erythritol;
   (d) optionally reacting the reaction mixture obtained in step (c) with adenosine triphosphate, a divalent metal salt and the protein of one of items (5) or (6) to produce 4-diphosphocytidyl-2C-methyl-D-erythritol 2-phosphate;
   (e) optionally reacting the reaction mixture obtained in step (d) with the protein of one of items (7) or (8) to produce 2C-methyl-D-erythritol 2,4-cyclopyrophosphate;
   (f) isolating the product of step (c) or step (d) or step (e).
(56) The process according to item (55), wherein steps (a) to (e) are carried out as a one-pot reaction.
(57) The process according to items (55) or (56) wherein the reactions are carried out at a pH in the range of 7 to 9.
(58) The process according to items (55) to (57) wherein the reactions are carried out at a temperature of 30 to 45 °C.
(59) The process according to items (55) to (57) wherein an excess of NADP⁺ is used and glucose and glucose dehydrogenase are eliminated in step (b).
(60) The process according to items (55) to (57) wherein the reactions are carried out in a Tris HCl buffer.
(61) The process according to any one of items (55) to (60), wherein glucose in conjunction with ATP and the glycolytic enzymes hexokinase, phosphoglucose isomerase, phosphofructokinase, aldolase and triosephosphate isomerase is used instead of dihydroxyacetone phosphate.
(62) The process according to any one of items (55) to (61), wherein phos phoenolpyruvate and pyruvate kinase are added in step (d) of item (55) for t h e regeneration of ATP.

With the findings of the invention an overall pattern of the non-mevalonate alternative isoprenoid pathway emerges which consists of three segments. The first (previously known) segment up to 2C-methyl-D-erythritol 4-phosphate concerns the formation of the isoprenoid carbon skeleton. The present invention is concerned with the second segment in which all three steps are phosphorylation steps to establish the form of activation as 2,4-cyclopyrophosphate that is required for the subsequent segment. This establishes the functional coherence and unity of the invention. The third segment concerns unknown reductive and eliminative steps for the formation of IPP, DMAPP or equivalent compounds.

### BRIEF DESCRIPTION OF THE DRAWINGS AND ANNEXES

Fig. 1 shows the previously established non-mevalonate pathway.
Fig. 2 shows the enzymatic steps, due to YgbP and YchB, downstream from the previously established non-mevalonate pathway of Fig. 1.
Fig. 3 shows a third enzymatic step, due to YgbB, downstream from the pathway of Fig. 2.

Annex A shows an alignment of YgbP and YgbB of various organisms.

Annex B shows an alignment of YchB of various organisms.

Annex C shows an alignment of the amino acid sequence of the cloned cDNA of *ygbP* of *A. thaliana* (without leader sequence) and the amino acid sequence of the YgbP gene product found in the database (see Table 1).

Annex Da shows the cDNA sequence and the corresponding amino acid sequence of *ygbP* of *A.thaliana* (without leader sequence).

Annex Db shows the cDNA sequence and the corresponding amino acid sequence of *ygbP* of *A.thaliana* (with leader sequence).

Annex Ea shows the DNA sequence and corresponding amino acid sequence of *ychB* of *A. thaliana* (without leader sequence).

Annex Eb shows the cDNA sequence and corresponding amino acid sequence of *ychB* of *A. thaliana* (with leader sequence).

Annex Ec shows an alignment of the wild type nucleotide sequence of *ychB* of *L. esculentum* (without leader sequence) and of a nucleotide sequence of *ychB* of *L. esculentum* adapted to E.coli codon usage for highly expressed genes (without leader sequence).

Annex F1 shows the nucleotide sequence of the plasmid pNCO113.

Annex F2 shows the nucleotide sequence of the plasmid pNCO-SB-H6-ACYC 184.

Annex G shows the cDNA sequence and corresponding amino acid sequence of *ygbB* of *P.falciparum.*

### DETAILED DESCRIPTION OF THE INVENTION

### The Bisosynthetic Pathway

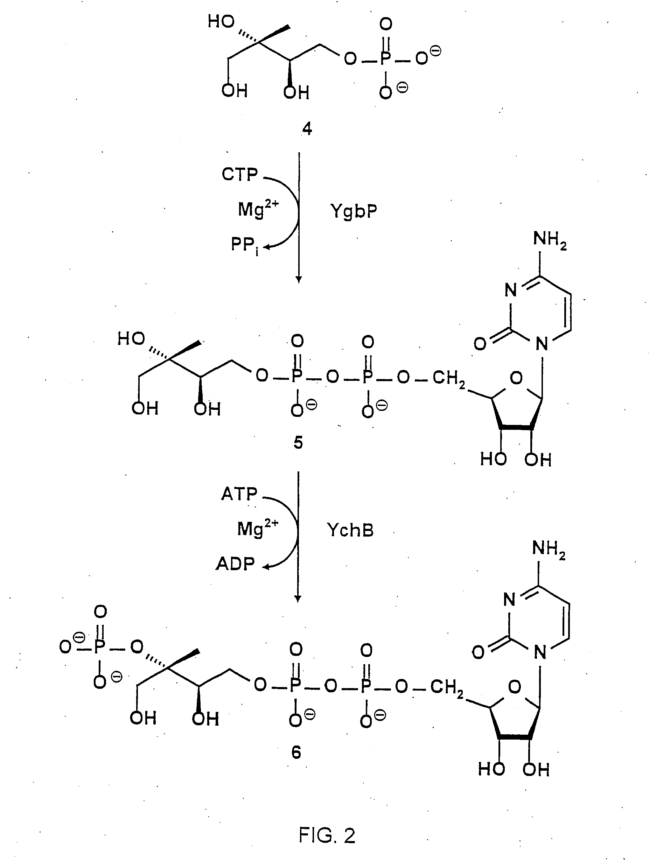

We have surprisingly discovered that the next intermediate downstream from 2C-methyl-D-erythritol 4-phosphate is 4-diphosphocytidyl-2C-methyl-D-erythritol (5) (Fig. 2). Based on this finding, we have established that this intermediate is biosynthesized from 2C-methyl-D-erythritol 4-phosphate with CTP, and we have also produced an *E. coli* protein in an enzymatically functional form for this conversion. This enzyme is called 4-diphosphocytidyl-2C-methyl-D-erythritol synthase. The amino acid sequence of this enzyme and the corresponding DNA sequence were contained as unannotated open reading frame in the genome of *E. coli* under the designation *ygbP* (accession number gb AE000358). The DNA corresponding to this ORF was isolated and cloned into a high copy expression vector and with this vector construct *E.coli* cells were transformed. Surprisingly in 12 tested recombinant *E. coli* clones the corresponding gene product was expressed in soluble form to a level of about 10 % of the total soluble cell fraction. This was judged by SDS-PAGE. Moreover, cell extracts of 4 tested recombinant clones showed activity according to the formation of 4-diphosphocytidyl-2C-methyl-D-erythritol from CTP and 2C-methyl-D-erythritol 4-phosphate. This specific activity was at least 100 times higher than in cell extracts of *E. coli* wild type cells.

We further have established the presence of sequences highly homologous to *ygbP* in a number of bacteria, in *Arabidopsis thaliana* as well as in *Plasmodium falciparum* by performing a blast search in GenBank as well as in the database of completed and uncompleted genomes. We have thus opened an avenue for the expression of functional forms of the protein YgbP in any of these or other organisms. Notably, cDNA of the corresponding open reading frame (ORF) of *Arabidopsis thaliana* (see Table 1) was isolated and cloned into a high copy expression vector and the corresponding gene product was expressed heterologously in *E*. *coli* in enzymatically activ form.

We further have established that in bacteria, *ygbP* is usually contained in an operon in which it is closely followed by an open reading frame (designated *ygbB* in *E. coli)* with a narrow gap or even an overlap. We further have established that these proteins YgbP and YgbB are fused in some bacteria to form a bifunctional enzyme. In agreement with these findings, we determined that YgbB is an enzyme active in the alternative biosynthesis of terpenoids downstream from YgbP and that it converts 4-diphosphocytidyl-2C-methyl-D-erythritol. The DNA corresponding to the ORF *ygbB* in the genome of *E. coli* was isolated and cloned into a high copy expression vector and the corresponding gene product was expressed in *E. coli* in enzymatically activ form.

The designations of YgbP and YgbB in the *E. coli* genome are used herein also for the homologous enzymes in other organisms. A set of open reading frames homologous to *ygbB* and *ygbP* is listed in Table 1. An alignment of the amino acid sequences of the corresponding gene products is shown in Annex A. The alignment was constructed with the Pileup program using the default setting (Genetic Computer Group, Madison, Wisconsin) and it was edited with the GeneDoc program (http://www.com/ketchup/genedoc.shtml). Organism names are abbreviated at the left side of the alignment as shown in Table 1. Amino acid residues are written in IUPAC one letter code and are numbered consecutively for reference purposes at the top of each alignment page. The YgbP functional domain extends from residue 1 to residue about 360 and the YgbB functional domain extends from residue about 360 to the end. Each accumulated total number of amino acids is given in each line in the right column of the alignment. Gaps in the alignment are symbolized by a dash (-). The symbols < and > indicate a fragment. The symbol \\ denotes a C-terminus. The symbol * denotes a stop codon due to a frame shift. In the case of a fragment, the sequences are missing at the beginning and/or end due to the fact that they are ignored by the Blast program for retrieving the sequences from the database of unfinished genomes. They are readily obtained from the corresponding nucleotide sequence, proceeding forward until the initiator codon ATG (or GTG or CTG) is encountered and backward until a stop codon is encountered.

**Table 1**

| Occurence of orthologous sequences to *ygbP* and *ygbB* in various organisms | | | |
|---|---|---|---|
| organism | | accession or Contig number*^{a}*, | basenpairs |
| | abbreviation*^{b}* | corresponding *ygbP* | corresponding *ygbB* |
| *Escherichia* coli K-*12* MG1655 | E.C. | gb AE000358, 6754-7464 | gb AE000358, 6278-6754 |
| *Haemophilus influenzae* Rd | H.I. | gb U32750, 2072-2749 | gb U32750, 1599-2075 |
| *Bacillus subtilis 168* | B.S. | emb Z99101, 109786- | emb Z99101, 110477- |
| | | 110484 | 110953 |
| *Synchocystis* sp. PCC6803 | S.S. | dbj D990914, 29703-30395 | dbj D90906, 58770-59255 |
| *Mycobacterium tuberculosis* H37Rv | M.T. | emb Z92774, 29591-30286 | emb Z92774, 29115-29594 |
| *Aquifex aeolicus* VF5 | A.E. | gb AE000734, 10078- | gb AE000715, 8239-8709 |
| | | 10719 | |
| *Chlamydia trachomatis* D/UW-3/CX | C.T. | gb AE001320, 3348-4007 | gb AE001317, 988-1524 |
| *Chlamydia pneumoniae* CWL029 | C.P. | gb AE001642, 7155-7790 | gb AE001639, 4613-5143 |
| *Thermotoga maritima* | T.M. | gb AE001792, 3951-4619 | gb AE001738, 11427- |
| | | | 11924 |
| *Pyrococcus horikoshii* OT3 | P.H. | dbj AP000002, 72589- | __*^{c}* |
| | | 73278 | |
| *Helicobacter pylori* strain J99 | H.P. | gb AE001474, 6197-7426*^{d}* | |
| *Treponema pallidum* | T.P. | gb AE001227, 7127-8326*^{d}* | |
| *Haemophilus ducreyi 35000* | H.D. | n. s.*^{e}* | gb U32175, 5939-6421 |
| *Salmonella typhi* | S.T. | Contig404, 129036-129737 | Contig404, 129746-130195 |
| *Yersinia pestis* | Y.S. | Contig730, 87298-87966 | Contig730, 88135-88605 |
| *Actinobacillus actinomycetemcomitans* | A.A. | Contig704, 2247-2927 | Contig704, 1771-2238 |
| *Vibrio cholerae* | V.C. | asm938, 1967-2641 | asm938, 2656-3129 |
| *Shewanella putrefaciens* | S.P. | gsp_845, 694-1359 | gsp_845, 183-629 |
| *Pasteurella multocida* PM70 | P.M. | Contig556, 1199-1876 | Contig556, 1891-2364 |
| *Pseudomonas aeruginosa* | P.A. | Contig52, 647221-647862 | Contig52, 652597-653061 |
| *Neisseria gonorrhoeae* | N.G. | Contig 181, 9088-9747 | Contig181, 9791-10267 |
| *Bordetella pertussis* | B.P. | Contig657, 1198-1860 | Contig657, 1860-2342 |
| *Neisseria meningitidis* MC 58 | N.M.MC | n.s. | GNMCP32F, 134-550 |
| *Neisseria meningitidis* serogroup A | N.M.SA | Contig3, 255019-255678 | Contig3, 254511-254963 |
| *Thiobacillus ferrooxidans* | T.F. | 949, 9-599 | 949, 646-1107 |
| *Deinococcus radiodurans* | D.R. | 8896, 4825-5430 | 8835, 20460-20888 |
| *Clostridium acetobutylicum* | C.A. | AE001437, 2979884- | AE001437, 160798- |
| *Mycobacterium avium* | M.A. | 2980552 5759, 2065-2721 | 161262 5759, 1598-2050 |
| *Mycobacterium bovis* | M.B. | Contig950, 5497-6156 | Contig950, 6186-6611 |
| *Chlorobium tepidum* | C.T. | gct_38, 2977-3687 | gct_41, 1403-1873 |
| *Porphyromonas gingivalis* W83 | P.G. | 1209, 31380-32024 | 1207, 93755-94237 |
| *Enterococcus faecalis* | E.F. | gef_6311, 4277-4960 | gef_6177, 5831-6301 |
| *Streptococcus pneumoniae* | S.Pn. | sp_72, 23704-24387 | n.s. |
| *Staphyloccoccus aureus* COL | S.T. | 2204, 9831-10517 | n.s. |
| *Plasmodium falciparum* 3D7 | P.F. | 1D_M9Fe7.p1 t, 22-202 | gb AE001394, 2617-3495 |
| *Arabidopsis thaliana* chromosome II | A.T. | gb AC004136, 79845- | gb AC010852, 376-1789 |
| BAC | | 81915 | |
| *Rhodobacter capsulatus* SB1003 | R.C. | emb X72382, 279-1418*^{d}* | |
| *Caulobacter crescentus* | C.C. | gcc_1641, 677-1243, gcc_! 574, | 1870-2430*^{d}* |
| *Campylobacter jejuni* NCTC 11168 | C.J. | Cj.seq, 1534779-1535867*^{d}* | |

| | | | |
|---|---|---|---|
| *^{a}*http://www.ncbi.nlm.nih.gov and The Institute for Genomic Research website http://www.tigr.org *^{b}*abbreviation in alignment (Annex A) *^{c}*no homologous sequence existent in the database of complete sequenced genomes *^{d}*bifunctional YgbP/YgbB gene product *^{e}*not sequenced | | | |

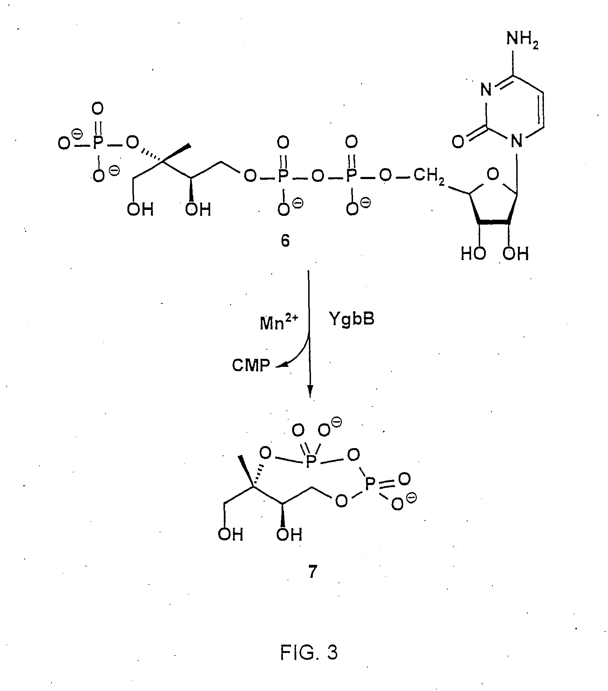

We have further surprisingly discovered that the next intermediates in the biosynthesis pathway downstream from 4-diphosphocytidyl-2C-methyl-D-erythritol are 4-diphosphocytidyl-2C-methyl-D-erythritol 2-phosphate (6) (Fig. 2) and 2C-methyl-D-erythritol 2,4-cyclopyrophosphate (7) (Fig. 3). Based on this finding, we have established that 4-diphosphocytidyl-2C-methyl-D-erythritol 2-phosphate is biosynthesized from 4-diphosphocytidyl-2C-methyl-D-erythritol with ATP, and we have also produced an *E. coli* protein (YchB) in an enzymatically functional form for this conversion. This enzyme is called 4-diphosphocytidyl-2C-methyl-D-erythritol kinase. We also found that the above-mentioned YgbB protein converts 4-diphosphocytidyl-2C-methyl-D-erythritol 2-phosphate into 2C-methyl-D-erythritol 2,4-cyclopyrophosphate and CMP (7) (FIG. 3).

The amino acid sequence of the YchB enzyme and the corresponding DNA sequence are contained as unannotated open reading frame in the genome of *E. coli* under the designation *ychB* (gb accession number AE000219). The DNA corresponding to this ORF was isolated and cloned into a high copy expression vector and with this vector construct *E*. *coli* cells were transformed. Surprisingly in 6 tested recombinant *E. coli* clones the corresponding gene product was expressed in soluble form to a level of about 10 % of the total soluble cell fraction. This was judged by SDS-PAGE. Moreover, cell extracts of 4 tested recombinant clones showed activity according to the formation of 4-diphosphocytidyl-2C-methyl-D-erythritol 2-phosphate from ATP and 4-diphosphocytidyl-2C-methyl-D-erythritol. This specific activity was at least 100 times higher than in cell extracts of *E. coli* wild type cells.

We further have established the presence of sequences highly homologous to *ychB* in a number of bacteria, in *Arabidopsis thaliana* as well as in *Lycopersicon esculentum* (tomato) by performing a blast search in GenBank as well as in the database of completed and uncompleted genomes. The orthologous cDNA pTOM41 sequence of tomato (gb accession no. U62773) was previously described erroneously as ripening associated transcription product.

We have thus opened an avenue for the expression of functional forms of the protein YchB in any of these or other organisms. Notably, the DNA corresponding to the ORF of *Arabidopsis thaliana* (gb accession no. AC005168) was isolated and cloned into a high copy expression vector.

The designations of YchB in the *E. coli* genome are used herein also for the orthologous enzymes in other organisms. The set of open reading frames homologous to *ychB* is listed in Table 2. An alignment of the amino acid sequences of the corresponding gene products is shown in Annex B. The alignment was constructed with the Pileup program using the default setting (Genetic Computer Group, Madison, Wisconsin) and it was edited with the GeneDoc program (http://www.comrketchup/genedoc.shtml). Organism names are abbreviated at the left side of the alignment as shown in Table 2. Amino acid residues are written in IUPAC one letter code and are numbered consecutively for reference purposes at the top of each alignment page. Each accumulated total number of amino acids is given in each line in the right column of the alignment. Gaps in the alignment are symbolized by a dash (-). The symbols < and > indicate a fragment. The symbol \\ denotes a C-terminus. The symbol * denotes a stop codon due to a frame shift.

In the case of a fragment, the sequences are missing at the beginning and/or end due to the fact that they are ignored by the Blast program for retrieving the sequences from the database of unfinished genomes. They are readily obtained from the corresponding nucleotide sequence, proceeding forward until the initiator codon ATG (or GTG or CTG) is encountered and backward until a stop codon is encountered.

**Table 2.**

| Occurrence of orthologous sequences to *ychB* in various organisms | | |
|---|---|---|
| organism | accession or Contig number*^{a}*, basepairs | |
| | abbreviation*^{b}* | corresponding *to ychB* |
| *Escherichia* coli K-12 MG1655 | E.C. | gb AE000219, 5720-6571 |
| *Haemophilus influenzae* Rd KW20 | H.I. | gb U32834, 7469-8410 |
| *Bacillus subtilis* 168 | B.S. | emb Z99104, 53514-54383 |
| *Synchocystis* sp. PCC6803 | S.S. | dbj D90899, 101884-102831 |
| *Mycobacterium tuberculosis* H37Rv | M.T. | emb Z94752, 23889-24809 |
| *Aquifex aeolicus* VF5 | A.E. | gb AE000713, 10428-11234 |
| *Chlamydia trachomatis* D/UW-3/CX | C.T. | gb AE001352, 9579-10445 |
| *Chlamydia pneumoniae* CWL029 | C.P. | gb AE001675, 2514-3406 |
| *Thermotoga maritima* | T.M. | gb AE001791.1, 13364-14179 |
| *Helicobacter pylori* strain J99 | H.P. | gb AE000644,8749-9555 |
| *Treponema pallidum* | T.P. | gb AE001226, 5348-6223 |
| *Samonella typhimurium* LT2 | S.TL. | gb M77236, 941-1792 |
| *Zymomonas mobilis* | Z.M. | gb AF088896.1, 14821-15606 |
| *Salmonella typhi* | S.T. | Contig334, 29152-30000 |
| *Salmonella paratyphi* | S.P. | SPA.0.2446, 209-1054 |
| *Yersinia pestis* | Y.S. | Contig648, 22578-23399 |
| *Actinobacillus actinomycetemcomitans* | A.A. | Contig510, 1486-2304 |
| *Vibrio cholerae* | V.C. | 666_1752, 2349936-2350756 |
| *Shewanella putrefaciens* | S.PU. | 4279, 66514-67320 |
| *Pasteurella multocida* PM70 | P.M. | Contig264, 2636-3454 |
| *Pseudomonas aeruginosa* | P.A. | Contig54, 3100316-3099489 |
| *Neisseria gonorrhoeae* | N.G. | Contig121, 24491-25306 |
| *Bordetella pertussis* | B.P. | Contig408, 16613-17356 |
| *Neisseria meningitidis* serogroup A | N.M. | NM.seq, 1040554-1041369 |
| *Klebsiella pneumoniae* | K.M. | Contig31, 604-1012 |
| *Thiobacillus ferrooxidans* | T.F. | 2031, 1218-1499 |
| *Deinococcus radiodurans* | D.R. | 8896, 5523-6026 |
| *Bordelella bronchiseptica* | B.B. | Contig2244, 31-691 |
| *Clostridium difficile* | C.D. | Contig1239, 9891-10664 |
| *Clostridium acetobutylicum* | C.A. | AE001437, 2693910-1694725 |
| *Mycobacterium avium* | M.A. | M.avium_24, 20876-21331 |
| *Mycobacterium bovis* | M.B. | Contig750, 2661-3116 |
| *Mycobacterium leprae* | M.L. | Contig1080, 6925-7380 |
| *Chlorobium tepidum* | C.TP. | gct_5, 4007-4783 |
| *Porphyromonas gingivalis* W83 | P.G. | 1194, 108718-109167 |
| *Enterococcus faecalis* | E.F. | gef 6342, 2564-3340 |
| *Streptococcus mutans* | S.M. | Contig435, 4960-5739 |
| *Streptococcus pyogenes* | S.PG. | Contig7, 1467238-1457675 |
| *Staphyloccoccus aureus* NCTC8325 | S.AN. | Contig856, 566-1342 |
| *Staphyloccoccus aureus* COL | S.A. | 4357, 3-779 |
| *Arabidopsis thaliana* chromosome II BAC F12620 | A.T. | gb AC005168, 10297-12697 |
| *Solanum lycopersicum* | S.L. | gb U62773, 78-1283 |
| *Sinrhizobium meliloti* | S.ML. | 423II4AI2.xl, 70-627, 423051H04.x1, 360- |
| *Caulobacter crescentus* | C.C. | 677 gcc_1346, 1215-1655 |
| *Campylobacter jejuni* NCTC 11168 | C.J. | Cj.seq, 1038338-1038884 |

With these functional assignments of YgbP, YchB and YgbB and with the production of proteins having enzymatically competent folding structures, we have entered these proteins as well as the purified isolated DNA coding for these proteins as novel compounds for technical and commercial usage into the body of technical knowledge. At the same time this provides avenues for the production of the products of the enzymatic reaction of YgbP, YchB and YgbB, namely 4-diphosphocytidyl-2C-methyl-D-erythritol, 4-diphosphocytidyl-2C-methyl-D-erythritol 2-phosphate and 2C-methyl-D-erythritol 2,4-cyclopyrophosphate or salts thereof, notably salts of alkali metals, like Li, Na, K or of ammonia or amines and for their use in various *in vitro* or *in vivo* reactions of the alternative pathway to isoprenoids.

Based on this achievement, we have opened new avenues for the inhibition of the alternative isoprenoid pathway in plants as well as bacteria and also in protozoa, like Plasmodium.

The enzymes YgbP and YgbB as well as YchB or major core fragments thereof of various organisms have been aligned as shown in Annex A and Annex B. Based on these alignments a broad but finite class of sequences can be defined of enzymes with the functions of YgbP, YgbB and YchB based on the amino acid variability given for each position by the alignments. For any protein in each class, a set of possible equivalent amino acids of any position can be taken immediately, unambiguously and individually from Annex A and Annex B. This will insure sufficient functional competence with a very high likelihood.

Alternatively an orthologous sequence class is established for each gene by nucleic acid hybridization under conditions of intermediate stringency (such as an aqueous solution of 2X SSC at 65°C) using cDNA or genome DNA or RNA.

We have found that in plants, notably *Arabidopsis thaliana* the enzymes homologous to YgbP, YchB and YgbB have a leader peptide not present in the bacterial enzymes. This leader peptide serves the purpose of transport of the enzyme into the plastids. Such specific leader sequence may be replaced by any other leader sequence from *A. thaliana* or from any other plant or it may also be eliminated.

The *A. thaliana* sequence of YgbP, identified in Table 1 and given in the alignment of Annex A has been obtained by genome sequencing. We have sequenced the gene *ygbP* of *A. thaliana* by isolating RNA from cells of *A. thaliana,* producing cDNAs complementary to said RNA by RT-PCR, subsequently amplifying the coding region for YgbP with the appropriate gene-specific primers by PCR and finally cloning the obtained DNA. The leader sequence was first not cloned and sequenced. But later the full-length gene was cloned from RNA.

The cDNA sequence of the cloned *ygbP* gene from *A. thaliana* was different from the DNA sequence found in the database (gb AC004136) due to introns. The amino acid sequence corresponding to this cDNA is also different from the amino acid sequence given in the database (gb AC004136). This seems due to erroneous computational intron splicing from chromosomal DNA. This finding is shown in an alignment of the amino acid sequence of the cloned cDNA and of the amino acid sequence of the YgbP gene product found in the database (Annex C). The amino acid sequences are numbered at the right side of the alignment. Number 1: cloned sequence of *ygbP* from *A. thaliana* without leader sequence; number 2: gb AC004136. Identical residues are boxed. The cDNA sequence and the corresponding protein sequence of *ygbP* of *A. thaliana* are shown in Annex Da. The cDNA leader sequence was found to be identical to the database prediction (Annex Db).

The genes *ygbP, ygbB* and *ychB* of *E. coli* were obtained by PCR using primers with specific restriction sites. In this PCR reaction two recognition sites for restriction enzymes are introduced at the 5'-end and at the 3' end. The prefered recognition site is *Nco*I or *Eco*RI at the 5'-end and *Pstl* at the 3' end. The amplified PCR fragment and an expression vector are digested with the same restriction enzymes and ligated together with T4-ligase to yield recombinant plasmid capable of autonomous replication in the host microorganism. The recombinant plasmid is used to transform the host microorganism. The preferred host is *E. coli.* The same method was used for the genes *ygbP, ygbB* and *ychB* of *Arabidopsis thaliana* and for *ychB* of tomato whereby the nucleotide sequence was modified for the codon usage of E.coli for highly expressed genes (without leader sequence).

The open reading frame of the *ygbB* gene from *E. coli* was cloned also into the high-copy expression vector pQE30 from Qiagen (Hilden, Germany). This vector provides the high-level expression in *E. coli,* of proteins containing a tag of 6 histidin residues at the N-terminal end. The recombinant "6-His-protein" could then easily be purified in one step to homogeneity by immobilized metal chelate affinity chromatography.

The cloning of the ygbB gene of E. coli into the pQE30 vector led to the expression of soluble, enzymatically active YgbB gene product, which was N-terminal His-tagged. Recombinant E. coli cells containing the overexpressed His-tagged fusion protein showed a specific activity, notably in converting 4-diphosphocytidyl-2C-methyl-D-erythritol, which was at least 80 times higher than in *E. coli* wild-type XL1-Blue cells. This enzyme was purified according to Ni^{2 +} chelate affinity chromatography to homogeneity. The purity of the enzyme was judged by sodium dodecylsulfate polyacrylamide gel electrophoresis.

The same method was used for *ygbB* of Plasmodium falciparum to obtain the fusion protein 6xHis-YgbB.

The corresponding protein sequence of the cloned *ychB* gene from *A. thaliana* was identical to the protein sequence of the computational cDNA sequence found in the database (gb AC005168). The DNA sequence and corresponding protein sequence is shown in Annex Ea without leader sequence. The full-length YchB gene of *Arabidopsis thaliana* has been additionally cloned from RNA. The sequence is shown in Annex Eb.

The strains harbouring the recombinant plasmids can be cultivated in conventional culture media at 15 to 40 °C. The preferred temperature is 37 °C. The E. coli strains are induced with 0.5 to 2 mM isopropyl-β-D-thiogalactoside at an opitical density at 600 nm from 0.5 to 0.8. The cells are incubated between 2 and 12 h, preferably 5 h. The cells are lysed with lysozyme and disrupted with a sonifier. The crude extract with recombinant YgbP, YgbB or YchB protein is purified by chromatography, notably anion exchange chromatography and affinity chromatography. Proteins are obtained which have the proper folding structure for exhibiting the desired enzyme activity, notably from E.coli, A.thaliana, L.esculentum and P.falciparum.

### Screening

The enzymes YgbP, YgbB and YchB do not occur in animals. Therefore, inhibitors against YgbP, YgbB and YchB have great value as (a) herbicides against weed plants or algae; (b) antibiotic agents against pathogenic bacteria; (c) agents against protozoa, like *Plasmodium falciparum,* the causative pathogen of malaria.

With the finding that 4-diphosphocytidyl-2C-methyl-D-erythritol, 4-diphosphocytidyl-2C-methyl-D-erythritol 2-phosphate and 2C-methyl-D-erythritol 2,4-cyclopyrophosphate are intermediates we have also acquired essential determinants of the structures of inhibitors. Namely, the structures of a subset of inhibitors should be similar to at least a portion of the starting compounds or the products or the transition state between the starting compounds e.g. (2C-methyl-D-erythritol 4-phosphate and CTP) and the products e.g. (pyrophosphate and 4-disphosphocytidyl-2C-methyl-D-erythritol). Based on these determinants ribitol 5-phosphate and erythritol 4-phosphate have been synthesized as putative inhibitors for YgbP.

We have also provided methods of screening for inhibitors of YgbP, YgbB and YchB. In these methods one of the enzymes YgbP, YgbB or YchB of the classes of enzymes defined above can be used. A monofunctional or bifunctional enzyme may be used. The reaction should preferably be carried out at a pH of 5.5 to 9, preferably 7 to 8.5. It may be carried out in the presence of a divalent metal salt, preferably Mg²⁺ in the case of YgbP or YchB and Mn²⁺ or Mg²⁺ in the case of YgbB. The temperature is preferably in the range of ± 10 °C from the optimum temperature. At least two consecutive enzymes of the set of enzymes YgbP, YchB and YgbB may also be used jointly in a combination screening test. Or YgbP may be combined with one or more enzymes upstream from YgbP in the pathway together with the appropriate substrates and cofactors.

The enzyme selected for the test should be preferably identical with the enzyme of the targeted organism. In case of a targeted group of plants (or bacteria), such as all mono- or dicotyledonous plants, any plant (bacterial) enzyme may be chosen or an enzyme whose sequence has the greatest commonality with all relevant plant (bacterial) sequences known and is thus representative for all relevant plant (bacterial) enzymes. In case of plant enzymes the leader sequence may be eliminated.

The start of this reaction can be timed by the addition of the last of the essential components. The reaction can be stopped by methanol, chelating agents, like EDTA or acids like trichloro acetic acid.

The activity of the enzyme can be detected (in the presence or absence of a potential inhibitor) by measuring in the case of YgbP either the formation of a product, namely pyrophosphate or 4-diphosphocytidyl-2C-methyl-D-erythritol or the consumption of starting material, namely CTP or 2C-methyl-D-erythritol 4-phosphate. In the case of YgbB the consumption of 4-diphosphocytidyl-2C-methyl-D-erythritol and/or the formation of 2C-methyl-D-erythritol 3,4-cyclophosphate may be measured; or alternatively the consumption of 4-diphosphocytidyl-2C-methyl-D-erythritol 2-phosphate and/or formations of 2C-methyl-D-erythritol 2,4-cyclopyrophosphate or cytidylmonophosphate. In the case of YchB the consumption of 4-diphosphocytidyl-2C-methyl-D-erythritol and/or adenosine 5-triphosphate (ATP) may be measured or the formation of 4-diphosphocytidyl-2C-methyl-D-erythritol 2-phosphate or adenosine 5-diphosphate (ADP). The measurements may be carried out either directly with the reaction mixture or after the separation of the reaction mixture by chromatography, such as HPLC.

The rate of pyrophosphate formation can be detected by coupling with UDP-glucose pyrophosphorylase, phosphoglucomutase and glucose 6-phosphate dehydrogenase. The formation of pyrophosphate is equivalent to the formation of NADPH, which can be monitored at 340 nm in a spectrophotometer. 4-diphosphocytidyl-2C-methyl-D-erythritol can also be detected directly by using ¹⁴C-labeled substrate and detecting the product with a radiomonitor. The consumption of 2C-methyl-D-erythritol 4-phosphate (unlabeled, ¹³C-labeled or ¹⁴C-labeled) and the formation of 4-diphosphocytidyl-2C-methyl-D-erythritol can also be monitored during the reaction or after the reaction by ³¹ P- or ¹³C-NMR spectroscopy or detection with a radiomonitor. The same method may be used for screening for an enzyme that is resistant to a specific inhibitor.

The rate of ADP formation can be detected by UV. 4-Diphosphocytidyl-2C-methyl-D-erythritol 2-phosphate can also be detected directly by using ¹⁴C-labeled substrate and detecting the product with a radiomonitor. The consumption of 4-diphosphocytidyl-2C-methyl-D-erythritol (unlabeled, ¹³C-labeled or ¹⁴C-labeled) and the formation of 4-diphosphocytidyl-2C-methyl-D-erythritol 2-phosphate can also be monitored during the reaction or after the reaction by ³¹P- or ¹³C-NMR spectroscopy or detection with a radiomonitor.

We have determined that YgbB can convert 4-diphosphocytidyl-2C-methyl-D-erythritol 2-phosphate (6) into 2C-methyl-D-erythritol 2,4-cyclopyrophosphate (7). The structure of the product was determined. The obtained 2C-methyl-D-erythritol-2,4-cyclopyrophosphate is a valuable compound for screening processes. It can be used as a reference compound for screening procedures in which the effectiveness of prospective inhibitors against YgbB is detected by the effectiveness of YgbB to produce 2C-methyl-D-erythritol-2,4-cyclopyrophosphate.

Our finding opens the way for novel screening procedures for finding inhibitors against YgbB. As an alternative to measuring the disappearance of 4-diphosphocytidyl-2C-methyl-D-erythritol 2-phosphate we can now measure the appearance of 2C-methyl-D-erythritol 2,4-cyclopyrophosphate. The measurement may be carried out either directly with the reaction mixture or after the separation of the reaction mixture by chromatography such as HPLC. The detection may be done by NMR or by a radio monitor.

The same methods may be used for screening for mutated enzymes that are resistant to a specific inhibitor.

### Large Scale Preparation

The starting materials for a comprehensive large-scale preparation of an intermediate are preferably dihydroxyacetone phosphate and pyruvate, whereby these starting materials may be used as free acids or as salts with a monovalent or divalent cation, preferably sodium or potassium. They may be used in equimolar amounts or in a molar ratio of 10:1 to 1:10 of dihydroxyacetone phosphate to pyruvate.

Glyceraldehyde 3-phosphate is the substrate for 1-deoxyxylulose 5-phosphate synthase. It is equivalent to dihydroxyacetone phosphate, which serves as its source in conjunction with triosephosphate isomerase. Dihydroxyacetone phosphate is preferred in view of its stability.

Further equivalent is glucose in the presence of ATP and the glycolytic enzymes hexokinase, phosphoglucose isomerase, phosphofructokinase, aldolase and triosephosphate isomerase.

The enzymes used may all be from the same organism, e.g. *E. coli,* or from different organisms. They are used in catalytic amounts with a molar ratio of 0.00001 to 0.1, preferably 0.001 to 0.1 to dihydroxyacetone phsophate or pyruvate.

The magnesium salt may be preferably magnesium chloride or sulfate and the Mn²⁺ salt may be a chloride or sulfate. Thiamine pyrophosphate may be used as free acid or as a salt, preferably with sodium or potassium.

Any enzymatically suitable buffer may be used. Tris hydrochloride is a preferred buffer. The pH is preferably 7 to 9 and especially 7.5 to 8.5, most notable 8.0. NADP⁺ may be used in stoichiometric amounts. It may also be regenerated in situ. For this purpose glucose and glucose dehydrogenase may be used. The molar ratio of dihydroxyacetone phosphate to pyruvate is preferably 1 to 10 and especially 1 to 3.

The reaction temperature should be chosen in accordance with the temperature optimum of the enzymes or differing from the optimum by up to 10 °C or preferably up to 5 °C. A preferred temperature range is 30 to 45 °C and especially 35 to 40 °C.

The reaction steps defined in claim 55 may be carried out separately, each under its own optimum conditions. The intermediate reaction mixtures may be stored in a freezer preferably at -30 °C to -10 °C, notably -20 °C. Prior to freezing the reaction may be stopped by adding an acid, such as HCl to lower the pH to 2 to 4, notably 2.5 to 3.5, preferably 3.0. The reaction steps may also be carried out as a one pot reaction. It is preferred to remove any precipitate formed in any of the steps by centrifugation.

### Labelling

The labelled substrates may be labelled by 32-phosphorus,14-carbon, 13-carbon, deuterium or tritium. These labelling types may be used alone or in any combination such as a combination of 13-carbon and deuterium.

The labelling with 14-carbon or 13-carbon may be single whereby any one of the C-positions may be labelled. Alternatively, the substrates may be multiply labelled, such as dual, triple, quadruple or quintuple. The total C-labelling is particularly preferred in case of 13-carbon labelling.

The labelling with deuterium or tritium may be single or multiple. 1-deoxy-D-xylulose 5-phosphate may be deuterium- or tritium-labelled in positions 1, 3, 4 or 5, preferably in positions 3, 4 or 5; and 2C-methyl-D-erythritol 4-phosphate may be deuterium- or tritium-labelled in positions 1, 3, 4 or the methyl group.

Other intermediates downstream from 2C-methyl-D-erythritol 4-phosphate with corresponding labelling may be used.

The labelled substrates may be prepared enzymatically or chemically.

Enzymatically, tritiated 1-deoxy-D-xylulose 5-phosphate may be prepared from [3-³H]pyruvate for tritiation in position 1; or from [1-³H]glyceraldehyde 3-phosphate or [1-³H]dihydroxyacetone 3-phosphate for tritiation in position 3 or from [2-³H]glyceraldehyde 3-phosphate for tritiation in position 4 or from [3-³H]glyceraldehyde 3-phosphate for tritiation in position 5; and subsequently the tritiated 2C-methyl-D-erythritol 4-phosphates may be obtained enzymatically from the corresponding tritiated 1-deoxy-D-xylulose 5-phosphates.

Specifically, [1-³H] glyceraldehyde 3-phosphate can be synthesized from [3-³H]glucose by enzymatic action of hexokinase, phosphoglucose isomerase, phosphofructokinase, aldolase and triose phosphate isomerase. The reaction mixture containing [1-³H] glyceraldehyde 3-phosphate can be directly used for synthesis of [3-³H]1-deoxy-D-xylulose 5-phosphate. [3-³H]1-Deoxy-D-xylulose 5-phopshate can be synthesized from [1-³H] glyceraldehyde 3-phosphate and pyrurate by enzymatic action of 1-deoxy-D-xylulose 5-phosphate synthase.

[1-³H]2C-methyl-D-erythritol 4-phosphate can be synthesized from [3-³H]1-deoxy-D-xylulose 5-phosphate by catalytic action of 1-deoxy-D-xylulose 5-phosphate reductoisomerase.

[2-³H]glyceraldehyde 3-phosphate can be synthesized from [2-³H]glucose by enzymatic action of hexokinase, phosphoglucose isomerase, phosphofructokinase and aldolase. The reaction mixture containing [2-³H]glyceraldehyde 3-phosphate can be directly used for synthesis of [4-³H] 1-deoxy-D-xylulose 5-phosphate as described above for [3-³H]1-deoxy-D-xyluose 5-phosphate. [3-³H]2C-methyl-D-erythritol 4-phosphate can be synthesized from [4-³H] 1-deoxy-D-xylulose 5-phosphate by catalytic action of 1-deoxy-D- xylulose 5-phosphate reductoisomerase.

Deuterium labelled, ¹³C-labelled or ¹⁴C-labelled substrates may be prepared analogously.

The basic enzymatic processes are known from G.A. Sprenger et al., Proc.Natl.Acad.Sci USA 94, 12857-12862 (1997); and Kuzuyama et al., Tetrahedron Lett. 39, 44509-4512 (1998).

Alternatively, the labelled 1-deoxy-D-xylulose compounds may be prepared by using the correspondingly labelled starting materials in the process described by Yokota, A and Sasajima, K. in Agric. Biol. Chem. 48, 149-158(1984) and ibid. 50, 2517-2524 (1986).

The labelled 2C-methyl-D-erythritol compounds may be obtained chemically by the following process, using correspondingly labelled starting materials:
(a) Reaction of 1,2,5,6-Di-O-ispropylidene-D-mannitol with lead tetracetate to isopropylidene glyceraldehyde; which is
(b) subsequently converted to 1,2-O-isopropylidene-(2R,3RS)-1,2,3 butanetriol by reaction with methyl magnesium iodide;
(c) formation of 3,4-O-isopropylidene-(3R)-3,4-dihydroxy-2-butanone from the product step (b) by oxidation, preferably with sodium periodate in the presence of ruthenium dioxide;
(d) formation of 1 ,2-0-isopropylidene-3-0-trimethylsilyl-(2R, 3RS)-1,2, 3 trihydroxy-3-cyano-butane by reacting the product of step (c) with trimethylsilyl cyanide;
(e) conversion of the product of step (d) to a mixture of 2C-methyl-D erythrono-1,4-lactone and 2C-methyl-D-threono-1,4-lactone by hydrolysis with an acid;
(f) production of 2,3-0-isopropylidene-2C-methyl-D-erythrono-1,4- lactone by reaction of the products of step (e) with acetone in the presence of anhydrous zink chloride;
(g) conversion of the product of step (f) to 2,3-0-ispropylidene-2C-methyl D-erythrofuranose by reaction with a hydride donor, preferably diisobutylaluminum hydride;
(h) conversion of the product of step (g) to 2,3-0-isopropylidene-2C-methyl D-erythrose-(O-benzyl)oxime by reaction with O-benzylhydroxylamine;
(i) reaction of the product of step (h) with tribenzylphosphite and iodine to obtain 2,3-O-isopropylidene-2C-methyl-D-erythrose-(O-benzyl) oxime 4 dibenzylphosphate;
(j) conversion of the product of step (i) to 2,3-O-isopropylidene-2C-methyl D-erythrose 4-dibenzylphosphate by ozonization;
(k) conversion of the product of step (j) to 2,3-0-isopropylidene-2C-methyl D-erythritol 4-dibenzylphosphate by reaction with sodium borohydride;
(l) converting the product of step (k) into 2C-methyl-D-erythritol 4-phosphate.

Tritiation in position 1 is possible by carrying out step (k) with tritiated sodium borohydride under otherwise identical conditions for the subsequent step (I). Tritiation in position 2' is possible by carrying out step (b) with tritiated methyl magnesium iodide prepared from tritiated methyl iodide and magnesium. The subsequent steps (c) to (I) remain unchanged. The combination of the tritiation steps is possible affording 2C-methyl-D-erythritol 4-phosphate acid tritiated in positions 1 and/or 2.

Deuterium labelled, ¹³C-labelled or ¹⁴C-labelled substrates may be prepared analogously.

Total C-labelling can be carried out advantageously starting from [U-¹³C₆] glucose and [U-¹³C₃] sodium pyruvate or [2,3-¹³C₂]pyruvate. In the presence of thiamine pyrophosphate, ATP and MgCl₂ the following enzymes are used for preparing [U-¹³C₅]1-deoxy-D-xylulose 5-phosphate: triose phosphate isomerase, hexokinase, phosphoglucose isomerase, phosphofructokinase, aldolase and 1-deoxy-D-xylulose 5-phosphate synthase. Subsequently, the product can be converted to [U-¹³C₅]2C-methyl-D-erythritol 4-phosphate with 1-deoxy-D-xylulose 5-phosphate reductoisomerase, glucose dehydrogenase and glucose, NADP⁺ and MgCl₂.

Further [U-¹³C₅]2C-methyl-D-erythritol 4-phosphate can be converted into [U-¹³C₅]4-diphosphocydidyl-2C-methyl-D-erythritol, [U-¹³C₅]4-diphosphocytidyl-2C-methyl-D-erythritol 2-phosphate and [U-¹³C₅]2C-methyl-D-erythritol 2,4-cyclopyrophosphate using the enzymes YgbP, YchB and YgbB in the presence of CTP, ATP, MgCl₂ and MnCl₂. For regeneration of ATP it is possible to use also pyruvate kinase in the presence of phosphoenol pyruvate.

### Nucleic Acids, Vectors, Expression Systems and Polypeptides

In practicing the present invention, many techniques in molecular biology, microbiology, recombinant DNA, and protein biochemistry such as these explained fully in, for example, Sambrook et al., 1989, Molecular Cloning, A Laboratory Manual, Second Edition, Cold Spring Harbor Laboratory Press, Cold Spring Harbor, New York; DNA Cloning: A practical Approach, Volumes I and II , 1985 (D.N. (Glover ed.); Oligonucleotide Synthesis, 1984, (M.L. Gait ed.); Transcription and Translation, 1984 (Hames and Higgins eds.); A Practical Guide to Molecular Cloning; the series, Methods in Enzymology (Academic Press, Inc.); and Protein Purtification: Principles and Practice, Second Edition (Springer-Verlag, NY.) are used.

The present invention encompasses nucleic acid sequences encoding (notably plant) enzyme, enzymatically active fragments derived therefrom, and related derived sequences from other (notably plant) species. As used herein, a nucleic acid that is "derived from" a sequence, refers to a nucleic acid sequence that corresponds to a region of the sequence, sequences that are homologous or complementary to the sequence, and "sequence-conservative variants" and " function-conservative variants". Sequence-conservative variants are those in which a change of one or more nucleotides in a given codon position results in no alteration in the amino acid encoded at that position. Function-conservative variants are those in which a given amino acid residue has been changed without altering the overall conformation and function of the polypeptide, including, but not limited to, replacement of an amino acid with one having similar physido-chemical properties (such as, for example, acidic, basic, hydrophobic, and the like). Enzymes fragments that retrain enzymatic activity can be identified according to the methods described herein, e.g, expression in *E. coli* followed by enzymatic assay of the cell extract.

Sequences derived from plants other than *Arabidopsis thaliana* can be isolated by routine experimentation using the methods and compositions provided herein. For example, hybridization of a nucleic acid comprising all or part of *Arabidopsis* sequence under conditions of intermediate stringency (such as, for example, an aqueous solution of 2X SSC at 65°C) to cDNA or genomic DNA derived from, other plant species can be used to identify homologues. cDNA libraries derived from different plant species are commercially available (Clontech, Palo Alto, CA; Stratagene, La Jolla, CA). Alternatively, PCR-based methods can be used to amplify related sequences from cDNA or genomic DNA derived from other plants.
Expression of the identified sequence in, e.g., *E. coli,* using methods described in more detail herein, is then performed to confirm the enzymatic activity of the polypeptide encoded by the sequence. Accordingly, sequences derived from dicotyledonous and monocotyledenous plants are within the scope of the invention.

The nucleic acids of the present invention include purine- and pyrimidine-containing polymers of any length, either polyribonucleotides or polydeoxyribonucleotides or mixed polyribo-polydeoxyribo-nucleotides. This includes single- and double-stranded molecules, i.e., DNA-DNA, DNA-RNA and RNA-RNA hybrids, as well as "protein nucleic acids" (PNA) formed by conjugating bases to an amino acid backbone. This also includes nucleic acids containing modified bases. The nucleic acids may be isolated directly from cells. Alternatively, PCR can be used to produce the nucleic acids of the invention, using either chemically synthesized strands or genomic material as templates. Primers used for PCR can be sythesized using the sequence information provided herein and can further be designed to introduce appropriate new restriction sites, if desirable, to introduce appropriate new restriction sites, if desirable, to facilitate incorporation into a given vector for recombinant expression.

The nucleic acids of the present invention may be flanked by natural *Arabidopsis* regulatory sequences, or may be associated with heterologous sequences, including promoters, enhancers, response elements, signal sequences, polyadenylation sequences, introns. 5'- and 3'- noncoding regions and the like. The nucleic acids may also be modified by many means known in the art. Non-limiting examples of such modification include methylation, "caps", substitution of one or more of the naturally occuring nucleotides with an analog, and internucleotide modification such as, for example, those with uncharged linkages (e.g., methyl phosphonates, phosphotriesters, phosphoromidates, carbamates, etc.) and with charged linkages (e.g., phosphorothioates, phosphorodithioates, etc.). Nucleic acids may contain one or more additional covalently linked moieties, such as, for example, proteins (e.g., nucleases, toxins, antibodies, signal peptides, poly-L-Lysine, etc.), intercalators (e.g., acridine, psoralen, etc.), chelators (e.g., metals, radioactive metals, iron, oxidative metals, etc.), and alkylators. The nucleic acid may be derivatized by formation of a methyl or ethyl phosphotriester or an alkyl phosphoramidate linkage. Furthermore, the nucleic acid sequences of the present invention may also be modified with a label capable of providing a detectable signal, either directly or indirectly. Exemplary labels include radioisotopes, fluorescent molecules, biotin, and the like.

The invention also provides nucleic acid vectors comprising the disclosed sequences or derivatives or fragments thereof. A large number of vectors, including plasmid and fungal vectors, have been described for replication and/or expression in an variety of eukaryotic and prokaryotic hosts. Non-limiting examples include pKK plasmids (Clontech), pUC plasmids, pET plasmids (Novagen, Inc., Madison, WI), or pRSET or pREP (Invitrogen, San Diego, CA), and many appropriate host cells, using methods disclosed or cited herein or otberwise known to those skilled in the relevant art. Recombinant cloning vectors will often include one or more replication sytems for cloning or expression, one or more markers for selection in the host, e.g. antibiotic resistance, and one or mose expression cassettes. Suitable host cells may be transformed/transfected/infected as appropriate by any suitable method including electroporation, CaCl₂ mediated DNA uptake, tungae infection, microinjection, miroprojectile, or other established methods.

Appropriate host cells include bacteria, archaebacteria, fungi, especially yeast, plant and animal cells, especially mammalian cells. Of particular interest are *E*. *coli, B. Subtilis, Saccharomyces cerevisiae, Saccharomyces carlsbergensis, Schizosacchromyces pombi, SF9* cells, C129 cells, 293 cells, *Neurospora,* and CHO cells, COS cells, HeLa cells, and immortalized mammalian myeloid and lyphoid cell lines. Preferred replication systems include M13, ColE1, SV40, baculovirus, lambda, adenovirus, and the like. A large number of transcription initation and termination regulatory regions have been isolated and shown to be effective in the transcription and translation of heterologeous proteins in the various hosts. Examples of these regions, methods of isolation, manner of manipulation, etc. are known in the art. Under appropriate expression conditions, host cells can be used as a source of recombinantly produced enzyme-derived peptides and polypeptides.

Advantageously, vectors may also include a transcription regulatory element (i.e., a promoter) operably linked to the enzyme portion. The promoter may optionally contain operator portions and/or ribosome binding sites. Non-limiting examples of bacterial promoters compatible with *E. coli* include: *trc* promoter, β-lactamase (penicillinase) promoter; lactose promoter; tryptophan (trp) promoter; arabinose BAD operon promoter, lambda-derived Pl promoter and N gene ribosome binding site; and the hybrid tac promoter derived from sequences of the trp and lac UV5 promoters. Non-limiting examples of yeast promoters include 3-phosphoglycerate kinase promoter, glyceraldehyde-3-phosphate dehydrogenase (GAPDH) promoter, galactokinase (GALI) promoter, galactoepimerase promoter, and alcohol dehydrogenase (ADH) promoter. Suitable promoters for mammalian cells include without limitation viral promoters such as that from Simian Virus 40 (SV40), Rous sarcoma virus (RSV), adenovirus (ADV), and bovine papilloma virus (BPV). Mammalian cells may also require terminator sequences and poly A addition sequences, and enhancer sequences which increase expression may also be included. Sequences which cause amplification of the gene may also be desirable. Furthermore, sequences that facilitate secretion of the recombinant product from cells, including, but not limited to, bacteria, yeast, and animal cells, such as secretory signal sequences and/or prohormone pro region sequences, may also be included.

Nucleic acids encoding wild-type or variant enzyme polypeptides may also be introduced into cells by recombination events. For example, such a sequence can be introduced into a cell, and thereby effect homologous recombination at the site of endogenous gene or a sequence with substantial identity to the gene. Other recombination-based methods, such as non-homologous recombinations or deletion of endogenous genes by homologous recombination, may also be used.

Enzyme-derived polypeptides according to the present invention, including function-conservative enzyme variants may be isolated from wild-type or mutant Arabidopsis cells, or from heterologous organisms or cells (including, but not limited to,bacteria, fungi, insect, plant, and mammalia cells) into which an enzyme-derived protein-coding sequence has been introduced and expressed. Furthermore, the polypeptides may be part of recombinant fusion proteins. Alternatively, polypeptides may be chemically synthesized by commercially available automated procedures, including, without limitation, exclusive solid phase synthesis, partial solid phase methods, fragment condensation or classical solution synthesis.

"Purification" of an enzyme polypeptide refers to the isolation of the enzyme polypeptide in a form that allows its enzymatic activity to be measured without interference by other components of the cell in which the polypeptide is expressed. Methods for polypeptide purification are well-known in the art, including, without limitation, preparative disc-gel electrophoresis, isoelectric focusing, reversed-phase HPLC gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibobdies produced against the enzyme or against peptides derived therefrom can be used as purification reagents. Other purification methods are possible.

The present invention also encompasses derivatives and homologues of the enzyme polypeptides. For some purposes, nucleic acid sequences encoding the peptides may be altered by substitutions, additions, or deletions that provide for functionally equivalent molecules, i.e., function conservative variants. For example, one or more amino acid residues within the sequence can be substituted by another amino acid of similar properties, such as, for example, positively charged amino acids (arginine, lysine, and histidine); negatively charged amino acids (aspartate and glutamate); polar neutral amino acids; and non polar amino acids.

The isolated polypeptides may be modified by, for example, phosphorylation, sulfation, acylation, or other protein modifications. They may also be modified with a label capable of providing a detectable signal, either directly or indirectly, including, but not limited to, radioisotopes and fluorescent compounds.

Genes corresponding to YgbP (or YgbB) or YchB from any plant may be readily isolated by well known techniques, for example by Southern hybridization or by PCR using degenerated primers. Notably, cDNA library of this plant in question is screened using the nucleic acid direct labelling and detection system kit supplied from Amersham-Pharmacia-Biotech (Heidelberg, Germany). Hybridization conditions are for example 7% sodium dodecyl sulfate (SDS), 0.5. Positively hybrizing plaques are detected by luminescence detection (or in other systems by autoradiography). After purification to single plaques, cDNA inserts are isolated, and their sequences determined by the chain termination method using dideoxy terminators labeled with fluorescent dyes (Applied Biosystems, ,Inc., Foster City, CA). This experimental protocol can be used by one of ordinary skill in the art to obtain genes substantially similar to the *Arabidopsis* gene from any other plant species.

### Screening Methods to Identifiy Enzyme Inhibitors/Herbicides

The methods and compositions of the present invention can be used to identify compounds that inhibit the function of the enzymes and thus are for example useful as herbicides or as lead compounds for the development of useful herbicides. This may be achieved by providing a cell that expresses the enzyme and thereby produces cell cultures expressing the enzyme are incubated in the presence of test compounds to form test cultures, and in the absence of lest compounds to form control cultures. Incubation is allowed to proceed for a sufficient time and under appropriate conditions to allow for interference with enzyme function. At a predetermined time after the start of incubation with a test compound, an assay is performed to monitor enzymatic activity. In one embodiment, enzyme activity is monitored in whole cells. Alternatively, enzymatic activity may be monitored in cell extracts or media containing the isolated enzyme using assays such as that described below. Additional controls; with respect to both cultur samples and assay samples, are also included, such as, for example, a host cell not expressing the enzyme (e.g., a host cell transformed with an expression plasmid containing the enzyme gene in a reverse orientation or with no insert). Enzyme inhibitory compounds are identified as those that reduce enzyme activity in the test cultures relative to the control cultures.

Host cells that may be used in practicing the present invention include without limitation bacterial, fungal, insect, mammalian, and plant cells. Preferably, bacterial cells are used. Most preferably, the bacterial cell is a variant (such as, e.g. the *imp* mutant of *E. coli )* that exhibits increased membrane permeability for test compounds relative to a wild-type host cell.

Preferably, the methods of the present invention are adapted to a high-throughput screen, allowing a multiplicity of compounds to be tested in a single assay. Such inhibitory compounds may be found in, for example, natural product libraries, fermentation libraries (encompassing plants and microorganisms), combinatorial libraries, compount files, and synthetic compound libraries. For example, synthetic compound libraries are commercially available from Maybridge Chemical Co. (Trevillet, Cornwall,UK), Comgenex (Princeton, NJ), Brandon Associates (Merrimack, NH), and Microsource (New Milford, CT). A rare chemical library is available from Aldrich Chemical Company, Inc. (Milwaukee, WI). Alternatively, libraries of natural compounds in the form of bacterial, fungal, plant and animal extracts are available from, for example, Pan Laboratories (Bothell, WA) or MycoSearch (NC), or are readily producible. Additionally, natural and synthetically produced libraries and compounds are readily modified through conventional chemical, physical, and biochemical means (Blondell et al., TibTech 14 : 60, 1996). Inhibitor assays according to the present invention are advantageous in accommodating many different types of solvents and thus allowing the testing of compounds from many sources.

Once a compound has been identified by the methods of the present invention as inhibitor, *in vivo* and *in vitro* tests may be performed to further characterize the nature and mechanism of the inhibitory activity. The effect of an identified compound on *in vitro* enzymatic activity of purified or partially purified may be determined and enzyme kinetic plots may be used to distinguish, e.g., competitive and non-competitive inhibitors.

Compounds identified as inhibitors using the methods of the present invention may be modified to enhance potency, efficacy, uptake, stability, and suitability for use in commercial herbicide applications, etc. These modifications are achieved and tested using methods well-known in the art.

### Isolation of Herbicide-Resistant Enzyme Variants

The present invention encompasses the isolation of enzyme variants that are resistant to the action of enzyme inhibitors/herbicides. The enzyme variants may be naturally occurring or may be obtained by random or site-directed mutagenesis.

In one embodiment, a population of cells or organisms expressing the enzyme of interest is mutagenized using procedures well-known in the art, after which the cells or organisms are subjected to a screening or selection procedure to identify those that are resistant to the toxic effects of an inhibitor. The variant enzyme gene is then isolated from the resistant cell or organism using, e.g., PCR techniques.

In another embodiment, an isolated enzyme gene is subjected to random or site-directed mutagenesis *in vitro,* after which mutagenized versions of the gene are reintroduced into an appropriat cell such as, e.g., *E. coli,* and the cells are subjected to a selection or screening procedure as above.

The variant enzyme genes are expressed in an appropriate host cell, and the enzymatic properties of variant enzyme polypeptides are compared to the wild-type enzyme. Preferably, a given mutation results in an enzyme variant polypeptide that retains in vitro enzymatic activity, while exhibiting catalytic activity that is relatively more resistant to the selected herbicide(s) than is wild-type enzyme. Preferably, when expressed in a cell that requires enzyme activity for viability, the variant exhibits (i) catalytic activity alone sufficient to maintain the viability of a cell in which it is expressed; or catalytic activity in combination with any herbicide resistant enzyme variant protein also expressed in the cell, which may be the same as or different than the first enzyme protein, sufficient to maintain the viability of a cell in which it is expressed; and (ii) catalytic activity that is more resisant to the herbicide than is wild type enzyme.

Therefore, any one specific enzyme variant protein need not have the total catalytic activity necessary to maintain the viability of the cell, but must have some catalytic activity in an amount, alone or in combination with the catalytic activity of additional copies of the same enzyme variant and/or the catalytic activity of other enzyme variant protein(s), sufficient to maintain the viability of a cell that requires enzyme activity for viability. For example, catalytic activity may be increased to minimum acceptable levels by introducing multiple copies of a variant encoding gene into the cell or by introducing the gene which further includes a relatively strong promoter to enhance the production of the variant.

More resistant means that the catalytic activity of the variant is diminished by the herbicide(s), if at all, to a lesser degree than wild-type enzyme catalytic activity is diminished by the herbicide(s). Preferred more resistant variant enzyme retains sufficient catalytic activity to maintain the viability of a cell, plant, or organism wherein at the same concentration of the same herbicide(s), wild-type enzyme would not retain sufficient catalytic activity to maintain the viability of the cell, plant or organism.

Preferably, the catalytic activity in the absence of herbicide(s) is at least about 5 % and, most preferably, is more than about 20 % of the catalytic activity of the wild-type enzyme in the absence of herbicide(s).

Herbicide-resistant enzyme variants can be used as genetic markers in any cell that is normally sensitive to the inhibitory effects of the herbicide formation. In one embodiment, DNA encoding an herbicide-resistant enzyme variant is incorporated into a plasmid under the control of a suitable promoter. Any desired gene can then be incorporated into the plasmid, and the final recombinant plasmid introduced into an herbicide-sensitive cell. Cells that have been transformed with the plasmid are then selected or screened by incubation in the presence of a concentration of herbicide sufficient to inhibit growth and/or pigment formation.

### Chemical-resistant Plants and Plants Containing Variant Enzyme Genes

The present invention encompasses transgenic cells, including, but not limited to seeds, organisms, and plants into which genes encoding herbicide-resistant enzyme variants have been introduced. Non-limiting examples of suitable recipient plants are listed in Table 3 below:

**Table 3**

| RECIPIENT PLANTS | | |
|---|---|---|
| **COMMON NAME** | FAMILY | LATIN NAME |
| | | |
| Maize | Gramineae | Zea mays |
| Maize; Dent | Gramineae | Zea mays dentiformis |
| Maize, Flint | Gramineae | Zea mays vulgaris |
| Maize, Pop | Gramineae | Zea mays microsperma |
| Maize, Soft | Gramineae | Zea mays amylacea |
| Maize, Sweet | Gramineae | Zea mays amyleasaccharata |
| Maize, Sweet | Gramineae | Zea mays saccharate |
| Maize, Waxy | Gramineae | Zea mays ceratina |
| | | |
| Wheat, Dinkel | Pooideae | Triticum spelta |
| Wheat, Durum | Pooideae | Triticum durum |
| Wheat, English | Pooideae | Triticum turgidum |
| Wheat, Large Spelt | Pooideae | Triticum spelta |
| Wheat, Polish | Pooideae | Triticum polonium |
| Wheat, Poulard | Pooideae | Triticum turgidum |
| Wheat, singlegrained | Pooideae | Triticum monococcum |
| Wheat, Small Spelt | Pooideae | Triticum monococcum |
| Wheat, Soft | Pooideae | Triticum aestivum |
| | | |
| Rice | Gramineae | Oryza sativa |
| Rice, American Wild | Gramineae | Zizania aquatica |
| Rice, Australian | Gramineae | Oryza australiensis |
| Rice, Indian | Gramineae | Zizania aquatica |
| Rice, Red | Gramineae | Oryza glaberrima |
| Rice, Tuscarora | Gramineae | Zizana aquatica |
| Rice, West African | Gramineae | Oryza glaberrima |
| | | |
| Barley | Pooideae | Hordeum vulgare |
| Barley, Abyssinian intermediate, also Irregular | Pooideae | Hordeum irregulare |
| Barley, Ancestral Tworow | Pooideae | Hordeum spontaneum |
| Barley, Beardless | Pooideae | Hordeum trifurcatum |
| Barley, Egyptian | Pooideae | Hordeum trifurcatum |
| Barley, fourrowed | Pooideae | Hordeum vulgare polystichon |
| Barley, sixrowed | Pooideae | Hordeum vulgare hexastichon |
| Barley, Tworrowed | Pooideae | Hordeum distichon |
| | | |
| Cotton, Abroma | Dicotyledoneae | Abroma augusta |
| Cotton, American Upland | Malvaceae | Gossypium hirsutum |
| Cotton, Asiatic Tree also Indian Tree | Malvaceae | Gossypium arboreum |
| Cotton, Brazilian, also, Kidney, and, Pernambuco | Malvaceae | Gossypium barbadense brasiliense |
| Cotton, Levant | Malvaceae | Gossypium herbaceum |
| Cotton Long Silk, also Long Staple, Sea Island | Malvaceae | Gossypium barbadense |
| Cotton Mexican, also Short Staple | Malvaveae | Gossypium hirsutum |
| | | |
| Soybean, Soya | Leguminosae | Glycine max |
| | | |
| Sugar beet | Chenopodiaceae | Beta vulgaris altissima |
| | | |
| Sugar cane | Woody-plant | Arenga pinnata |
| | | |
| Tomato | Solanaceae | Lycopersicon esculentum |
| Tomato, Cherry | Solanaceae | Lycopersicon esculentum cerasiforme |
| Tomato, Common | Solanaceae | Lycopersicon esculentum commune |
| Tomato, Currant | Solanaceae | Lycopersicon pimpinellifolium |
| Tomato, Husk | Solanaceae | Physalis ixocarpa |
| Tomato, Hyenas | Solanaceae | Solanum incanum |
| Tomato, Pear | Solanaceae | Lycopersicon esculentum pyriforme |
| Tomato, Tree | Solanaceae | Cyphomandra betacea |
| | | |
| Potato | Solanaceae | Solanum tuberosum |
| | | |
| Potato, Spanish, Sweet potato | Convolvulaceae | Ipormoca batatas |
| | | |
| Rye, Common | Pooideae | Secale cereale |
| Rye, Mountain | Pooideae | Secale montanum |
| | | |
| Pepper, Bell | Solanaceae | Capsicum annuum grossum |
| Pepper, Bird, also Cayenne, Guinea | Solanaceae | Capsicum annuum minimum |
| Pepper, Bonnet | Solanaceae | Capsicum sinense |
| Pepper, Bullnose, also Sweet | Solanaceae | Capsicum annuum grossum |
| Pepper, Cherry | Solanaceae | Capiscum annuum cerasiforme |
| Pepper, Cluster, also Red Cluster | Solanaceae | Capsicum annuum fasciculatum |
| Pepper, Cone | Solanaceae | Capsicum annuum conoides |
| Pepper, Goat, also Spur | Solanaceae | Capsicum frutescens |
| Pepper, Long | Solanaceae | Capsicum frutescens longum |
| Pepper, Ornamental Red, also Wrinkled | Solanaceae | Capsicum annuum abbreviatum |
| Pepper, Tabasco Red | Solanaceae | Capsicum annuum conoides |
| | | |
| Lettuce, Garden | Compositae | Lactuca sativa |
| Lettuce, Asparagus, also Celery | Compositae | Lactuca sativa asparagina |
| Lettuce, Blue | Compositae | Lactuca perennis |
| Lettuce, Blue, also Chicory | Compositae | Lactuca pulchella |
| Lettuce, Cabbage, also Head | Compositae | Lactuca satica capitata |
| Lettuce, Cos, also Longleaf, Romain | Compositae . | Lactuca sativa longifolia |
| Lettuce, Crinkle, also Curled, Cutting, Leaf | Compositae | Lactuca sativa crispa |
| | | |
| Celery | Umbelliferae | Apium graveolens dulce |
| Celery, Blanching, also Garden | Umbelliferae | Apium graveolens dulce |
| Celery, Root, also Turniproote | Umbelliferae | Apium graveolens rapaceum |
| | | |
| Eggplant, Garden | Solanaceae | Solanum melongena |
| | | |
| Sorghum | Sorghum | All crop specie |
| | | |
| Alfalfa | Leguminosae | Medicago sativum |
| | | |
| Carrot | Umbelliferae | Daucus carota sativa |
| | | |
| Bean, Climbing | Leguminosae | Phaseolus vulgaris vulgaris |
| Bean, Sprouts | Leguminosae | Phaseolus aureus |
| Bean, Brazilian Broad | Leguminosae | Canavalia ensiformis |
| Bean, Broad | Leguminosae | Vicia faba |
| Bean, Common, also French, White, Kidney | Leguminosae | Phaseolus vulgaris |
| Bean, Egyptian | Leguminosae | Dolichos lablab |
| Bean, Long, also Yardlong | Leguminosae | Vigna sesquipedalis |
| Bean, Winged | Leguminosae | Psophocarpus teragonolobus |
| | | |
| Oat, also Common, Side, Tree | Avena | Sativa |
| Oat, Black, also Bristle, Lopsided | Avena | Strigosa |
| Oat, Bristle | Avena | |
| | | |
| Pea, also Garden, Green, Shelling | Leguminosae | Pisum, sativum sativum |
| Pea, Blackeyed | Leguminosae | Vigna sinensis |
| Pea, Edible Podded | Leguminosae | Pisum sativum axipluum |
| Pea, Grey | Leguminosae | Pisum sativum speciosum |
| Pea, Winged | Leguminosae | Tetragonolobus purpureus |
| Pea, Wrinkled | Leguminosae | Pisum sativum meduilare |
| | | |
| Sunflower | Compositae | Helianthus annuus |
| | | |
| Squash, Autumn, Winter | Dicotyledoneae | Cucurbita maxima |
| Squash, Bush, also Summer | Dicotyledoneae | Cucurbita pepo melopepo |
| Squash, Turban | Dicotyledoneae | Cucurbita maxima turbaniformis |
| | | |
| Cucumber | Dicotyledoneae | Cucumis sativus |
| Cucumber, African, also Bitter | | Momordica charantia |
| Cucumber, Squirting, also Wild | | Ecbalium elaterium |
| Cucumber, Wild | | Cucumis anguria |
| | | |
| Poplar, California | Woody-Plant | Populus trichocarpa |
| Poplar, European Black | | Populus nigra |
| Poplar, Gray | | Populus canescens |
| Poplar, Lombardy | | Populus italica |
| Poplar, Silverleaf, also White | | Populus alba |
| Poplar, Wester Balsam | | Populus trichocarpa |
| | | |
| Tobacco | Solanaceae | Nicotiana |
| | | |
| Arabidopsis Thaliana | Cruciferae | Arabidopsis thaliana |
| | | |
| Turfgrass | Lolium | |
| Turfgrass | Agrostis | |
| | Other families of turfgrass | |
| | | |
| Clover | Leguminosae | |

Expression of the variant polypeptides in transgenic plants confers a high level of resistance to herbicides allowing the use of these herbicides during cultivation of the transgenic plants.

Methods for the introduction of foreign genes into plants are known in the art. Non-limiting examples of such methods include *Agrobacterium infection,* particle bombardment, polyethylene glycol (PEG) treatment of protoplasts, electroporation of protoplasts, microinjection, macroinjection, tiller injection, pollen tube pathway, dry seed inhibition, laser perforation, and electrophoresis. These methods are described in, for example, B. Jenes et al., and S.W, Ritchie et al. In Transgenic Plants, Vol. 1, Engineering and Utilization, ed. S.-D. Kung, R. Wu, Academic Press, Inc., Harcourt Brace Jovanovich 1993; and L. Mannonen et al., Critical Reviews in Biotechnology, 14: 287-310, 1994.

In a preferred embodiment, the DNA encoding a variant enzyme is cloned into a DNA vector containing an antibiotic resistance marker gene, and the recombinant enzyme DNA-containing plasmid is introduced into *Agrobacterium tumefaciens* containing a Ti plasmid. This "binary vector system" is described in, for example, U.S. Patent No. 4, 490,838, and in An et al.. Plant Mol. Biol. Manual A3: 1-19 (1988). The transformed *Agrobacterium* is then co-cultivated with leaf disks from the recipient plant to allow infection and transformation of plant cells. Transformed plant cells are then cultivated in regeneration medium, which promotes the formation of shoots, first in the presence of the appropriate antibiotic to select for transformed cells, then in the presence of herbicide. In plant cells successfully transformed with DNA encoding herbicide-resistant enzyme, shoot formation occurs even in the presence of levels of herbicide that inhibit shoot formation from non-transformed cells. After confirming the presence of variant enzyme DNA using, for example, polymerase chain reaction (PCR) analysis, transformed plants are tested for their ability to withstand herbicide spraying and for their capabilities for seed germination and root initiation and proliferation in the presence of herbicide.

The methods and compositions of the present invention can be used for the production of herbicide-resistant enzyme variants, which can be incorporated into plants to confer selective herbicide resistance on the plants. Intermediate variants of enzyme (for example, variants that exhibit sub-optimal specific activity but high herbicide resistance, or the converse) are useful as templates for the design of second-generation enzyme variants that retain adequate specific activity and high resistance.

Herbicide resistant enzyme genes can be tansformed into crop species in single or multiple copies to confer herbicide resistance. Genetic engineering of crop species with reduced sensitivity to herbicides can:
(1) Increase the spectrum and flexibility of application of specific effective and enviromentally benign herbicides;
(2) Enhance the commercial value of these herbicides;
(3) Reduce weed pressure in crop fields by effective use of herbicides on herbicide resistant crop species and a corresponding increase in harvest yields;
(4) Increase sales of seed for herbicid resistant plants;
(5) Increase resistance to crop darnage from carry-over of herbicides applied in previous planting;
(6) Decrease susceptiblity to changes in herbicide characteristics due to adverse climate conditions; and
(7) Increase tolerance to unevenly or mis-applied herbicides.

For example, transgenic enzyme variant protein containing plants can be cultivated. The crop can be treated with a weed controlling effective amount of the herbicide to which the enzyme variant transgenic plant is resistant, resulting in weed control in the crop without detrimentally affecting the cultivated crop.

The compounds detected as inhibitors by the above screening methods may be used as pure compound or in combination together with appropriate additives for inhibiting the enzymes in plant, bacterial or protozoal organisms. Conventional additives in the field of herbicides, antibacterial agents or antiprotozoal agents may be used.

The invention shall now be described with reference to specific examples.

### Example 1

Construction of expression vectors

### (a) pNC0113

2.0 *µ*g of the vector pQE30 (Qiagen, Hilden, Germany) is digested with 30 U of *Nco*I (New England Biolabs, Schwalbach, Germany (NEB)) in a total volume of 60 *µ*l containing 6 *µ*l of NEB4 buffer. The reaction mix is incubated for 3 h at 37 °C. After adding 33 *µ*M of each dNTP (NEB) and 5 U Klenow fragment of polymerase I from *E. coli* (NEB) the reaction mix is incubated for additional 30 min at 25 °C. The vector DNA is purified using the PCR purification kit from Qiagen. 500 *µ*l of buffer PB (Qiagen) are added to 98 *µ*l of PCR reaction mixture and applied to a Quiaquick column and centrifuged for 1 min at 114,000 rpm. The flow through is discarded. 0.75 ml of buffer PE (Qiagen) are loaded on the column and centrifuged as before. The flow through is discarded and the column is centrifuged for an additional 1 min at 14,000 rpm. The column is placed in a clean 1.5 ml eppendorf tube. 50 *µ*l of H₂O (redistilled, sterile) are added to the column and it is centrifuged for 1 min at 14,000 rpm. The flow through contained 1.5 *µ*g of purified vector DNA.

20 ng of vector DNA is religated with 1 U of T4-Ligase from Gibco-BRL (Eggenstein, Germany), 2 *µ*l of T4-Ligase buffer (Gibco-BRL) in a total volume of 10 *µ*l yielding the plasmid pQE_noNco. The ligation mixture is incubated over night at 4°C. With 2 *µ*l of the ligation mixture electrocompetent *E*. *coli* XLI-Blue (Bullock, W. 0., Fernandez, J. M., and Short, J. M. (1987). XLI-Blue: a high efficieny plasmid transforming recA Escherichia coli with β-galactosidase selection. BioTechniques 5, 376-379; commercial source: Stratagene, LaJolla, CA, USA) cells are transformed.

Preparation of electrocompetent cells: 1 liter of LB medium is inoculated 1:100 with fresh overnight culture. The cells are grown at 37 °C with shaking at 220 rpm to an optical density of 0.5 at 600 nm. The cells are chilled on ice for 20 min and centrifuged for 15 min at 4,000 rpm at 4°C. The supernatant is removed and the pellet is resuspended in 1 liter of ice-cold sterile 10 % (v/v) glycerol. The cells are centrifuged two times as described before resuspending the cells in 0.5 liter and in 20 ml of ice-cold sterile 10 % (v/v) glycerol, respectively. The cells are centrifuged an additional time and the pellet is resuspended in a volume of 2 ml of ice-cold 10 % (v/v) glycerol. This suspension is frozen in aliquots of 80 *µ*l and stored in liquid nitrogen.

Electro-transformation using the Gene Pulser apparatus from Biorad (Munich, Germany): The electrocompetent cells are thawed on ice. 40 *µ*l of the cell suspension are mixed with 2 *µ*l of ligation mixture and transferred into a prechilled, sterile 0.2 cm cuvette (Biorad). The suspension is shaked to the bottom and the cuvette is placed into the prechilled chamber slide. The chamber slide is pushed into the chamber and the cells are pulsed at 2.50 kV, 25 *µ*F and Pulse Controller setting 200 Ω. The cuvette is removed from the chamber and the cells are suspended in 1 ml of SOC medium (2 % (w/v) casein hydrolysate, 0.5 % (w/v) yeast extract, 10 mM NaCl, 2.5 mM KCI, 10 mM MgCl₂, 10 mM MgSO₄ and 20 mM glucose. The suspension is shaked for 1 h at 37 °C and 100 *µ*l of the suspension is plated on LB plates containing 150 mg/l ampicillin for maintenance of the plasmid pQE_noNco.

Cells of *Escherichia coli* XLI-Blue harboring the vector pQE_noNco, are grown overnight in Luria Bertani (LB) medium containing 180 mg/l of ampicillin for maintenance of the plasmid in the host cells. 7 ml of the culture are centrifuged for 20 min at 5,000 rpm. The cell pellet is used for isolation of the plasmid pQE_noNco with the mini plasmid isolation kit from Qiagen (Hilden, Germany). The pellet is resuspended in 0.3 ml of 10 mM EDTA in 50 mM Tris hydrochloride, pH 8.0. 30 *µ*g RNase A are added. 0.3 ml of 1 % (w/v) SDS in 200 mM sodium hydroxide are added and incubated for 5 min at room temperature. 0.3 ml of chilled 3.0 M sodium acetate, pH 5.5 are added and incubated for 10 min on ice. The mixture is centrifuged for 15 min at 14,000 rpm in a minifuge. The supernatant is applied onto a Quiagen-tip 20, which is previously equilibrated with 1 ml of 750 mM NaCl, 15 % (v/v) ethanol and 0.15 % (v/v) Triton X-100 in 50 mM MOPS, pH 7.0. The Quiagen-tip is washed four times with 1 ml of of 1000 mM NaCl and 15 % (v/v) ethanol in 50 mM MOPS, pH 7.0. The DNA is eluted with 0.8 ml of 1250 mM NaCl and 15 % (v/v) ethanol in 50 mM Tris hydrochloride, pH 8.5. The DNA is precipated with 0.56 ml of isopropanol, centrifuged 30 min at 14,000 rpm and washed with 1 ml of ice-cold 70 % (v/v) ethanol. After drying in a speedvac for 5 min, the DNA is dissolved in 50 µl of redistilled H₂O. The solution contained 8.3 µg of the vector DNA pQE_noNco.

The DNA sequence of the vector pQE_noNco is sequenced by the automated dideoxynucleotide method (Sanger, F., S. Nicklen, und A. R. Coulson. (1977). DNA sequence analysis with chain terminating inhibitors. Proc. Acad. Natl. Sci. USA 74, 5463-5468) using an ABI Prism 377^{™} DNA sequencer from Perkin Elmer (Norwalk, USA) with the ABI Prism^{™} Sequencing Analysis Software from Applied Biosystems Divisions (Foster City, USA).

The DNA sequence is found to be as expected.

2.0 *µ*g of the vector pQE_noNco is digested with 30 U of *Eco*RI and 30 U of *Sal*I (NEB) in a total volume of 60 *µ*l containing 6 *µ*l of *Eco*RI buffer (NEB). The reaction mix is incubated for 3 h at 37 °C. The vector DNA is purified using the PCR purification kit from Qiagen.

25 pmol of the oligonucleotides 5'-CACACAGAATTCATTAAAGAG GAGAAATTAA CCATGGGAGGATCCGTCGACCTGCAGCC-3' and 5'-GGCTGCAGGTCGACGGA TCCTCCCATGGTTAATTTCTCCTCTTTA ATGAATTCTGTGTG-3' are dissolved in 6 *µ*l *Eco*RI buffer (NEBand 54 µl H₂O. The solution is heated at 96°C for 2 min and cooled down to 10°C within 12 h in order to hybridisize the DNA linker. The reaction mix is supplied with 30 U of *Eco*RI and 30 U of Sa/I (NEB) and incubated for 3 h at 37°C. The reaction mix is heated to 65°C for 30 min in order to inactivate the enzymes and cooled down to 10°C within 12 h for hybridisation.The reaction mix contains approximately 730 ng of the DNA linker.

20 ng of the digested pQ.E_noNco vector DNA (see above) and 300 pg of the DNA linker, 2 *µ*l of T4-Ligase buffer (Gibco-BRL) are ligated together with 1 U of T4-Ligase from Gibco-BRL (Eggenstein, Germany),), 2 *µ*l of T4-Ligase buffer (Gibco-BRL) in a total volume of 10 *µ*l yielding the plasmid pNCO1 13. The ligation mixture is incubated over night at 4 °C. With 2 *µ*l of the ligation mixture electrocompetent *E. coli* XL1-Blue cells are transformed.

5 *µ*g of the plasmid pNCO113 are isolated and the DNA sequence of the vector pNCO113. is sequenced as described above. The DNA sequence is shown in Annex F1. The culture is on deposit with ATCC as a patent deposit with the title *Escherichia coli* strain XL1-Blue habouring plasmid pNCO113, assigned PTA-852, date of deposit: October 14, 1999.

### (b) pNCO-SB-H₆-ACYC184 (expression of His₆-X fusion proteins)

5.0 µg of the vector pACYC184 (New England Biolabs, Schwalbach, Germany (NEB)) is digested with 30 U of *Nco*I (NEB) and 40 U of *BamH*I (NEB) in a total volume of 70 µl containing 7 µl of NEB4 buffer. The reaction mixture is incubated for 3 h at 37 °C and size-separated on a 0.8 % agarose gel electrophoresis. A 2.2 kB Ncol/BamHl DNA fragment is excised from the gel and purified with the QlAquick gel extraction kit from Qiagen (Hilden, Germany). To 500 mg of gel slice 1500 µl of QG buffer are added and the mixture is incubated at 50 °C for 10 min. 500 µl of isopropanol are added and the mixture is applied to a Quiaquick spin column and centrifuged for 1 min at 14,000 rpm. The flow through is discarded. 0.75 ml of buffer PE (Qiagen) are loaded on the column and centrifuged as before. The flow through is discarded and the column is centrifuged for an additional 1 min at 14,000 rpm. The column is placed in a clean 1.5 ml eppendorf tube. 50 µl of H₂O (redistilled, sterile) are added to the column and it is centrifuged for 1 min at 14,000 rpm. The flow through contains 1.5 µg of purified DNA fragment NB-ACYC184. 3 µg of the vector pNCO113 is digested with 30 U of *Nco*I (NEB) and 40 U of *BamH*I (NEB) in a total volume of 70 µl containing 7 µl of NEB4 buffer. The reaction mixture is incubated for 3 h at 37 °C. The Ncol/BamHI digested pNCO113 vector is purified with the PCR purification kit from Qiagen. 210 µl of PB buffer are added to the 70 µl of the restriction mixture and the total mixture is applied to a Quiaquick spin column and centrifuged for 1 min at 14,000 rpm. The flow through is discarded. 0.75 ml of buffer PE (Qiagen) are loaded on the column and centrifuged as before. The flow through is discarded and the column is centrifuged for an additional 1 min at 14,000 rpm. The column is placed in a clean 1.5 ml eppendorf tube. 50 µl of H₂O (redistilled, sterile) are added to the column and it is centrifuged for 1 min at 14,000 rpm. The flow through contains 1.4 µg of the purified *Nco*I/*BamH*I restricted vector pNCO113.

20 ng of vector DNA and 10 ng of the DNA fragment NB-ACYC184 are ligated with 1 U of T4-Ligase from Gibco-BRL (Eggenstein, Germany), 2 µl of T4-Ligase buffer (Gibco-BRL) in a total volume of 10 µl yielding the plasmid pNCO-NB-ACYC184.

The ligation mixture is incubated over night at 4 °C. With 2 µl of the ligation mixture electrocompetent *E*. *coli* XL1-Blue (Bullock, W.O., Fernandez, J. M., and Short, J. M. (1987). XL1-Blue: a high efficieny plasmid transforming recA Escherichia coli with β-galactosidase selection. BioTechniques 5, 376-379; commercial source: Stratagene, LaJolla, CA, USA) cells are transformed.

Preparation of electrocompetent cells: 1 liter of LB medium is inoculated 1:100 with fresh overnight culture. The cells are grown at 37 °C with shaking at 220 rpm to an optical density of 0.5 at 600 nm. The cells are chilled on ice for 20 min and centrifuged for 15 min at 4,000 rpm at 4 °C. The supernatant is removed and the pellet is resuspended in 1 liter of ice-cold sterile 10 % (v/v) glycerol. The cells are centrifuged two times as described before resuspending the cells in 0.5 liter and in 20 ml of ice-cold sterile 10 % (v/v) glycerol, respectively. The cells are centrifuged an additional time and the pellet is resuspended in a volume of 2 ml of ice-cold 10 % (v/v) glycerol. This suspension is frozen in aliquots of 80 µl and stored in liquid nitrogen.

Electro-transformation using the Gene Pulser apparatus from Biorad (Munich, Germany): The electrocompetent cells are thawed on ice. 40 µl of the cell suspension are mixed with 2 µl of ligation mixture and transferred into a prechilled, sterile 0.2 cm cuvette (Biorad). The suspension is shaked to the bottom and the cuvette is placed into the prechilled chamber slide. The chamber slide is pushed into the chamber and the cells are pulsed at 2.50 kV, 25 µF and Pulse Controller setting 200 Ω. The cuvette is removed from the chamber and the cells are suspended in 1 ml of SOC medium (2 % (w/v) casein hydrolysate, 0.5 % (w/v) yeast extract, 10 mM NaCl, 2.5 mM KCI, 10 mM MgCl₂, 10 mM MgSO₄ and 20 mM glucose. The suspension is shaked for 1 h at 37 °C and 100 µl of the suspension is plated on LB plates containing 150 mg/l ampicillin for maintenance of the plasmid pNCO-NB-ACYC184.

Cells of *Escherichia coli* XLI-Blue harboring the vector pNCO-N8-ACYC184, are grown overnight in Luria Bertani (LB) medium containing 180 mg/l of ampicillin for maintenance of the plasmid in the host cells. 7 ml of the culture are centrifuged for 20 min at 5,000 rpm. The cell pellet is used for isolation of the plasmid pNCO-NB-ACYC184 with the mini plasmid isolation kit from Qiagen: The pellet is resuspended in 0.3 ml of 10 mM EDTA in 50 mM Tris hydrochloride, pH 8.0. 30 µg RNase A are added. 0.3 ml of 1 % (w/v) SDS in 200 mM sodium hydroxide are added and incubated for 5 min at room temperature. 0.3 ml of chilled 3.0 M sodium acetate, pH 5.5 are added and incubated for 10 min on ice. The mixture is centrifuged for 15 min at 14,000 rpm in a minifuge. The supernatant is applied onto a Quiagen-tip 20, which is previously equilibrated with 1 ml of 750 mM NaCl, 15 % (v/v) ethanol and 0.15 % (v/v) Triton X-100 in 50 mM MOPS, pH 7.0. The Quiagen-tip is washed four times with 1 ml of of 1000 mM NaCl and 15 % (v/v) ethanol in 50 mM MOPS, pH 7.0. The DNA is eluted with 0.8 ml of 1250 mM NaCl and 15 % (v/v) ethanol in 50 mM Tris hydrochloride, pH 8.5. The DNA is precipated with 0.56 ml of isopropanol, centrifuged 30 min at 14,000 rpm and washed with 1 ml of ice-cold 70 % (v/v) ethanol. After drying in a speedvac for 5 min, the DNA is dissolved in 50 µl of redistilled H₂O. The solution contains 7.5 µg of the vector DNA pNCO-NB-ACYC184.

The DNA sequence of the vector pNCO-NB-ACYC184 is sequenced by the automated dideoxynucleotide method (Sanger, F., S. Nicklen, und A. R. Coulson. (1977). DNA sequence analysis with chain terminating inhibitors. Proc. Acad. Natl. Sci. USA 74, 5463-5468) using an ABI Prism 377^{™} DNA sequencer from Perkin Elmer (Norwalk, USA) with the ABI Prism^{™} Sequencing Analysis Software from Applied Biosystems Divisions (Foster city, USA).
The DNA sequence is found to be as expected.

4.0 µg of the vector pNCO-NB-ACYC184 is digested with 30 U of *Nco*I and 30 U of *Sac*II (NEB) in a total volume of 60 µl containing 6 µl of NEB4 buffer (NEB). The reaction mix is incubated for 3 h at 37 °C. The reaction mixture si size-seperated by agarose gel electrophoresis and the vector DNA is purified using the Gel extraction kit kit from Qiagen as described above. 2.1 µg DNA are obtained.

Each 1 nmol of the oligonucleotides 5'-CATGCACCACCACCACCACCACGCGTCCATGGCCGC-3' and 5'-GGCCATGGACGCGTGGTGGTGGTGGTGGTG-3' are dissolved in 15 µl of 66 mM MgCl₂, 0.5 mM NaCl, 10 mM DTT and 66 mM Tris hydrochloride, pH 7.6. Water is added to a final volume of 100 µl and the reaction mixture is heated at 94 °C for 15 min. After heating at 60 °C for further 15 min the reaction mixture is cooled down to room temperature within 1 h in order to hybridisize the His-tag DNA linker.

20 ng of the digested vector DNA (see above) and 300 pg of the DNA linker, 2 µl of T4-Ligase buffer (Gibco-BRL, Eggenstein, Germany) are ligated together with 1 U of T4-Ligase from Gibco-BRL, 2 µl of T4-Ligase buffer (Gibco-BRL) in a total volume of 10 µl yielding the plasmid pNCO-SB-H₆-ACYC184. The ligation mixture is incubated over night at 4 °C. With 2 µl of the ligation mixture electrocompetent *E. coli* XL1-Blue cells are transformed.
4.5 µg of the plasmid pNCO-SB-H₆-ACYC184 are isolated and the DNA sequence of the vector pNCO-SB-H₆-ACYC184 is sequenced as described above. The DNA sequence is shown in Annex F2.

### Example 2

### Production of an expression clone and construction of an expression vector for ygbP of E. coli

Cells of *Escherichia coli* XL1-Blue harboring the expression vector pNCO113, are grown overnight in Luria Bertani (LB) medium containing 180 mg/l of ampicillin for maintenance of the plasmid in the host cells. 7 ml of the culture are centrifuged for 20 min at 5,000 rpm. The cell pellet is used for isolation of the plasmid pNCO1 13 with the mini plasmid isolation kit from Qiagen (Hilden, Germany). The pellet is resuspended in 0.3 ml of 10 mM EDTA in 50 mM Tris hydrochloride, pH 8.0. 30 µg RNase are added. 0.3 ml of 1 % (w/v) SDS in 200 mM sodium hydroxide are added and incubated for 5 min at room temperature. 0.3 ml of chilled 3.0 M sodium acetate, pH 5.5 are added and incubated for 10 min on ice. The mixture is centrifuged for 15 min at 14,000 rpm in a minifuge. The supernatant is applied onto a Quiagen-tip 20, which is previously equilibrated with 1 ml of 750 mM NaCl, 15 % (v/v) ethanol and 0.15 % (v/v) Triton X-100 in 50 mM MOPS, pH 7.0. The Quiagen-tip is washed four times with 1 ml of of 1000 mM NaCl and 15 % (v/v) ethanol in 50 mM MOPS, pH 7.0. The DNA is eluted with 0.8 ml of 1250 mM NaCl and 15 % (v/v) ethanol in 50 mM Tris hydrochloride, pH 8.5. The DNA is precipated with 0.56 ml of isopropanol, centrifuged 30 min at 14,000 rpm and washed with 1 ml of ice-cold 70 % (v/v) ethanol. After drying in a speedvac for 5 min, the DNA is dissolved in 50 *µ*l of redistilled H₂O. The solution contained 8.3 *µ*g of DNA.

Chromosomal DNA from *Escherichia coli* strain XL1-Blue is isolated according to a method described by Meade et al. (Meade, H. M., Long, S. R., Ruvkun, C. B., Brown, S. E., and Auswald, F. M. (1982). Physical and genetic characterization of symbiotic and auxotrophic mutants of Rhizobium meliloti induced by transposon Tn5 mutagenis. J. Bacteriol. 149, 114-122). The *E. coli* ORF *ygbP* (accession no. gb AE000358) from basepair (bp) position 6754 to 7464 is amplified by PCR using chromosomal *E. coli* DNA as template. The reaction mixture contained 25 pmol of primer AAATTAACCATGGCAACCACTCAT TTGG, 25 pmol of primer TTGGGCCTGCAGCGCCAAAGG, 20 ng of chromosomal DNA, 2U of Taq DNA polymerase (Eurogentec, Seraing, Belgium) and 20 nmol of dNTPs in 1.5 mM MgCl₂, 50 mM KCI, 10 mM Tris-hydrochloride, pH 8.8 and 0.1 % (w/w) Triton X-100 in a total volume of 100 *µ*l.

The mixture is denaturated for 3 min at 95 °C. Then 25 PCR cycles for 30 sec at 94°C, 30 sec at 50 °C and 45 sec at 72 °C follow. After further incubation for 7 min at 72 °C, the mixture is cooled to 4 °C. An aliquot of 2 *µ*l is subjected to agarose gel electrophoresis. The PCR amplificate is purified with PCR purification kit from Qiagen. 500 *µ*l of buffer PB (Qiagen) are added to 98 *µ*l of PCR reaction mixture and applied to a Quiaquick column and centrifuged for 1 min at 14,000 rpm. The flow through is discarded. 0.75 ml of buffer PE (Qiagen) are loaded on the column and centrifuged as before. The flow through is discarded and the column is centrifuged for an additional 1 min at 14,000 rpm. The column is placed in a clean 1.5 ml eppendorf tube. 50 *µ*l of H₂O (redistilled, sterile) are added to the column and it is centrifuged for 1 min at 14,000 rpm. The flow through contained 1.5 µg of purified PCR product:

2.0 µg of the vector pNCO113 and 1.5 µg of the purified PCR product are digested in order to produce DNA fragments with overlapping ends. Each restriction mixture contained 7 *µ*l of NEB3 buffer (NEB), 7 *µ*g of BSA, 40 U of *Nco*I (NEB), 30 U of *Pst*I (NEB) in a total volume of 70 *µ*l and is incubated for 3 h at 37 °C. Digested vector DNA and PCR product are purified using the PCR purification kit from Qiagen.

20 ng of vector DNA and 16 ng of PCR product are ligated together with 1 U of T4-Ligase from Gibco-BRL (Eggenstein, Germany), 2 *µ*l of T4-Ligase buffer (Gibco) in a total volume of 10 *µ*l yielding the plasmid pNCOygbP. The ligation mixture is incubated over night at 4 °C. With 2 *µ*l of the ligation mixture electrocompetent *E. coli* XL1-Blue cells are transformed.

The plasmid pNCOygbP is isolated as described in example 1.

The DNA insert of the plasmid pNCOygbP is sequenced as described in example 1.

### Example 3a

### Construction of an expression vector for ygbP of A. thaliana without leader sequence.

1 g of 2 weeks old Arabidopsis thaliana var. Columbia plants (stems and leafs) are frozen and homogenisated in liquid nitrogen. 8 ml of a sterile solution of 600 g/l guanidine thiocyanate, 5 g/l sodium-N-lauroylsarcosine, 50 mM trisodium citrate and 5 ml/l 2-mercaptoethanol are added. This mixture is added carefully to 3 ml of a solution (autoclaved) of 959 g/l CsCl and 37,2 g/l EDTA and centrifugated at 33000 rpm at 18°C for 24 h. The supernatant is dicarded and the pellet is airdried for 10 min. The dried pellet is dissolved in 360 ml H₂O (bidestillated, sterile). The solution is centrifugated at 14000 rpm for 10 min. The supernatant is mixed with 40 ml 3 M sodium acetate and 1 ml ethanol. The RNA is precipitated over night at -20°C, centrifugated at 14000 rpm at 4°C for 15 min. and washed twice with 500 ml 75% ethanol. The pellet is airdried and dissolved in 200 ml H₂O (bidestillated, sterile). 500 *µ*g RNA are obtained.

A mixture containing 2.75 mg RNA, 50 nmol dNTP's, 1 mg random hexameric primer, 1 mg T₁₅-primer and 20 % first strand 5x buffer (Promega, Madison, USA) in a total volume of 50 ml is incubated for 5 min. at 95 °C, cooled on ice and 500 U M-MLV reverse transkriptase (Promega) are added. The mixture is incubated for 1 h at 42 °C. After incubation at 92 °C for 5 min., RNase A (20 U) and RNase H (2 U) are added and the mixture is incubated for 30 min. at 37 °C.
The resulting cDNA (1 ml of this mixture) is used for the amplification of the *ygbP* gene by PCR.

The expression vector pNCO113 is isolated as described in example 1. The *A*. *thaliana* ORF *ygbP* (accession no. gbAL004136) without the coding region for the putative leader sequence from basepair (bp) position 79845 to 81915 is amplified by PCR using cDNA from A. *thaliana* as template. The reaction mixture contained 25 pmol of primer TTGTTGTGAAGGAGAAGAGTG, 25 pmol of primer CATGCATACCCTTGACACGTC, 1 *µ*g of cDNA, 2U of Taq DNA polymerase (Eurogentec, Seraing, Belgium) and 20 nmol of dNTPs in 1.5 mM MgCl₂, 50 mM KCl, 10 mM Tris-hydrochloride, pH 8.8 and 0.1 % (w/w) Triton X-100 in a total volume of 100 *µ*l.

The mixture is denaturated for 3 min at 95 °C. Then 40 PCR cycles for 45 sec at 94 °C, 45 sec at 50 °C and 60 sec at 72 °C followed. After further incubation for 20 min at 72 °C, the mixture is cooled to 4 °C. An aliquot of 2 *µ*l is subjected to agarose gel electrophoresis. The PCR amplificate is purified with the PCR purification kit from Qiagen as described in Example 1. 1.5 µg of purified PCR product are obtained.

The PCR amplificate is used as template for a second PCR reaction. The reaction mixture contained 25 pmol of primer CAATGTTGTTGCCAT GGAGAAG, 25 pmol of primer ACACGTCTTCTGCAGAAGTAAATG, 2 *µ*l of the first PCR amplification , 2U of Taq DNA polymerase (Eurogentec, Seraing, Belgium) and 20 nmol of dNTPs in a total volume of 100 *µ*l of 1.5 mM MgCl₂, 50 mM KCI, 10 mM Tris-hydrochloride, pH 8.8 and 0.1 % (w/w) Triton X-100.

The mixture is denaturated for 3 min at 95 °C. Then 40 PCR cycles for 45 sec at 94 °C, 45 sec at 50 °C and 60 sec at 72 °C follow. After further incubation for 20 min at 72 °C, the mixture is cooled to 4 °C. An aliquot of 2 *µ*l is subjected to agarose gel electrophoresis. An aliquot of 2 *µ*l is subjected to agarose gel electrophoresis.

The PCR amplificate is purified with PCR purification kit from Qiagen as described in Example 1. 1.2 *µ*g of purified PCR product are obtained. 2.0 *µ*g of the vector pNCO113 (isolated as described in example 2) and 1.5 *µ*g of the purified PCR product are digested in order to produce DNA fragments with overlapping ends. Each restriction mixture contained 7 *µ*l of NEB3 buffer, 40 U of *NcoI* (NEB), 30 U of *PstI* (NEB) in a total volume of 70 *µ*l and is incubated for 3 h at 37 °C. Digested vector DNA and PCR product are purified using the PCR purifiaction kit from Qiagen.

20 ng of vector DNA and 8 ng of PCR product are ligated together with 1 U of T4-Ligase (Gibco), 2 µl of T4-Ligase buffer (Gibco) in a total volume of 10 µl, yielding the plasmid PNCOygbPara. The ligation mixture is incubated over night at 4°C. 2 µl of the ligation mixture is transformed into electrocompetent *E. coli* XL1-Blue cells as described in example 1. The electrocompetent cells are prepared as described in example 1.

The DNA insert of the plasmid pNCOygbPara is sequenced as described in Example 1. The results are shown in Annex Da.

### Example 3 b

### Production of an expression clone and construction of an expression vector for the full-length ygbP of Arabidopsis thaliana

1 g of 2 weeks old *Arabidopsis thaliana* var. Columbia plants (stems and leafs) are frozen and homogenisated in liquid nitrogen. 8 ml of a sterile solution of 600 g/I guanidine thiocyanate, 5 g/l sodium-N-lauroylsarcosine, 50 mM trisodiumcitrate and 5 ml/l 2-mercaptoethanol are added. This mixture is added carefully to 3 ml of a solution (autoclaved) of 959 g/l CsCl and 37,2 g/l EDTA and centrifugated at 33,000 rpm at 18°C for 24 h. The supernatant is dicarded and the pellet is airdried for 10 min. The dried pellet is dissolved in 360 µl H₂O (bidestillated, sterile). The solution is centrifugated at 14,000 rpm for 10 min. The supernatant is mixed with 40 µl 3 M sodium acetate and 1 ml ethanol. The RNA is precipitated over night at - 20°C, centrifugated at 14,000 rpm at 4°C for 15 min. and washed twice with 500 µl 75% ethanol. The pellet is airdried and dissolved in 200 µl H₂O (bidestillated, sterile). 500 µg RNA are obtained.

A mixture containing 2.75 µg RNA, 50 nmol dNTP's, 1 µg random hexameric primer, 1 µg T₁₅-primer and 20 % first strand 5x buffer (Promega) in a total volume of 50 µl is incubated for 5 min. at 95 °C, cooled on ice and 500 U M-MLV reverse transkriptase (Promega) are added. The mixture is incubated for 1 h at 42 °C. After incubation at 92 °C for 5 min., RNase A (20 U) and RNase H (2 U) are added and the mixture is incubated for 30 min. at 37 °C.
The resulting cDNA (1 µl of this mixture) is used for the amplification of *ygbP* by PCR.

The expression vector pQE30 (Qiagen) is isolated as described in example 1. The full-length A. *thaliana* ORF *ygbP* (accession no. gb AC004136) is amplified from base pair (bp) position 19412 to 21482 by PCR using cDNA from *A. thaliana* as template (see above). The reaction mixture contains 25 pmol of the primer 5'-CTTCTCTCAGGCGAGATAAAACATGG-3', 25 pmol of the primer 5'-CATGCATACCCTTGACACGTC-3', 1 µg of cDNA, 2 U of Taq-DNA polymerase (Eurogentec, Seraing, Belgium) and 20 nmol of dNTPs in a total volume of 100 µl of 1.5 mM MgCl₂, 50 mM KCl, 10 mM Tris-hydrochloride, pH 8.8 and 0.1 % (w/w) Triton X-100.

The mixture is denaturated for 3 min at 95 °C. Then 40 PCR cycles for 45 sec at 94 °C, 45 sec at 50 °C and 60 sec at 72 °C followed. After further incubation for 20 min at 72 °C, the mixture is cooled to 4 °C. An aliquot of 2 µl is subjected to agarose gel electrophoresis. The PCR amplificate is purified with the PCR purification kit from Qiagen as described in Example 1. 1.7 µg of purified PCR product are obtained.

The PCR amplificate is used as template for a second PCR reaction. The reaction mixture contains 25 pmol of the primer 5'-GGCGAGAGGATCCATGGCGATGTCTCAGACG-3', 25 pmol of the primer 5'-ACACGTCTTCTGCAGAAGTAAATG-3', 2 µl of the first PCR amplification, 2 U of Taq DNA polymerase (Eurogentec, Seraing, Belgium) and 20 nmol of dNTPs in a total volume of 100 µl containing 1.5 mM MgCl₂, 50 mM KCI, 10 mM Tris-hydrochloride, pH 8.8 and 0.1 % (w/w) Triton X-100.

The mixture is denaturated for 3 min at 95 °C. Then 40 PCR cycles for 45 sec at 94 °C, 45 sec at 50 °C and 60 sec at 72 °C follow. After further incubation for 20 min at 72 °C, the mixture is cooled to 4 °C. An aliquot of 2 µl is subjected to agarose gel electrophoresis. An aliquot of 2 µl is subjected to agarose gel electrophoresis.

The PCR amplificate is purified with PCR purification kit from Qiagen as described in Example 1. 1.2 µg of purified PCR product are obtained. 2.0 µg of the vector pQE30 and 1.5 µg of the purified PCR product are digested in order to produce DNA fragments with overlapping ends. Each restriction mixture contains 7 µl of NEB3 buffer from New England Biolabs (NEB), 40 U of BamH/ (NEB), 30 U of *Pst*I (NEB) in a total volume of 70 µl and is incubated for 3 h at 37 °C. Digested vector DNA and PCR product are purified using the PCR purification kit from Qiagen.

20 ng of vector DNA and 8 ng of PCR product are ligated together with 1 U of T4-Ligase (Gibco), 2 µl of T4-Ligase buffer (Gibco) in a total volume of 10 µl, yielding the plasmid pQEygbParakom. The ligation mixture is incubated over night at 4 °C. 2 µl of the ligation mixture is transformed into electrocompetent *E. coli* XL1-Blue cells as described in example 1. The electrocompetent cells are prepared as described in example 1.

The DNA insert of the plasmid pQEygbParakom is sequenced as described in example 1 and is not identical to the caclulated cDNA sequence of the database entry (gb AC004136). The DNA and the corresponding amino acid sequence of the full-length *ygbP* gene of A. *thaliana* is shown in Annex Db.

### Example 4

### Preparation and purification of recombinant YgbP protein of E. coli

0.5 liter of Luria Bertani (LB) medium containing 90 mg of ampicillin are inoculated with 10 ml of an overnight culture of *E. coli* strain XL1-Blue harboring plasmid pNCOygbP. The culture is grown in a shaking culture at 37 °C. At an optical density (600 nm) of 0.7, the culture is induced with 2 mM IPTG. The culture is grown for further 5 h. The cells are harvested by centrifugation for 20 min at 5,000 rpm and 4 °C. The cells are washed with 50 mM Tris hydrochloride pH 8.0 , centrifuged as above and frozen at -20 °C for storage.

The cells are thawed in 10 ml of 20 mM tris hydrochloride pH 8.0 containing 1 mM dithioerythritol, 0.02 % sodium azide (buffer A) in the presence of 4 mg/ml lysozyme and 10 *µ*g/ml DNasel. The mixture is incubated at 37 °C for 1 h, cooled on ice and sonified 6 × 10 sec with a Branson Sonifier 250 (Branson SONIC Power Company, Danbury, USA) set to 70 % duty cycle output, control value of 4 output. The suspension is centrifuged at 15, 000 rpm at 4 °C for 30 min. The supernatant is applied on a column of Sepharose Q FF (size 4.6 × 24 cm, Amersham Pharmacia Biotech, Freiburg, Germany) previously equilibrated with 200 ml buffer A. The column is washed with buffer A monitoring at 280 nm. 4-Diphosphocytidyl-2C-methyl-D-erythritol synthase is eluted from the column with a gradient from 0-0.5 M sodium chloride in 300 ml of buffer A. The enzyme is identified by SDS-PAGE showing a band at 26 kDa. Fractions showing this protein band are collected and dialysed against buffer A overnight. The enzyme is further purified on a column of Red Sepharose CL-6B (size 2.6 × 10 cm, Amersham Pharmacia Biotech) equilibrated with buffer A. The enzyme is passed throughout the column which is loaded on Source 15Q (column volume 20 ml, Amersham Pharmacia Biotech). The enzyme is eluted by gradient of 0-0.5 M sodium chloride in 250 ml buffer A. The homogeneity of 4-diphosphocytidyl-2C-methyl-D-erythritol synthase is judged by SDS-PAGE.

### Example 5

Preparation and purification of recombinant YgbP protein of *A. thaliana* without leader sequence.

Cells of *E. coli* strain XL1-Blue harboring the plasmid pNCOygbPara were grown, induced and harvested as described in example 3. The cells (2g) are suspended in 30 ml of buffer A (50 mM Tris HCl pH 8.0, 1 mM DTE, 0.02% sodium azide) in the presence of 12 mg of lysozyme, 1.2 mg of DNasel. The suspension is incubated at 37 °C for 30 min. The extraction is performed by ultrasonification as described in example 4. Cell debris is got rid by centrifugation at 15,000 rpm for 30 min. The cell free extract is loaded on Sepharose Q FF (size 2.6 × 10 cm) equilibrated with buffer A at flow rate 3 ml/min. The column is washed with buffer A monitoring at OD 280 nm. 4-Diphosphocytidyl-2C-methyl-D-erythritol synthase from *A. thaliana* is eluted by linear gradient of 0-0.5 M NaCl in buffer A. The fractions containing enzyme activity are pooled. The volume of pooled fraction is reduced to ca. 2 ml by ultrafiltration (MWCO 10 kDa, Amicon, USA). The concentrated 4-diphosphocytidyl-2C-methyl-D-erythritol synthase from A. thaliana loaded on Superdex 75 HR 26/60 at flow rate 2 ml/min using buffer A containing 100 mM NaCl as a running buffer. The active fractions are pooled. The elution volume of 4-diphosphocytidyl-2C-methyl-D-erythritol synthase from A. thaliana is 140 ml. The homogeinity of 4-diphosphocytidyl-2C-methyl-D-erythritol synthase from A. thaliana is judged by SDS-PAGE.

### Example 6

### Preparation of D-erythritol 4-phosphate

The sodium salt of D-erythrose 4-phosphate (14.6 mg, 65.7 *µ*mol) is dissolved in methanol (600 *µ*l, 40 % (v/v)). Sodium borohydride is added. The reaction is monitored by detection of the aldehyde according to the method of Stahl (Bollinger, H.R., Brenner , M., Gänshirt, H., Mangold, H.K., Seiler, H., Stahl, E. ad Waldi, D. (1964) IN: Dünnschicht-Chromatographie; Ein Laboratoriumshandbuch (ed. Stahl, E.) Springer Verlag, Berlin, Göttingen; Heidelberg). After consumption of the aldehyde the pH of the solution is adjusted with acetic acid to 4-5. The reaction mixture is lyophillized yielding D-erythritol 4-phosphate as a dry compound.
¹H-NMR (500 MHz, D₂O, pH 7) d (ppm) 1.81 (s, acetate), 3.20-3.55 (m, 1 H), 3.60-3.67 (m, 2H), 3.68-3.71 (m, 1 H), 3.80-3.87 (m, 1 H), 3.88-3.92 (m, 1 H).

### Example 7

### Preparation of D-ribitol 5-phosphate

The sodium salt of D-ribose 5-phosphate (18.5 mg, 67.5 *µ*mol) is dissolved in methanol (600 *µ*l, 40 % (v/v)). Sodium borohydride is added. The reaction is monitored by detection of the aldehyde according to the method of Stahl (Bollinger, H.R., Brenner , M., Gänshirt, H., Mangold, H.K., Seiler, H., Stahl, E. ad Waldi, D. (1964) IN: Dünnschicht-Chromatographie; Ein Laboratoriumshandbuch (ed. Stahl, E.) Springer Verlag, Berlin, Göttingen; Heidelberg). After consumption of the aldehyde the pH of the solution is adjusted with acetic acid to 4-5. The reaction mixture is lyophillized yielding D-ribitol 5-phosphate as a dry compound.
¹H-NMR (500 MHz, D₂O, pH 7) d (ppm) 1.81 (s, acetate), 3.54 (dd, J = 11.9 Hz, J = 7.1 Hz, 1H), 3.63 (t, J = 3.3 Hz, 1 H), 3.69 (dd, J = 11.9 Hz, J = 3.0 Hz, 1 H), 3.73-3.76 (m, 1 H), 3.80-3.87 (m, 2H), 3.93 (ddd, J = 2.9 Hz, J = 5.8 Hz, J = 8.6 Hz, 1H).

### Example 8

### Screening of 4-diphosphocytidyl-2C-methyl-D-erythritol synthase

### 8.1 By Spectrophotometric method

Recombinant 4-diphosphocytidyl-2C-methyl-D-erythritol synthase is tested by a spectrophotometric assay at 340 nm, in which the inorganic pyrophosphate formed from 2-C-methyl-D-erythritol 4-phosphate and CTP is used in a cascade of downstream reactions leading to the reduction of NADP⁺. Reactions mixtures contained 50 mM Tris hydrochloride pH 8.0, 200 *µ*M 2C-methyl-D-erythritol 4-phosphate, 200 *µ*M CTP, 5 mM MgCl₂, 1 mM DTT, 1 *µ*M glucose 1,6-biphosphate, 500 *µ*M UDP-Glucose, 174 *µ*M NADP⁺, 0.125 U of UDP-glucose pyrophosphorylase, 0.16 U of phosphoglucomutase, and 1 U of glucose 6-phosphate deydrogenase, various concentrations of D-erythritol 4-phosphate respectively D-ribitol 5-phosphate as shown in Table 4 and 10 *µ*l of enzyme in a total volume of 1 ml. One unit of enzyme activity is defined as the amount of enzyme catalyzing the conversion of 1 *µ*mol of substrate per min at 37 °C. The results are shown in Table 4.

**Table 4. Inhibition of YgbP by D-erythritol 4-phosphate and D-ribitol 5-phospate**

| test compount concentration (mM) | D-erythritol 4-phosphate specific activity (µmol min⁻¹ mg⁻¹) | D-ribitol 5-phosphate specific activity (µmol min⁻¹ mg⁻¹) |
|---|---|---|
| 0 | 17,2 (100 %) | 16,1 (100 %) |
| 0,2 | 15,9 (92 %) | 16,5 (102 %) |
| 0,4 | 15,9 (92 %) | n.d.^{a} |
| 0,8 | 12,9 (75 %) | n.d. |
| 1,6 | 5,9 (34 %) | 16,9 (105) |
| 3,2 | 0 (0 %) | n.d. |

| | | |
|---|---|---|
| ^{a} not determined | | |

### 8.2 By Phosphor Imager screening method

Assay mixtures containing 100 mM Tris hydrochloride pH 8.0, 20 mM sodium fluoride, 10 mM MgCl₂, 100 *µ*M CTP, 10 nCi of [2-¹⁴C]2C-methyl-D-erythritol-4-phosphate, various concentrations of D-erythritol 4-phosphate respectively D-ribitol 5-phosphate as shown in Table 4 and YgbP protein are incubated at 37 °C for 20 min. The reaction is terminated by addition of 20 *µ*l of methanol. After centrifugation, aliquots are spotted on Polygram^{®} SIL N-HR thin layer plates (Macherey-Nagel, Düren, Germany) which are developed with a mixture of n-propanol/ethyl acetate/H₂O (6: 1 :3, v/v). The radiochromatogram is monitored and evaluated by a Phosphor Imager (Storm 860, Molecular Dynamics, USA). The Rf value of the product is 0.36. Similar results are obtained as in example 8.1

### 8.3 By Nuclear magnetic resonance (NMR) method

A solution containing 100 mM Tris HCl pH 8.0, 10 mM MgCl₂, 5 mM CTP, 5 mM of 2C-methyl-D-erythritol 4-phosphate, various concentrations of D-erythritol 4-phosphate resp. D-ribitol 5-phosphate as shown in Table 4 and 0.1 mg of YgbP protein from recombinant *E. coli* is incubated at 37 °C for 1 h. The reaction is monitored by ³¹P-NMR. ³¹P-NMR spectra are recorded using a AC 250 spectrometer from Bruker at a transmitter frequency of 101.3 MHz. The chemical shifts are referenced to external 85 % H₃PO₄. The product displayed two ³¹P NMR dubletts at -7.2 ppm and -7.8 ppm. Similar results are obtained as in example 8.1

### Example 9

### Enzymatic production of 4-diphosphocytidyl-2C-methyl-D-erythritol.

A solution containing 100 mM Tris HCl pH 8.0, 10 mM MgCl₂, 10 mM CTP, 0.12 *µ*Ci of [2-¹⁴C]2C-methyl-D-erythritol 4-phosphate, 46 mM of 2C-methylerythritol 4-phosphate and 225 *µ*g of YgbP protein from recombinant E. coli is incubated at 37 °C for 1 h. The reaction is monitored by ³¹ P-NMR. The product displaying two ³¹ P NMR dubletts at -7.2 ppm and -7.8 ppm is purified by HPLC on a column of the anionic exchanger Nucleosil 10SB (4.6 × 250 mm) using 0.1 M ammonium formate in 40 % (v/v) methanol as eluent at a flow rate of 1 ml/min. The eluent is monitored by a UV-diode array detector (J&M TIDAS) and a radiomonitor from Berthold. 4-Diphosphocytidyl-2C-methyl-D-erythritol is eluted at 30 ml. The fraction containing 4-diphosphocytidyl-2C-methyl-D-erythritol is collected and lyophyllized. The residue is dissolved in 0.5 ml of deuterated water and subjected to NMR analysis.

### Example 10

### Identification of 4-diphosphocytidyl-2C-methyl-D-erythritol.

¹H NMR and ¹H decoupled ¹³C NMR spectra are recorded using a AVANCE DRX 500 spectrometer from Bruker, Karlsruhe, Germany. The frequencies are 500.1 MHz and 125.6 MHz for ¹H and ¹³C, respectively. The chemical shifts are referenced to external trimethylsilylpropane sulfonate. Two-dimensional correlation experiments (gradient enhanced double quantum filtered COSY, HMQC) are performed using XWINNMR software from Bruker. ³¹P NMR spectra are recorded using a AC 250 spectrometer from Bruker at a transmitter frequency of 101.3 MHz. The chemical shifts are referenced to external 85 % H₃PO₄.

The structure of the product is evaluated by a multinuclear multidimensional NMR approach (Table 5). Specifically, the compound is characterized by two ³¹P NMR signals at -7.2 ppm and -7.8 ppm (dubletts with ³¹ P-³¹ P coupling constants of 20 Hz, each). A ³¹P NMR signal for the substrate 2-C-methylerythritol 4-phosphate (singlet at 4.9 ppm) is absent. The detected ³¹P NMR chemical shift range, as well as the ³¹ P- ³¹P couplings implied that the unknown compound is a pyrophosphate. For comparison, the ³¹ P NMR signals of cytidine diphosphate (CDP) are found as dubletts at -5.1 ppm and -7.6 ppm with coupling constants of 21.5 Hz, each (²J_{PP}).

The presence of phosphorous atoms in the unknown compound is further reflected in the ¹³C NMR spectrum where four of 14 signals showed coupling with 31 P (³¹ P-¹³C coupling constants in the range of 9 Hz to 5 Hz).

The ¹H NMR and ¹³C NMR signals are further analyzed by two-dimensional COSY and HMQC experiments. Whereas the detected chemical shifts are different from CDP and 2C-methylerythritol 4-phosphate, the observed correlation patterns in the homonuclear ¹H-¹ H COSY and in the heteronuclear ¹H-¹³C HMQC experiment matched perfectly the correlation signatures of CDP (comprising the spin systems of the ribosyl moiety and the cytosine moiety) and of 2C-methyl-D-erythritol 4-phosphate. This result established the structure of the product as the 4-diphosphocytidyl adduct of 2-C-methyl-D-erythritol.

**Table 5. NMR-data of 4-diphosphocytidyl-2C-methyl-D-erythritol**

| Position | Chemical shifts, ppm | | | Coupling constants, Hz | | | |
|---|---|---|---|---|---|---|---|
| | ¹H | ¹³C | ³¹P | J_{HH} | J_{PH} | J_{PC} | J_{PP} |
| 1 | 3.36 (d, 1H)^{a} | 66.24 (s)^{b} | | 11.7(1*)^{c} | | | |
| 1* | 3.48 (d, 1 H) | | | 11,7(1) | | | |
| 2 | | 73.76 (s) | | | | | |
| 2-Me | 1.02 (s) | 18.13 (s) | | | | | |
| 3 | 3.72 (dd, 1 H) | 73.27 (d) | | 8.3(4), 2.7(4*) | | 7.5 | |
| 4 | 3.85 (ddd, 1 H) | 66.87 (d) | | 11.0(4*), | 6.8 | 5.7 | |
| | | | | 8.3(3) | | | |
| 4* | 4.10 (ddd, 1 H) | | | 11.0(4), 2.7(3) | 6.1 | | |
| 1' | 5.68 (d, 1H) | 89.25 (s) | | 4.1(2') | | | |
| 2' | 4.24 (m, 1H) | 74.21 (s) | | | | | |
| 3' | 4.21 (m, 1H) | 69.09 (s) | | | | | |
| 4' | 4.17 (m, 1 H) | 82.83 (d) | | | | 9.1 | |
| 5' | 4.10 (m, 1 H) | 64.41 (d) | | | | 5.5 | |
| 5'* | 4.17 (m, 1 H) | | | | | | |
| Cyt-2 | | 163.87 (s) | | | | | |
| Cyt-4 | | 170.51 (s) | | | | | |
| Cyt-5 | 6.09 (d, 1H) | 95.99 (s) | | 7.8(Cyt-6) | | | |
| Cyt-6 | 7.96 (d, 1 H) | 142.46 (s) | | 7.8(Cyt-5) | | | |
| P | | | -7.2 (d)^{d} | | | | 19.6 |
| P* | | | -7.8 (d) | | | | 20.4 |

| | | | | | | | |
|---|---|---|---|---|---|---|---|
| ^{a}Referenced to external trimethylsilylpropane sulfonate. The multiplicities and the relative integral values of signals in the ¹H NMR spectrum are given in parentheses. ^{b}Referenced to external trimethylsilylpropane sulfonate. The multiplicities of the ¹H decoupled ¹³C NMR signals are given in parentheses. ^{c}Coupling partners as analysed from two-dimensional COSY experiments are given in parentheses. ^{d}Referenced to external 85 % ortho-phosphoric acid. The multiplicities of the ¹H decoupled ³¹ P NMR signals are given in parentheses. | | | | | | | |

### Example 11

### Production of an expression clone and construction of an expression vector for ychB of E. coli

Chromosomal DNA from *Escherichia coli* strain XLI-Blue is isolated according to a method described by Meade et al. (Meade, H. M., Long, S. R., Ruvkun, C. B., Brown, S. E., and Auswald, F. M. (1982). Physical and genetic characterization of symbiotic and auxotrophic mutants of Rhizobium meliloti induced by transposon Tn5 mutagenis. J. Bacteriol. 149, 114-122).

The *E. coli* ORF *ychB* (accession no. gb AE000219) from basepair (bp) position 5720 to 6571 is amplified by PCR using chromosomal *E. coli* DNA as template. The reaction mixture contained 25 pmol of primer r 5'-GAGGAGAAATTAACCATGCGGACACAGTGGCC-3', 25 pmol of primer 5'-GTCACCGAACTGCAGCTTGCCCG-3', 20 ng of chromosomal DNA, 2U of Taq DNA polymerase (Eurogentec, Seraing, Belgium) and 20 nmol of dNTPs in of 1.5 mM MgCl₂, 50 mM KCl, 10 mM Tris-hydrochloride, pH 8.8 and 0.1 % (w/w) Triton X-100 in a total volume of 100 *µ*l.

The mixture is denaturated for 3 min at 95 °C. Then 25 PCR cycles for 30 sec at 94 °C, 30 sec at 50 °C and 45 sec at 72 °C follow. After further incubation for 7 min at 72 °C, the mixture is cooled to 4 °C. An aliquot of 2 µl is subjected to agarose gel electrophoresis. The PCR amplificate is purified with PCR purification kit from Qiagen. 1.5 *µ*g of purified PCR product are obtained.

The PCR amplificate is used as template for a second PCR reaction. The reaction mixture contained 25 pmol of primer 5'-ACACAGAATTCATTAAAGAGGAGAAATTAACCATG-3', 25 pmol of primer GTCACCGAACTGCAGCTTGCCCG-3', 2 *µ*l of the first PCR amplification, 2U of Taq DNA polymerase (Eurogentec, Seraing, Belgium) and 20 nmol of dNTPs in a total volume of 100 *µ*l of 1.5 mM MgCl₂, 50 mM KCI, 10 mM Tris-hydrochloride, pH 8.8 and 0.1 % (w/w) Triton X-100.

The mixture is denaturated for 3 min at 95 °C. Then 40 PCR cycles for 45 sec at 94 °C, 45 sec at 50 °C and 60 sec at 72 °C follow. After further incubation for 20 min at 72 °C, the mixture is cooled to 4 °C. An aliquot of 2 *µ*l is subjected to agarose gel electrophoresis.

The PCR amplificate is purified with the PCR purification kit from Qiagen as described in example 1 .

2.0 *µ*g of the vector pNC0113 and 1.5 *µ*g of the purified PCR product are digested in order to produce DNA fragments with overlapping ends. Each restriction mixture contained 6 *µ*l of NEB3 buffer, 6 *µ*g of BSA, 30 U of *Eco*RI (NEB), 30 U of *Pstl* (NEB) in a total volume of 60 *µ*l and is incubated for 3 h at 37 °C. Digested vector DNA and PCR product are purified using the PCR purification kit from Qiagen.

20 ng of vector DNA and 18 ng of PCR product are ligated together with IU of T4-Ligase from Gibco-BRL (Eggenstein, Germany), 2 *µ*l of T4-Ligase buffer (Gibco-BRL) in a total volume of 10 *µ*l yielding the plasmid pNCOychB. The ligation mixture is incubated over night at 4 °C. With 2 *µ*l of the ligation mixture electrocompetent *E, coli* XLI-Blue cells are transformed and 100 *µ*l of the cell/DNA suspension is plated on LB plates containing 150 mg/l ampicillin for maintenance of the plasmid pNCOychB. The plasmid pNCOychB is isolated as described before. 9 *µ*g of plasmid DNA are obtained.

The DNA insert of the plasmid pNCOychB is sequenced as described in example 1. The DNA sequence is found to be identical with the sequence in the data base (accession no. gb AE000219).

### Example 12 a

### Cloning of the ychB gene from A. thaliana without leader sequence.

### Arbabidopsis cDNA is prepared as described in example 3.

The resulting cDNA (1 ml of this mixture) is used for the amplification of *ychB* by PCR.

The expression vector pNCO113 is isolated as described in example 1. The *A*. *thaliana* ORF *ychB* without the coding region for the putative leader sequence is amplified by PCR using cDNA from *A. thaliana* as template. The reaction mixture contained 25 pmol of primer 5'-CTGATGAGAGGCTTAATAAGATAGG-3', 25 pmol of primer 5'-TTACATGTTTGTAACATCTCATTGG-3', 1 *µ*g of cDNA, 2 U of Taq DNA polymerase (Eurogentec, Seraing, Belgium) and 20 nmol of dNTPs 1.5 mM MgCl₂, 50 mM KCl, 10 mM Tris-hydrochloride, pH 8.8 and 0.1 % (w/w) Triton X-100 in a total of 100*µ*l.

The mixture is denaturated for 3 min at 95°C. Then 40 PCR cycles for 45 sec at 94 °C, 45 sec at 50 °C and 60 sec at 72 °C followed. After further incubation for 20 min at 72 °C, the mixture is cooled to 4 °C. An aliquot of 2 *µ*l is subjected to agarose gel electrophoresis. The PCR amplificate is purified with the PCR purification kit from Qiagen as described in Example 2. 2.1 µg of purified PCR product are obtained.

The PCR amplificate is used as template for a second PCR reaction. The reaction mixture contained 25 pmol of primer 5'-GTTGACACCATGGCTCCTTTGTCC-3', 25 pmol of primer 5'-TGTTTGTCTGCAGCTCATTGGAAATCC-3', 2 µl of the first PCR amplification, 2U of Taq DNA polymerase (Eurogentec, Seraing, Belgium) and 20 nmol of dNTPs in a total volume of 100 *µ*l of 1.5 mM MgCl₂, 50 mM KCI, 10 mM Tris-hydrochloride, pH 8.8 and 0.1 % (w/w) Triton X-100.

The mixture is denaturated for 3 min at 95 °C. Then 40 PCR cycles for 45 sec at 94 °C, 45 sec at 50 °C and 60 sec at 72 °C follow. After further incubation for 20 min at 72 °C, the mixture is cooled to 4°C. An aliquot of 2 µl is subjected to agarose gel electrophoresis.

The PCR amplificate is purified with PCR purification kit from Qiagen as described in Example 2. 2.4 *µ*g of purified PCR product are obtained. 2 *µ*g of the vector pNCO113 (isolated as described in example 1) and 2.5 *µ*g of the purified PCR product are digested in order to produce DNA fragments with overlapping ends. Each restriction mixture contained 6 *µ*l of NEB3 buffer from New England Biolabs (NEB), 30 U of *Nco*I (NEB), 30 U of *PstI* (NEB) in a total volume of 60 *µ*l and is incubated for 3 h at 37 °C. Digested vector DNA and PCR product are purified using the PCR purifiaction kit from Qiagen.

20 ng of vector DNA and 23 ng of PCR product are ligated together with l U of T4-Ligase (Gibco-BRL), 2 *µ*l of T4-Ligase buffer (Gibco-BRL) in a total volume of 10 *µ*l, yielding the plasmid pNCOychBara. The ligation mixture is incubated over night at 4 °C. 2 µl of the ligation mixture is transformed into electrocompetent *E. coli* XLI-Blue cells as described in example 1. The electrocompetent cells are prepared as described in example 1. The plasmid pNCOychBara is isolated as described before. 6 µg of plasmid DNA are obtained.

The DNA insert of the plasmid pNCOychBara is sequenced as described in example 1. The corresponding protein sequence is identical to the calculated protein sequence of the calculated cDNA squence in the database (gb accession no. AC005168) as shown in Annex Ea.

### Example 12 b

### Construction of an expression vector and production of an expression clone for the full-length ychB gene of A. thaliana

The cDNA of *A. thaliana* is prepared as described in example 2.

The expression vector pQE30 is isolated as described in example 1. The full-length *A. thaliana* ORF *ychB* (accession no. gb AC005168) from basepair (bp) position 82996 to 85396 is amplified by PCR using cDNA from *A. thaliana* as template. The reaction mixture contains 25 pmol of the primer 5'-GGTGACATATCAGATCAAAGAG-3', 25 pmol of primer 5'-TTACATGTTTGTAACATCTCATTGG-3', 1 µg of cDNA, 2 U of Taq-DNA polymerase (Eurogentec, Seraing, Belgium) and 20 nmol of dNTPs in a total volume of 100 µl of 1.5 mM MgCl₂, 50 mM KCI, 10 mM Tris hydrochloride, pH 8.8 and 0.1 % (w/w) Triton X-100.

The mixture is denaturated for 3 min at 95°C. Then 40 PCR cycles for 60 sec at 94 °C, 60 sec at 50 °C and 90 sec at 72 °C follow. After further incubation for 20 min at 72 °C, the mixture is cooled to 4 °C. An aliquot of 2 µl is subjected to agarose gel electrophoresis. The PCR amplificate is purified with the PCR purification kit from Qiagen as described in Example 1. 1.9 µg of purified PCR product are obtained.

The PCR amplificate is used as template for a second PCR reaction. The reaction mixture contains 2 5 pmol of primer 5 '-AGAAACAGGATCCATGGCAACGGCTTCTCCTCCTCC-3', 25 pmol of primer ACACGTCTTCTGCAGAAGTAAATG, 2 µl of the first PCR amplification , 2 U of Taq-DNA polymerase (Eurogentec, Seraing, Belgium) and 20 nmol of dNTPs in a total volume of 100 µl containing 1.5 mM MgCl₂, 50 mM KCI, 10 mM Tris-hydrochloride, pH 8.8 and 0.1 % (w/w) Triton X-100.

The mixture is denaturated for 3 min at 95 °C. Then 40 PCR cycles for 60 sec at 94 °C, 60 sec at 50 °C and 90 sec at 72 °C follow. After further incubation for 20 min at 72 °C, the mixture is cooled to 4 °C. An aliquot of 2 µl is subjected to agarose gel electrophoresis. An aliquot of 2 µl is subjected to agarose gel electrophoresis.

The PCR amplificate is purified with PCR purification kit from Qiagen as described in Example 1. 1.4 µg of purified PCR product are obtained. 2.0 µg of the vector pQE30 and 1.5 µg of the purified PCR product are digested in order to produce DNA fragments with overlapping ends. Each restriction mixture contains 7 µl of NEB3 buffer from New England Biolabs (NEB), 40 U of *BamH*I (NEB), 30 U of *Pstl* (NEB) in a total volume of 70 µl and is incubated for 3 h at 37 °C. Digested vector DNA and PCR product are purified using the PCR purifiaction kit from Qiagen.

20 ng of vector DNA and 12 ng of PCR product are ligated together with 1 U of T4-Ligase (Gibco), 2 µl of T4-Ligase buffer (Gibco) in a total volume of 10 µl, yielding the plasmid pQEychBarakom. The ligation mixture is incubated over night at 4 °C. 2 µl of the ligation mixture is transformed into electrocompetent *E*. *coli* XL1-Blue cells as described in example 1. The electrocompetent cells are prepared as described in example 1.

The DNA insert of the plasmid pQEychBarakom is sequenced as described in example 1. The DNA and the corresponding amino acid sequence of the full-length *ychB* gene of *A. thaliana* is shown in Annex Eb.

### Example 12 c

### Construction of a synthetic gene and an expression for ychB of Lycopersicon esculentum (tomato) without leader sequence.

A cDNA library of tomato leafs is prepared as described by Schmid J. *et al.,* 1992 (Schmid J., Schaller A., Leibinger U., Boll W. and Amrhein, N. (1992). The in-vitro synthesized tomato shikimate kinase precursor is enzymatically active and is imported and processed to the mature enzyme by chloroplasts. The Plant Journal 2(3), 375-383).

In order to adapt the codon usage for high level expression in *E. coli* a synthetic gene coding for the putative tomato YchB protein (accession no. gb U62773, bp position 78 to 1283) was constructed by 8 consecutive PCR reactions using the cDNA library of tomato as template. The oligonucleotides used and the resulting DNA sequence of this gene are shown in Annex Ec.

### Step 1

A part of the *L. esculentum* ORF *ychB* is amplified by PCR using cDNA *from L. esculentum* (from leaf) as template. The reaction mixture contains 25 pmol of primer TM-YCHB-A 5'-GGTACAGACAATTACTTTTGGATTCATC-3', 25 pmol of primer TM-YCHB-B 5'-AAGAGATGGAAGAACTTCAAAGGCAGGAGG-3', 1 µl of cDNA library, 1 U of Vent DNA polymerase (New England Biolabs, Schwalbach, Germany), 20 nmol of dNTPs in a total volume of 100 µl containing 10 mM KCl, 10 mM (NH₄)₂SO₄, 2 mM MgSO₄, 0.1 % Triton X-100, 20 mM Tris hydrochloride, pH 8.8.

The mixture is denaturated for 5 min at 95 °C. Then 20 PCR cycles for 30 sec at 95 °C, 30 sec at 48 °C and 45 sec at 72 °C follow. After further incubation for 3 min at 72 °C, the mixture is cooled to 4 °C. The total mixture is subjected to an 2 % agarose gel electrophoresis. The PCR product with 447 bp is excised from the gel and is purified using the PCR purification kit from Quiagen as described in example 1.

### Step 2

20 ng of the PCR product from step 1 is used as template for a second PCR. The reaction mixture contains 25 pmol of primer TM-YCHB-1 5'-CTGATTATCAAAGCCCTCAATCTTTATCGTAAAAAGACCGGTACAGACAATTA CTTTTGGATTCATC-3', 25 pmol of primer TM-YCHB-2 5'-GACCGCGGC CAGCAGCAATTACACGTTGTTTTAAACGTTTAAGAGATGGAAGAACTTCAAAG CAGGAGG-3', 20 ng of the purified product of the first PCR, 1 U of Vent DNA polymerase (New England Biolabs, Schwalbach, Germany), 20 nmol of dNTPs in a total volume of 100 µl containing containing 10 mM KCl, 10 mM (NH₄)₂SO₄, 2 mM MgSO₄, 0.1 % Triton X-100, 20 mM Tris hydrochloride, pH 8.8.

The mixture is denaturated for 5 min at 95 °C. Then 20 PCR cycles for 30 sec at 95 °C, 30 sec at 48 °C and 45 sec at 72 °C follow. After further incubation for 3 min at 72 °C, the mixture is cooled to 4 °C. The total mixture is subjected to an 2 % agarose gel electrophoresis. The PCR product with 525 bp is excised from the gel and is purified using the PCR purification kit from Quiagen as described above.

### Step 3

20 ng of the PCR product from step 2 is used as template for a 3. PCR. The reaction mixture contains 25 pmol of primer TM-YCHB-3 5'-ACTAATGTTGCTGGCGTTCCACTCGATGAGCGTAATCTGATTATCAAAGCCCT CAATCTTTATCG-3', 25 pmol of primer TM-YCHB-4 5'-TGTGCTGCCACTACCAGACATGAAGACTGCATCATATTGACCGCGGCCAGCA GCAATTACACG-3', 20 ng of the purified product of the first PCR, 1 U of Vent DNA polymerase (New England Biolabs, Schwalbach, Germany), 20 nmol of dNTPs in a total volume of 100 µl containing 10 mM KCI, 10 mM (NH₄)₂SO₄, 2 mM MgSO₄, 0.1 % Triton X-100, 20 mM Tris hydrochloride, pH 8.8.

The mixture is denaturated for 5 min at 95 °C. Then 20 PCR cycles for 30 sec at 95 °C, 30 sec at 48 °C and 45 sec at 72 °C follow. After further incubation for 3 min at 72 °C, the mixture is cooled to 4 °C. The total mixture is subjected to an 2 % agarose gel electrophoresis. The PCR product with 599 bp is excised from the gel and is purified using the PCR purification kit from Quiagen as described above.

### Step 4

20 ng of the PCR product from step 3 is used as template for a 4. PCR. The reaction mixture contains 25 pmol of primer TM-YCHB-5 5'-AAAATTAAGTTCTCGCTGTCACCATCGAAATCAAAGGATCGTTTATCTACTAAT GTTGCTGGCGTTCCACTC-3', 25 pmol of primer TM-YCHB-6 5'-CATAGACAAATTGTGGCGATCTGGAGAGCCAACACCTACGATTGTGCTGCCA CTACCAGACATGAAG-3', 20 ng of the purified product of the first PCR, 1 U of Vent DNA polymerase (New England Biolabs, Schwalbach, Germany), 20 nmol of dNTPs in a total volume of 100 µl containing 10 mM KCI, 10 mM (NH₄)₂SO₄, 2 mM MgSO₄, 0.1 % Triton X-100, 20 mM Tris hydrochloride, pH 8.8.

The mixture is denaturated for 5 min at 95 °C. Then 20 PCR cycles for 30 sec at 95 °C, 30 sec at 48 °C and 45 sec at 72 °C follow. After further incubation for 3 min at 72 °C, the mixture is cooled to 4 °C. The total mixture is subjected to an 2 % agarose gel electrophoresis. The PCR product with 690 bp is excised from the gel and is purified using the PCR purification kit from Quiagen as described above.

### Step 5

20 ng of the PCR product from 4 is used as template for a 5. PCR. The reaction mixture contains 25 pmol of primer TM-YCHB-7 5'-GACGGTTATCATGATCTGGCGTCTCTCTTTCATGTAATTAGTCTTGGCGATAA AATTAAGTTCTCGCTGTCACC-3', 25 pmol of primer TM-YCHB-8 5'-TGCTTCTGACAAGAAGACATCTTTGTACTCTTCGTCATCATAGACAAATTGTG GCGGATCTGG-3', 20 ng of the purified product of the first PCR, 1 U of Vent DNA polymerase (New England Biolabs, Schwalbach, Germany), 20 nmol of dNTPs in a total volume of 100 µl containing 10 mM KCI, 10 mM (NH₄)₂SO₄, 2 mM MgSO₄, 0.1 % Triton X-100, 20 mM Tris hydrochloride, pH 8.8.

The mixture is denaturated for 5 min at 95 °C. Then 20 PCR cycles for 30 sec at 95 °C, 30 sec at 48 °C and 60 sec at 72 °C follow. After further incubation for 3 min at 72 °C, the mixture is cooled to 4 °C. The total mixture is subjected to an 2 % agarose gel electrophoresis. The PCR product with 779 bp is excised from the gel and is purified using the PCR purification kit from Quiagen as described above.

### Step 6

20 ng of the PCR product from step 5 is used as template for a 6. PCR. The reaction mixture contains 25 pmol of primer TM-YCHB-9 5'-TTTTCTCCTTGCAAGATTAATGTTTTCCTGCGCATCACAAGCAAACGTGATGA CGGTTATCATGATCTGGCGTCTC-3', 25 pmol of primer TM-YCHB-10 5'-CAACATACCACTCGTTGGCTGGACGAGTGATGAAACTTGCTTCTGACAAGAA GACATCTTTG-3', 20 ng of the purified product of the first PCR, 1 U of Vent DNA polymerase (New England Biolabs, Schwalbach, Germany), 20 nmol of dNTPs in a total volume of 100 µl containing 10 mM KCl, 10 mM (NH₄)₂SO₄, 2 mM MgSO₄, 0.1 % Triton X-100, 20 mM Tris hydrochloride, pH 8.8.

The mixture is denaturated for 5 min at 95°C. Then 20 PCR cycles for 30 sec at 95 °C, 30 sec at 48 °C and 60 sec at 72 °C follow. After further incubation for 3 min at 72 °C, the mixture is cooled to 4 °C. The total mixture is subjected to an 2 % agarose gel electrophoresis. The PCR product with 867 bp is excised from the gel and is purified using the PCR purification kit from Quiagen as described above.

### Step 7

20 ng of the PCR product from step 6 is used as template for a 7. PCR. The reaction mixture contains 25 pmol of primer TM-YCHB-11 5'-CGTGAAGCTGGTCTTTCACGCCTCACTCTTTTTTCTCCTTGCAAGATTAATGTT TTCCTG-3', 25 pmol of primer TM-YCHB-12 5'-CAGGTTGATCACCAATAGTGCTACCTGAAACAGGTTCAACATACCACTCGTTG GCTGGACG-3', 20 ng of the purified product of the first PCR, 1 U of Vent DNA polymerase (New England Biolabs, Schwalbach, Germany), 20 nmol of dNTPs in a total volume of 100 µl containing 10 mM KCl, 10 mM (NH₄)₂SO₄, 2 mM MgSO₄, 0.1 % Triton X-100, 20 mM Tris hydrochloride, pH 8.8.

The mixture is denaturated for 5 min at 95 °C. Then 20 PCR cycles for 30 sec at 95 °C, 30 sec at 48 °C and 60 sec at 72 °C followed. After further incubation for 3 min at 72 °C, the mixture is cooled to 4 °C. The total mixture is subjected to an 2 % agarose gel electrophoresis. The PCR product with 933 bp is cleaved out of the gel and is purified using the PCR purification kit from Quiagen as described above.

### Step 8

20 ng of the PCR product from step 7 is used as template for a 8. PCR. The reaction mixture contains 25 pmol of primer TM-YCHB-13 5'-ATAATAGAATTCATTAAAGAGGAGAAATTAACCATGGATCGTGAAGCTGGTCT TTCACGCCTC-3', 25 pmol of primer TM-YCHB-14 5'-TATTATTATAAGCTTAAGACATGTCAAAAGATGTAGAGAACTCAGGTTGATCA CCAATAGTGCTACC-3', 20 ng of the purified product of the first PCR, 0.5 U of Goldstar-Taq-DNA polymerase (Eurogentec, Seraing, Belgium), 20 nmol of dNTPs in a total volume of 100 µl containing 1.5 mM MgCl₂, 75 mM Tris hydrochloride, pH 9.0; 20 mM (NH₄)₂SO₄ and 0.01 % (w/v) Tween 20.

The mixture is denaturated for 5 min at 95 °C. Then 20 PCR cycles for 30 sec at 95 °C, 30 sec at 48 °C and 60 sec at 72 °C followed. After further incubation for 7 min at 72 °C, the mixture is cooled to 4 °C. An aliquot of 2 µl is subjected to an 2 % agarose gel electrophoresis. The PCR product with 1015 bp is purified using the PCR purification kit from Quiagen as described above.

2 µg of the vector pNCO-SB-H6-ACYC184 (isolated as described in example 1) and 2 µg of the purified PCR product from step 8 are digested in order to produce DNA fragments with overlapping ends. Each restriction mixture contains 10 µl of OPA buffer (10 x; 500 mM Potassium acetate; 100 mM Magnesium acetate; 100 mM ;Tris acetate, pH 7,5) from Amersham Pharmacia Biotech (Freiburg, Germany) and 50 U of *Hind*III (Amersham Pharmacia Biotech, Freiburg, Germany) in a total volume of 100 µl and is incubated for 3 h at 37 °C. After incubation 18 µl OPA buffer and 50 U of *Nco*I (Amersham Pharmacia Biotech, Freiburg, Germany) are added to a total volume of 140 µl. The resulting mixture is incubated for additional 3 h. Digested vector DNA and PCR product are purified using the PCR purification kit from Quiagen as described above.

20 ng of prepared vector DNA and 25 ng of PCR product are ligated together with 1 U of T4-Ligase (Gibco-BRL, Eggenstein, Germany), 4 µl of T4-Ligase buffer (5 x; 250 mM Tris/HCl, pH 7.6; 50 mM MgCl₂; 5 mM ATP; 5 mM DTT; 25 % (w/v) Polyethylenglycol-8000) in a total volume of 20 µl, yielding the plasmid pNCO-HIS6-TM-YCHB. The incubation mixture is incubated over night at 4 °C. Competent *E. coli* XL1-Blue cells (Stratagene, LaJolla, CA, USA) are transformed with the ligation mixture. Cells are selected on LB-plates containing 170 mg/l ampicillin. Competent cells are prepared using the method from Hanahan D. (Hanahan, D. Studies on transformation of *Escherichia coli* with plasmids. J Mol Biol. 1983, 166(4), 557-80.).

The plasmid is isolated as described above. 5 µg DNA are obtained.

The DNA insert of the plasmid pNCO-HIS6-TM-YCHB is sequenced as described in example 1 using the following oligonucleotides as primers: TM-YCHB-A 5'-GGTACAGACAATTACTTTTGGATTCATC-3', TM-YCHB-B 5'-AAGAGATGGAAGAACTTCAAAGGCAGGAGG-3', PNCO-T5 5'-GAGCGGATAACAATTATAATAGATTC-3.' and mRNA5 5'-CTCCATTTTAGCTTCCTTAGCTCCTG-3'. The corresponding protein sequence is identical to the calculated protein sequence of the calculated cDNA sequence in the database (gb accession no. U62773).

### Example 13 a

### Preparation and purification of recombinant YchB protein of E. coli

0.5 liter of Luria Bertani (LB) medium containing 90 mg of ampicillin are inoculated with 10 ml of an overnight culture of *E. coli* strain XL1-Blue harboring plasmid pNCOychB. The culture is grown in a shaking culture at 37 °C. At an optical density (600 nm) of 0.7, the culture is induced with 2 mM IPTG. The culture is grown for further 5 h. The cells are harvested by centrifugation for 20 min at 5,000 rpm and 4 °C. The cells are washed with 50 mM Tris hydrochloride pH 8.0, centrifuged as above and frozen at -20 °C for storage.

The cells are thawed in 10 ml of 20 mM tris hydrochloride pH 8.0 containing 1 mM dithioerythritol, 0.02 % sodium azide (buffer A) in the presence of 4 mg/ml lysozyme and 10 µg/ml DNasel. The mixture is incubated at 37 °C for 1 h, cooled on ice and sonified 6 × 10 sec with a Branson Sonifier 250 (Branson SONIC Power Company, Danbury, USA) set to 70 % duty cycle output, control value of 4 output. The suspension is centrifuged at 15, 000 rpm at 4 °C for 30 min. The supernatant is applied on a column of Sepharose QFF (column volume 30 ml, Amersham Pharmacia Biotech, Freiburg, Germany) previously equilibrated with 150 ml buffer A. The column is washed with buffer A monitoring at 280 nm. YchB protein is eluted from the column with a gradient from 0-0.5 M sodium chloride in 150 ml of buffer A. The enzyme is identified by SDS-PAGE showing a band at 30 kDa. Fractions showing this protein band are collected and added ammonium sulfate to 0.5 M final concentration. The enzyme is further purified on a column of Phenyl Sepharose 6FF (column volume 16 ml, Amersham Pharmacia Biotech) equilibrated with buffer A containing 0.5 M ammonium sulftate. Then the YchB protein is eluted by linear gradient from 0.5- 0 M ammonium sulfate in 100 ml of buffer A. Fractions containing protein are pooled and concentrated to 3 ml by ultrafiltration (MWCO 10 kDa, Amicon, USA). Then the enzyme is further purified on Superdex 75 HR 26/60 equilibrated with buffer A in the presence of 100 mM sodium chloride. The YchB protein is eluted at 165 ml. The homogeneity of the YchB protein is judged by SDS-PAGE.

### Example 13 b

### Preparation and purification of the recombinant 6xHis-YchB fusion protein of tomato

0.5 liter of Luria Bertani (LB) medium containing 90 mg of ampicillin are inoculated with 10 ml of an overnight culture of *E. coli* strain XL1-Blue harboring plasmid pNCO-HIS6-TM-YCHB. The culture is grown in a shaking culture at 37 °C. At an optical density (600 nm) of 0.7, the culture is induced with 2 mM IPTG. The culture is grown for further 5 h. The cells are harvested by centrifugation for 20 min at 5,000 rpm and 4 °C. The cells are washed with 50 mM Tris hydrochloride pH 8.0 , centrifuged as above and frozen at -20 °C for storage.

The cells are thawed in 20 ml of 20 mM imidazole in 100 mM tris hydrochloride pH 8.0 and 0.5 M sodium chloride (standard buffer) in the presence of 1 mg/ml lysozyme and 100 µg/ml DNasel. The mixture is incubated at 37 °C for 30 min, cooled on ice and sonified 6 × 10 sec with a Branson Sonifier 250 (Branson SONIC Power Company) set to 70 % duty cycle output, control value of 4 output. The suspension is centrifuged at 15, 000 rpm at 4 °C for 30 min. The cell free extract of recombinant YchB protein of tomato is applied on a column of Ni²⁺-Chelating sepharose FF (size 2.6 × 6 cm, Amersham Pharmacia Biotech) previously equilibrated with 20 mM imidazole in standard buffer. The column is washed with 100 ml of starting buffer. YchB protein is eluted with a linear gradient of 20-500 mM imidazole in 100 ml of standard buffer. YchB protein containing fractions are combined according to SDS-PAGE and dialysed overnight against 100 mM Tris hydrochloride pH 8.0, 5 mM dithioerythritol, 0.02 % sodium azide. The dialysed YchB protein is loaded on a Mono Q HR 5/5 column (Amersham Pharmacia Biotech). The column is developed with a linear gradient of 0-0.5 M sodium chloride in 60 ml standard buffer. The homogeneity YchB protein is judged by SDS-PAGE. The objected band at 43 kDa is in agreement with the calculated molecular mass. 3 mg of pure enzyme were obtained.

### Example 14

### Screening of YchB enzyme activity

### 14.1 By a radiochemical method using [2-¹⁴C]4-diphosphocytidyl-2C-methyl-D-erythritol as substrate

Assay mixtures, containing 100 mM Tris hydrochloride, pH 8.0, 100 *µ*M ATP, 10 mM MgCl₂, 1 mM DTT, 20 mM sodium fluoride, 10 nCi of [2-¹⁴C]4-diphosphocytidyl-2C-methyl-D-erythritol and YchB protein and are incubated at 37 °C for 30 min. After centrifugation, aliquots are spotted on SIL-NHR thin layer plates which are developed with a mixture of n-propanol/ethyl acetate/H₂O (6: 1 :3, v/v). The radiochromatogram is monitored and evaluated using a Phosphor Imager (Storm 860, Molecular Dynamics, USA). The Rf value of the YchB product is 0.25. This screening method can be carried out in the presence or absence of prospective inhibitors.

### 14.2 By a Nuclear magnetic resonance (NMR) method using 4-diphosphocytidyl-2C-methyl-D-erythritol as substrate

A solution containing 100 mM Tris hydrochloride, pH 8.0, 10 mM MgCl₂, 5 mM ATP, 1 mM DTT, 5 mM of [2,2-Me-¹³C₂]4-diphosphocytidyl-2C-methyl-D-erythritol and 0.1 mg of YchB protein from recombinant *E. coli* is incubated at 37 °C for 1 h. The reaction is monitored by ¹³C-NMR. ¹³C NMR spectra are recorded using a DRX 500 spectrometer from Bruker at a transmitter frequency of 125.6 MHz. The product displayes two intense double dubletts at 81.91 and 16.86 ppm (referenced to external trimethyl silylpropane sulfonate) with coupling constants of 38.9 and 7.4 Hz, and 38.9 and 1.9 Hz, respectively.

### Example 15

### Enzymatic preparation of 4-diphosphocytidyl-2C-methyl-D-erythritol 2-phosphate

A solution, containing 5 mM [2-¹⁴C]4-diphosphocytidyl-2C-methyl-D-erythritol (0.04 *µ*Ci/mmol), 5 mM ATP, 5 mM MgCl₂, 5 mM DTT, 100 *µ*g of purified YchB protein and 100 mM Tris-hydrochloride, pH 8.0 in a total volume of 4 ml is incubated for 2 h at 37 °C. The reaction is monitored by ³¹P- NMR spectroscopy. Then the sample is centrifuged through a Nanosep 10K membrane (PALLGelmann, Roßdorf, Germany). The product displaying ³¹P signals at 0.49, -7.28, and -8.00 ppm (referenced to external 85 % phosphoric acid) is purified by HPLC on a column of the anionic exchanger Nucleosil 10SB (4.6 × 250 mm, Macherey-Nagel, Düren, Germany), equilibrated with 0.1 M ammonium formate in 40 % (v/v) methanol at a flow rate of 1 ml/min. The HPLC system is equipped with a Wellchrom HPLC pump K-1001, a Wellchrom Spectro-Photometer K-2600 (Knauer, Berlin, Germany) and a radiomonitor (Berthold, Wildbad, Germany). After injection of the sample, the column is washed with 30 ml of 0.1 M ammonium formate in 40 % (v/v) methanol. 4-Diphosphocytidyl-2C-methyl-D-erythritol 2-phosphate is eluted at 14 ml by a linear gradient from 0.1 M ammonium formate in 40 % (v/v) methanol to 1 M ammonium formate in 0 % (v/v) methanol in 30 ml. Fractions containing 4-diphosphocytidyl-2C-methyl-D-erythritol 2-phosphate are collected and lyophylized. The residue is dissolved in 0.5 ml of deuterated water and subjected to NMR analysis. The concentration of 4-diphosphocytidyl-2C-methyl-D-erythritol 2-phosphate is 21 mM.

### Example 16

### Identification of 4-diphosphocytidyl-2C-methyl-D-erythritol 2-phosphate

The elucidation of the structure is performed with [2,2-Me-¹³C₂]-, [1,3,4-¹³C₁]- and [1,2,2-Me,3,4-¹³C₅]4-diphosphocytidyl-2C-methyl-D-erythritol 2-phosphate (Table 6).

The ¹H-decoupled ³¹P NMR spectrum of the enzyme product obtained from [2,2-Me-¹³C₂]4-diphosphocytidyl-2C-methyl-D-erythritol displayes two dubletts at -7.28 ppm resp. -8.00 ppm (³¹P-³¹P coupling constant, 20.8 Hz) and a double-dublett at 0.49 ppm (³¹P-¹³C coupling constants, 7.6 Hz and 1.7 Hz). Without ¹H-decoupling the ³¹P NMR signals at -7.28 and -8.00 ppm are broadened whereas the signal at 0.49 ppm is not affected by ¹H coupling. The chemical shifts as well as the observed coupling pattern suggest the presence of a pyrophosphate moiety and a monophosphate moiety located at position 2 of the 2C-methyl-erythritol moiety. More specificallly, scalar coupling between ³¹P and ¹H is expected in the case of a phosphate residue at position 1 or 3. On the other hand, no ³¹P coupling is expected in the case of a phosphate moiety at position 2. Moreover the observed scalar coupling between the ¹³C-2-Methyl and the ³¹P atom of the phosphate group is only compatible with location 2.

The ³¹P-¹³C coupling pattern is further analyzed using a sample obtained from [1,3,4-¹³C₁]4-diphosphocytidyl-2C-methyl-D-erythritol (Table 6). The ¹³C and ¹H NMR signals are assigned by HMQC, HNQC-TOCSY, and INADEQUATE experiments using the sample obtained from [1,2,2-Me,3,4-¹³C₅]4-diphosphocytidyl-2C-methyl-D-erythritol. With these assignments the structure of 4-diphosphocytidyl-2C-methyl-D-erythritol 2-phosphate is established.

**Table 6 NMR data of 4-diphosphocytidyl-2C-methyl-D-erythritol 2-phosphate**

| Position | Chemical shifts, ppm | | | | | Coupling constants, Hz | | | |
|---|---|---|---|---|---|---|---|---|---|
| | ¹H^{a} | ¹³C^{b} | ³¹P^{c} | J_{HH} | J_{PH} | J_{PC} | J_{PP} | J_{CH} | J_{CC} |
| 1 | 3.58 (m, 1) | 65.78^{e} (d) | | | | 3.8^{e} (P-2) | | 1.7^{d} | 39.8^{l} (2) |
| 1* | 3.64 (m, 1) | | | | | | | | |
| 2 | | 81.91^{d} (dd) | | | | 7.4^{d} (P-2) | | | 38.9^{d} (2-Me) |
| 2-Methyl | 1.26(s,3) | 17.92^{d} (dd) | | | | 1.9^{d} (P-2) | | 127.9^{d}, 3.9^{d} | 38.9^{d} (2) |
| 3 | 3.81 (m, 1) | 73.96^{e} (t) | | | | 7.3^{e} (P-2, P-4) | | | |
| 4 | 3.89 (m,1) | 67.16^{e} (d) | | | | 5.7^{e} (P-4) | | | 42.9^{f} (3) |
| 4* | 4.19 (m, 1) | | | | | | | | |
| 1' | 5.87 (d, 1) | | | 4.4 | | | | | |
| 2' | 4.21 (t, 1) | | | 4.9 | | | | | |
| 3' | 4.25 (t, 1) | | | 4.9 | | | | | |
| 4' | 4.17 (m, 1) | | | | | | | | |
| 5' | 4.09 (ddd, 1) | | | 12.2, 5.4 | 3.2 | | | | |
| 6' | 4.17 (m, 1) | | | | | | | | |
| Cyt-2 | | | | | | | | | |
| Cyt-4 | | | | | | | | | |
| Cyt-5 | 6.07 (d, 1) | | | 7.6 | | | | | |
| Cyt-6 | 7.92 (d, 1) | | | 7.6 | | | | | |
| P (2) | | | 0.49^{d} (dd) | | | 1.7^{d} (2-Me), 7.6^{d} (2) | | | |
| P (4) | | | - 7.28^{d} (d) | | | | 20.8 | | |
| P (5') | | | - 8.00^{d} (d) | | | | 20.8 | | |

| | | | | | | | | | |
|---|---|---|---|---|---|---|---|---|---|
| ^{a}Referenced to external trimethylsilylpropane sulfonate. The multiplicities and the relative integral values of signals in the ¹H NMR spectrum are given in parentheses. ^{b}Referenced to external trimethylsilylpropane sulfonate. The multiplicities of the ¹H decoupled ¹³C NMR signals are given in parentheses. ^{c}Referenced to external 85 % ortho-phosphoric acid. The multiplicities of the ¹H decoupled ³¹P NMR signals are given in parentheses. ^{d}observed with [2,2-Me-¹³C₂]4-diphosphocytidyl-2C-methyl-D-erythritol 2-phosphate ^{e}observed with [1,3,4-¹³C₁]4-diphosphocytidyl-2C-methyl-D-erythritol 2-phosphate ^{f}From the spectrum of [1,2,2-Me,3,4-¹³C₅]4-diphosphocytidyl-2C-methyl-D-erythritol 2-phosphate | | | | | | | | | |

### Example 17

### Construction of an expression clone for ygbB from E. coli

The *E. coli* ORF *ygbB* (accession no. gb AE000358) from bp position 6231 to 6754 is amplified by PCR using chromosomal *E. coli* DNA as template. Chromosomal DNA from Escherichia coli strain XL1-Blue is isolated according to a method described by Meade et al., (Meade, H. M., Long, S. R., Ruvkun, C. B., Brown, S. E., and Auswald, F. M. (1982). Physical and genetic characterization of symbiotic and auxotrophic mutants of Rhizobium meliloti induced by transposon Tn5 mutagenis. J. Bacteriol. 149, 114-122).

The reaction mixture contained 10 pmol of primer GAGGAGAAATTAACCAT GCGAATTGGACACGGTTTTG, 10 pmol of primer TATTATCTGCAGCCTTG CGGTTTACCGTGGAGG, 20 ng of chromosomal DNA, 2 U of Taq DNA polymerase (Eurogentec, Seraing, Belgium) and 20 nmol of dNTPs in a total volume of 100 µl of 1.5 mM MgCl₂, 50 mM KCI, 10, mM Tris-hydrochloride, pH 8.8 and 0.1 % (w/w) Triton X-100.

The mixture is denaturated for 5 min at 94 °C. Then 30 PCR cycles for 30 sec at 94 °C, 45 sec at 50 °C and 45 sec at 72 °C followed. After further incubation for 7 min at 72 °C, the mixture is cooled to 4 °C. An aliquot of 2 µl is subjected to agarose gel electrophoresis. The PCR amplificate is used as template for a second PCR reaction. The reaction mixture contained 50 pmol of primer ACACAGAATTCATTAAAGAGGAGAAATTAACCATG, 50 pmol of primer TATTATCTGCAGCCTTGCGGTTTACCGTGGAGG, 2.5 µl of the first PCR amplification , 10 U of Taq DNA polymerase (Eurogentec, Seraing, Belgium) and 100 nmol of dNTPs in a total volume of 500 µl of 1.5 mM MgCl₂, 50 mM KCl, 10 mM Tris-hydrochloride, pH 8.8 and 0.1 % (w/w) Triton X-100. The mixture is apportioned in 5 PCR-tubes.

The mixtures iare denaturated for 5 min at 94 °C. Then 25 PCR cycles for 30 sec at 94 °C, 45 sec at 50 °C and 45 sec at 72 °C followed. After further incubation for 7 min at 72 °C, the mixture is cooled to 4 °C. An aliquot of 2 *µ*l is subjected to a agarose gel electrophoresis.

The PCR amplificates are purified with a PCR purification kit from Qiagen as described in example 1.

4.5 µg of the vector pNCO113 (isolated as described in example 1) and 3.4 µg of the purified PCR product are digested in order to produce DNA fragments with overlapping ends. Each restriction mixture containes 20 *µ*l of NEB3 buffer, 100 U of *Eco*RI (NEB), 100 U of *Pstl* (NEB) in a total volume of 200 *µ*l and is incubated for 3 h at 37 °C. Digested vector DNA and PCR product are purified using the PCR purifiaction kit from Qiagen as described in Example 1.

100 ng of vector DNA and 35 ng of PCR product are ligated together with 1 U of T4-Ligase (Gibco), 2 µl of T4-Ligase buffer (Gibco) in a total volume of 10 µl, yielding the plasmid pNCOygbB. The ligation mixture is incubated for 2 h at 25 °C. 1 µl of the ligation mixture is transformed into electrocompetent *E. coli* XL1-Blue cells as described in Example 1. The electrocompetent cells are prepared as described in Example 1.

### Example 18

### Preparation and purification of recombinant YgbB protein of E. coli

The cell free extract of YgbB protein from *E*. *coli* is prepared identical to the preparation in example 4. The supernatant is applied on a column of Sepharose Q FF (column volume 30 ml, Amersham Pharmacia Biotech, Freiburg, Germany) previously equilibrated with 120 ml of buffer A. The column is washed with 90 ml of buffer A. Then the YgbB protein is eluted with a linear gradient of 0-0.5 M NaCl in 150 ml buffer A. The homogeneity of YgbB potein is judged by SDS-PAGE.

### Example 19

### Screening of YgbB enzyme activity

The YgbB enzyme activity is screened by radiochemical method. Assay mixtures contained 100 mM tris hydrochloride pH 8.0, 10 mM MnCl₂, 14 nCi of [2-¹⁴C]4-diphosphocytidyl-2C-methyl-D-erythritol and 2 *µ*g of YgbB protein from recombinant *E. coli.* They are incubated at 37°C for 30 min. After centrifugation, aliquots are spotted on Sil-NHR thin layer plates which are developed with a mixture of n-propanol/ethyl acetate/H₂O(6:1:3, v/v). The radiochromatogram is monitored and evaluated by Phosphor Imager (Storm 860, Molecular Dynamics,USA). The Rf value of the YgbB product is 0.5. This screening method can be carried out in the presence or absence of prospective inhibitors.

### Example 20 a

### Production of an expression clone and construction of an expression vector for a 6xHis-ygbB fusion protein of E. coli.

The *E. coli* ORF *ygbB* (accession no. gb AE000358) from bp position 6231 to 6754 is amplified by PCR using chromosomal *E. coli* DNA as template. Chromosomal DNA from Escherichia coli strain XL1-Blue is isolated according to a method described in example 2.

The reaction mixture contained 10 pmol of primer GAGAAGGATCCATGCGAATTGGACACGGTTTTGACG, 10 pmol of primer TATTATCTGCAGCCTTGCGGTTTACCGTGGAGG, 20 ng of chromosomal DNA, 2 U of Taq DNA polymerase (Eurogentec, Seraing, Belgium) and 20 nmol of dNTPs in a total volume of 100 *µ*l of 1.5 mM MgCl₂, 50 mM KCI, 10 mM Tris-hydrochloride, pH 8.8 and 0.1 % (w/w) Triton X-100.

The mixture is denaturated for 5 min at 94 °C. Then 30 PCR cycles for 30 sec at 94 °C, 45 sec at 50 °C and 45 sec at 72 °C followed. After further incubation for 7 min at 72 °C, the mixture is cooled to 4 °C. An aliquot of 2 µl is subjected to agarose gel electrophoresis.

The PCR amplificate is purified with a PCR purification kit from Qiagen as described in example 1.

1.0 *µ*g of the vector pQE30, isolated as described in example 1 (Quiagen) and 0.5 *µ*g of the purified PCR product are digested in order to produce DNA fragments with overlapping ends. Each restriction mixture containes 10 *µ*l of NEB3 buffer from New England Biolabs (NEB), 100 U of BamHI (NEB), 100 U of PstI (NEB) in a total volume of 100 *µ*l and is incubated for 3 h at 37 °C. Digested vector DNA and PCR product are purified using the PCR purifiaction kit from Qiagen as described in example 1.

5 fmol of vector DNA and 14 fmol of PCR product are ligated together with 1 U of T4-Ligase (Gibco), 2 µl of T4-Ligase buffer (Gibco) in a total volume of 10 µl, yielding the plasmid pQEygbB. The ligation mixture is incubated for 2 h at 25 °C. 1 *µ*l of the ligation mixture is transformed into electrocompetent *E*. *coli* XL1-Blue cells as described in example 2. The electrocompetent cells are prepared as described in example 1. The plasmid pQEygbB is isolated as described in example 1.12 *µ*g of plasmid DNA are obtained.

The DNA insert of the plasmid pQEygbB is sequenced as described in example 2 and is identical to the sequence in the database (accession no. gb AE000358). The 5'-end of the DNA insert carries the coding region for 6 histidine residues.

### Example 20 b

### Construction of expression vectors and production of expression clones for ygbB of Plasmodium falciparum

The expression vector pQE30 is isolated as described in example 1.

A cDNA library from P. *falciparum* (strain HB3) is prepared using the SuperSrip Plasmid System for cDNA Synthesis and plasmid cloning from Gibco.

The full-length P. *falciparum* ORF *ygbB* (accession no. gb AE001394) from bp position 2617 to 3495 is amplified by PCR using cDNA from *P. falciparum* as template. The reaction mixture contains 25 pmol of primer 5'-TCCATATGGATCCATGTTTTTAAAAGGATACACC-3', 25 pmol of primer 5'-GACCTGCCTGCAGTTATGAATTTTTAGGTATTAAC-3', 1 µg of cDNA, 2 U of Taq DNA polymerase (Eurogentec, Seraing, Belgium) and 20 nmol of dNTPs in a total volume of 100 µl 1.5 mM MgCl₂, 50 mM KCI, 10 mM Trishydrochloride, pH 8.8 and 0.1% (w/w) Triton X-100.

The mixture is denaturated for 3 min at 95°C. Then 30 PCR cycles for 45 sec at 94 °C, 45 sec at 50 °C and 60 sec at 72 °C followed. After further incubation for 20 min at 72 °C, the mixture is cooled to 4 °C. An aliquot of 2 µl is subjected to agarose gel electrophoresis. The PCR amplificate is purified with the PCR purification kit from Qiagen as described in Example 1. 2.2 µg of purified PCR product are obtained.

2.0 µg of the vector pQE30 and 1.5 µg of the purified PCR product are digested in order to produce DNA fragments with overlapping ends. Each restriction mixture contains 7 µl of NEB3 buffer from New England Biolabs (NEB), 40 U of *BamH*I (NEB), 30 U of *Pstl* (NEB) in a total volume of 70 µl and is incubated for 3 h at 37°C. Digested vector DNA and PCR product are purified using the PCR purifiaction kit from Qiagen.

20 ng of vector DNA and 8 ng of PCR product are ligated together with 1 U of T4-Ligase (Gibco), 2 µl of T4-Ligase buffer (Gibco) in a total volume of 10 µl, yielding the plasmid pQEygbBPlaskom The ligation mixture is incubated over night at 4 °C. 2 µl of the ligation mixture is transformed into electrocompetent *E. coli* XL1-Blue cells as described in example 1. The electrocompetent cells are prepared as described in example 1.

The DNA insert of the plasmid pQEygbBPlaskom is sequenced as described in example 1 and is identical to the calculated cDNA sequence of the database entry (gb AE001394). The cDNA sequence and corresponding amino acid sequence of the *ygbB* gene of P. *falciparum* is shown in Annex G.

A N-terminal truncated *ygbB* expression clone of P. *falciparum* ORF *ygbB* was constructed lacking the coding region for the putative leader sequence. The PCR reaction mixture contains 25 pmol of primer 5'-TTATTTGGATCCATGGGTATAAGAATAGGTCAAGG-3', 25 pmol of primer 5'-GACCTGCCTGCAGTTATGAATTTTTAGGTATTAAC-3', 1 µg of cDNA, 2 U of Taq DNA polymerase (Eurogentec, Seraing, Belgium) and 20 nmol of dNTPs in a total volume of 100 µl 1.5 mM MgCl₂, 50 mM KCI, 10 mM Tris hydrochloride, pH 8.8 and 0.1 % (w/w) Triton X-100.

The mixture is denaturated for 3 min at 95 °C. Then 30 PCR cycles for 45 sec at 94 °C, 45 sec at 50 °C and 45 sec at 72 °C followed. After further incubation for 20 min at 72 °C, the mixture is cooled to 4 °C. An aliquot of 2 µl is subjected to agarose gel electrophoresis. The PCR amplificate is purified with the PCR purification kit from Qiagen as described in Example 1. 3.0 µg of purified PCR product are obtained.

2.0 µg of the vector pQE30 and 1.5 µg of the purified PCR product are digested in order to produce DNA fragments with overlapping ends. Each restriction mixture contains 7 µl of NEB3 buffer from New England Biolabs (NEB), 40 U of *BamH*I (NEB), 30 U of *Pstl* (NEB) in a total volume of 70 µl and is incubated for 3 h at 37 °C. Digested vector DNA and PCR product are purified using the PCR purifiaction kit from Qiagen.

20 ng of vector DNA and 6 ng of PCR product are ligated together with 1 U of T4-Ligase (Gibco), 2 µl of T4-Ligase buffer (Gibco) in a total volume of 10 µl, yielding the plasmid pQEygbBPlas The ligation mixture is incubated over night at 4 °C. 2 µl of the ligation mixture is transformed into electrocompetent *E*. *coli* XL1-Blue cells as described in example 1. The electrocompetent cells are prepared as described in example 1.

The DNA insert of the plasmid pQEygbBPlas is sequenced as described in example 1.

### Example 21 a

### Preparation and purification of recombinant 6xHis-YgbB fusion protein of E. coli

Recombinant XL1-Blue cells of *E. coli* containing overexpressed YgbB (N-terminal His-tagged) of E. coli are prepared as in example 12. The cells are thawed in 20 ml of 20 mM imidazole in 100 mM tris hydrochloride pH 8.0 and 0.5 M sodium chloride (standard buffer) in the presence of 1 mg/ml lysozyme and 100 *µ*g/ml DNasel. The mixture is incubated at 37°C for 30 min, cooled on ice and sonified 6×10 sec with a Branson Sonifier 250 (Branson SONIC Power Company) set to 70 % duty cycle output, control value of 4 output. The suspension is centrifuged at 15, 000 rpm at 4 °C for 30 min. The cell free extract of recombinant YgbB protein of *E. coli* is applied on a column of Ni²⁺ -Chelating sepharose FF (column volume 25 ml, Amersham Pharmacia Biotech) previously equilibrated with 20 mM imidazole in standard buffer. The column is washed with 100 ml of starting buffer. YgbB protein is eluted with a linear gradient of 20-500 mM imidazole in standard buffer. YgbB protein containing fractions are combined according to SDS-PAGE and dialysed overnight against 100 mM tris hydrochloride pH 8.0. The dialysed YgbB protein is concentrated by ultrafiltration (MWCO 10 kDa, Amicon, USA.) and applied on Superdex 75 HR 26/60 (Amersham Pharmacia Biotech). The homogeneity YgbB protein is judged by SDS-PAGE. The objected band at 17 kDa is in agreement with the calculated molecular mass. 27 mg of pure enzyme were obtained.

### Example 21 b

### Preparation and purification of the recombinant 6xHis-YgbB fusion protein of P. falciparum

Recombinant cells of strain XL1-pQEygbBPlas containing overexpressed YgbB protein of *P. falciparum* are prepared as described in example 6.

The cells are thawed in 20 ml of 20 mM imidazole in 100 mM tris hydrochloride pH 8.0 and 0.5 M sodium chloride (standard buffer) in the presence of 1 mg/ml lysozyme and 100 µg/ml DNasel. The mixture is incubated at 37 °C for 30 min, cooled on ice and sonified 6 × 10 sec with a Branson Sonifier 250 (Branson SONIC Power Company) set to 70 % duty cycle output, control value of 4 output. The suspension is centrifuged at 15, 000 rpm at 4 °C for 30 min. The cell free extract of recombinant YgbB protein of tomato is applied on a column of Ni²⁺-Chelating sepharose FF (column volume 25 ml, Amersham Pharmacia Biotech) previously equilibrated with 20 mM imidazole in standard buffer. The column is washed with 100 ml of starting buffer. YgbB protein is eluted with a linear gradient of 20-500 mM imidazole in standard buffer. YgbB protein containing fractions are combined according to SDS-PAGE and dialysed overnight against 100 mM Tris hydrochloride pH 8.0. The dialysed YgbB protein is concentrated by ultrafiltration (MWCO 10 kDa, Amicon, USA.) and applied on Superdex 75 HR 26/60 (Amersham Pharmacia Biotech). The homogeneity YgbB protein is judged by SDS-PAGE. The objected band at 22 kDa is in agreement with the calculated molecular mass. 22 mg of pure enzyme are obtained.

### Example 22

### Enzymatic production of 2C-methyl-D-erythritol 3,4-cyclophosphate

A solution of 500 *µ*l containing 100 mM Tris HCl, pH 8.0, 10 mM MnCl₂, 0.12 *µ*Ci of [2-¹⁴C]diphosphocytidyl-2C-methyl-D-erythritol, 46 mM of diphosphocytidyl-2C-methyl-D-erythritol and 225 µg of YgbB protein from recombinant E. coli is incubated at 37 °C for 1 h. The reaction is monitored by ³¹ P-NMR. The product displaying one ³¹ P NMR singlet at +21.7 ppm is purified by HPLC on a column of the anionic exchanger Nucleosil 10SB (4.6 × 250 mm) using 50 mM ammonium formate in 40 % (v/v) methanol as eluent at a flow rate of 1 ml/min. The eluent is monitored by a radiomonitor from Berthold. 2C-methyl-D-eryrithritol 3,4-cyclophosphate is eluted at 10 ml. The fraction containing 2C-methyl-D-eryrithritol 3,4-cyclophosphate is collected and lyophyllized (2.6 mg). The residue is dissolved in 0.5 ml of deuterated water and subjected to NMR analysis.

### Example 23

### identification of 2C-methyl-D-erythritol 3,4-cyclophosphate

¹H NMR and ¹H decoupled ¹³C NMR spectra are recorded using a AVANCE DRX 500 spectrometer from Bruker, Karlsruhe, Germany. The frequencies are 500.1 MHz and 125.6 MHz for ¹H and ¹³C, respectively. The chemical shifts are referenced to external trimethylsilylpropane sulfonate. Two-dimensional correlation experiments (gradient enhanced double quantum filtered COSY, HMQC) are performed using XWINNMR software from Bruker. ³¹ P NMR spectra are recorded using a AC 250 spectrometer from Bruker at a transmitter frequency of 101.3 MHz. The chemical shifts are referenced to external 85 % H₃PO₄.

The structure of the product is evaluated by a multinuclear multidimensional NMR approach (Table 7). Specifically, the compound is characterized by a single ³¹P NMR signal at +21.7 ppm. The detected ³¹P NMR chemical shift range implied that the unknown compound is a pentacyclic monophosphate.

The presence of a phosphorous atom in the unknown compound is further reflected in the ¹³C NMR spectrum where three of five signals showed coupling with ³¹P (³¹P-¹³C coupling constants in the range of 6 Hz to 1 Hz).

More specifically, the ¹³C NMR signal of C2 is a dublett with a ³¹P-¹³C coupling constant of 6.5 Hz being typical for a ³J_{PC} coupling constant. The ³¹P-¹³C couplings for C3 and C4 are smaller (1 .7 resp. 1.1 Hz) reflecting ²J_{PC} coupling constants. Thus the detected ³¹ P-¹³C coupling signature implied a 3,4-cyclophosphate structure.

**Table 7. NMR data of 2C-methyl-D-erythritol 3,4-cyclomonophosphate**

| Position | Chemical shifts, ppm | | | Coupling constants, Hz | | |
|---|---|---|---|---|---|---|
| | ¹H | ¹³C | ³¹P | J_{HH} | J_{PH} | J_{PC} |
| 1 | 3.38 (d, 1H)^{a} | 65.64 (s)^{b} | | 12.0 (1*)^{c} | | |
| 1* | 3.47 (d, 1 H) | | | 12.0(1) | | |
| 2 | | 73.02 (d) | | | | 6.5 |
| 2-Me | 1.09 (s) | 17.73 (s) | | | | |
| 3 | 4.15(m, 1H) | 77.61 (d) | | | | 1.7 |
| 4 | 4.18 (m, 1 H) | 64.96 (d) | | | | 1.1 |
| 4* | 4.34 (ddd, 1 H) | | | 11.2 (4), | 3.8 (3) 7.2 | |
| P | | | +21 .67 (s)^{d} | | | |

| | | | | | | |
|---|---|---|---|---|---|---|
| ^{a}Referenced to external trimethylsilylpropane sulfonate. The multiplicities and the relative integral values of signals in the ¹H NMR spectrum are given in parentheses. *^{b}*Referenced to external trimethylsilylpropane sulfonate. The multiplicities of the ¹H decoupled ¹³C NMR signals are given in parentheses. ^{c}Coupling partners as analysed from two-dimensional COSY experiments are given in parentheses. *^{d}*Referenced to external 85 % ortho-phosphoric acid. The multiplicities of the ¹H decoupled ³¹P NMR signals are given in parentheseses. | | | | | | |

### Example 24

### Screening of YgbB enzyme activity by NMR.

A solution containing 100 mM Tris HCl pH 8.0, 10 mM MnCl₂, 5 mM of 4-diphosphocytidyl-2C-methyl-D-erythritol and 0.1 mg of YgbB protein from recombinant E. coli is incubated at 37 °C for 1 h. The reaction is monitored by ³¹P-NMR. ³¹P NMR spectra are recorded using a AC 250 spectrometer from Bruker at a transmitter frequency of 101.3 MHz. The chemical shifts are referenced to external 85 % H₃PO₄. The screening method is carried out in the presence or absence of prespective inhibitors by measuring the residual starting material and comparing the results.

The product displayed one ³¹ P singlet at +21.7. The enzme activity can therefore also be determined by measuring this signal for determining the amount of product.

### Example 25

### Enzymatic preparation of 2C-methyl-D-erythritol 2,4-cylopyrophosphate

A solution containing 5mg [2-¹⁴C]4-diphosphocytidyl-2C-methyl-D-erythritol (0.02µCi/mmol), 5 mM MgCl₂, 5mM ATP, 5mM DDT, 100*µ*g purified YchB protein, 200 *µ*g purified YgbB protein and 100 mM Tris hydrochloride pH 8.0 in a total volume of 4 ml is incubated for 2 h at 37°C. The reaction is monitored by ¹³C NMR and ³¹P spectroscopy. The solution is passed through a Nanosep 1OK membrane (PALLGemann, Roßdorf, Germany). The product displaying two ³¹ P NMR signals at -7.65 ppm and -11.66 ppm (dubletts, ³¹P-³¹-P coupling contstant, 23.6 Hz) and displaying two intense ¹³C NMR signals at 83.87 ppm is purified by HPLC on a column of the anionic exchanger Nucleosil 10SB (4.6 × 250 mm, Macherey-Nagel, Düren, Germany) using 40% (v/v) methanol containing 0.1 M ammonium formate as eluent at a flow rate of 1 ml/min. 2C- methyl-D-erythritol 2,4-cyclopyrophosphate is eluted at 34 ml. Fractions containing 2C-methyl-D-erythritol 2,4-cyclopyrophosphate are collected and lyophylized. The residue is dissolved in 0.5 ml of deuterated water and subjected to NMR anylysis. The concentration of 2C-methyl-D-erythritol 2,4-cyclopyrophosphate is 18 mM.

### Example 26

### Identification of 2C-methyl-D-erythritol 2,4-cyclopyrophosphate

The elucidation of the structure is performed with [2,2'-Me-¹³C₂]2C-methyl-D-erythritol 2,4-cyclopyrophosphate (Table 8).

¹HNMR and ¹H decoupled ¹³C NMR spectra is recorded using a AVANCE DRX 500 spectrometer from Bruker (Karlsruhe, Germany). The frequencies are 500.1 MHZ and 125.6 Mhz for ¹H and ¹³C, respectively. The chemical shifts are referenced to external trimethylsilylpropane sulfonate. ³¹P NMR spectra are recorded using a AC 250 spectrometer from Bruker at a frequency of 101.3 MHz. The chemical shifts are referenced to external 85% H₃PO₄.

The structure of the product is evalutated by a multinuclear multidimensional NMR approach (tabel 8). Specifically, the compound is characterized by two ¹H decoupled ³¹P NMR signals at -7.65ppm (dublett with ³¹P-³¹P coupling constant of 23.6 Hz) and -11.66 ppm (double-double dublett with 31 P-31 P coupling constant of 23.6 Hz and ³¹P-¹³C coupling constants of 8.5 Hz, respectively). The ³¹P NMR signal at 7.65 ppm is broadened without ¹H decoupling. The detected ³¹P NMR chemical shift range, as well as the ³¹P-³¹P couplings implied that the unknown compound is a pyrophosphate. Moreover, the detected ³¹P-¹³C couplings for the ³¹P NMR signal at -11.66 ppm in conjunction with the missing. ³¹ P-¹H coupling for the signal indicate that one phosphate unit of the pyrophosphate moiety is connected to C-2 of 2C-methyl-D-erythritol. In line with this conclusion. ¹³C-³¹P couplings are observed for the ¹³C NMR signals reflecting C-2 and C-2-methyl.

In conjunction with the observed ¹³C-¹³C couplings (Table 8), these data are the basis of the ¹H and ¹³C NMR signal assignments. The ¹³C signal at 65.72 ppm (reflecting C-4) showed 13C-³¹P coupling suggesting that the pyrophosphate motif is also connected to C-4. The ¹³C NMR assignments are further confirmed by two-dimensional INADEQUATE experiments establishing the ¹³C-¹³C connectivities.

In summary, the ¹H, ¹³C and ³¹P NMR data clearly established the product as 2C-methyl-D-erythritol 2,4-cylopyrophosphate. Th NMR data were in close correspondance to reported data for this compound (Ostrovsky, D., Kharatian, E.; Dubrovsky, T., Ogrel, O., Shipanova, I., and Sibeldina, L. (1992). The ability of bacteria to synthesize a new cyclopyrophosphate correlates with their tolerance to redox-cycling drugs: on a crossroad of chemotherapy, enviromental toxicology and immunobiochemical problems. Biofactors 4 (1), 63-68; 1992; Truner, D.L., Santos, H., Fareleira, P., Pacheco, I., LeGall, J., and Xavier, A.V. (1992). Structure determination of a novel cyclic phosphocompound isolated from Desulfovibrio desulfuricans. Biochem. J. 285, 387-390.)

**Table 8. NMR data of [2,2-Me-¹³C₂]2C-methyl-D-erythritol 2,4-cyclopyrophosphate**

| Position | Chemical shifts, ppm | | | | | Coupling constants, Hz | | | |
|---|---|---|---|---|---|---|---|---|---|
| | ¹H | ¹³C | ³¹_{P} | J_{PH} | J_{PC} | J_{PP} | J_{CH} | J_{CC} | J_{HH} |
| 1 | 3.51 (dt, 1H)^{a} | 66.95 (d)^{b} | | | | | 1.7 | 41.8 (2) | 12.4 (1) |
| 1* | 3.66 (dd, 1 H) | | | | | | 1.8 | | 12.4 (1) |
| 2 | | 83.87 (dd) | | | 8.4 | | | 39.8 (2-Me) | |
| 2-Methyl | 1.31 (dd, 3H) | 16.30 (dd) | | | 5.3 | | 128.4, 4.0 | 39.8 (2) | |
| 3 | 3.98 | 68.42 (dm) | | | n.d. | | | 46.0 (2) | n.d |
| 4 | - | 65.72 (d) | | | 6.6 | | | | n.d. |
| 4^{*} | 4.13 (m, 3H) | | | | | | | | n.d. |
| P (4) | | | -7.65 (d)^{c} | n.d. | | 23.6 | | | |
| P (2) | | | -11.66(dd d) | | 8.5, 5.3 | 23.6 | | | |

| | | | | | | | | | |
|---|---|---|---|---|---|---|---|---|---|
| ^{a}Referenced to external trimethylsilylpropane sulfonate. The multiplicities and the relative integral values of signals in the ¹H NMR spectrum are given in parentheses. ^{b}Referenced to external trimethylsilylpropane sulfonate. The multiplicities of the ¹H decoupled ¹³C NMR signals are given in parentheses. ^{c}Referenced to external 85 % ortho-phosphoric acid. The multiplicities of the ¹H decoupled ³¹ P NMR signals are given in parentheses. | | | | | | | | | |

### Example 27

### Production of an expression clone and construction of an expression vector for 1-deoxy-D-xylulose 5-phosphate synthase of Bacillus subtilis.

The expression vector pNCO1 13 is isolated as described in example 1. Chromosomal DNA from *Bacillus subtilis* strain BR151 (Williams, D.M., Duvall E.J., and Lovett, P.S. (1981). Cloning restriction fragments that promote expression of a gene in Bacillus subtilis. J. Bacteriol. 146(3), 1162-1165.1981) is isolated according to a method described in example 2.

The putative ORF *yqiE* coding for 1-deoxy-D-xylulose 5-phosphate synthase of B. subtilis (accession no. dbj D84432) from basepair (bp) position 193991 to 195892 is amplified by PCR using chromosomal *B. subtilis* DNA as template. The reaction mixture contained 25 pmol of primer TGATCCGCCATGGATCTTTTATCAATACAGG, 25 pmol of primer TTGAATAGAGGATCCCCGCC, 20 ng of chromosomal DNA, 2U of Taq DNA polymerase (Eurogentec, Seraing, Belgium) and 20 nmol of dNTPs in a total volume of 1.5 mM MgCl₂, 50 mM KCI, 10 mM Tris-hydrochloride, pH 8.8 and 0.1 % (w/w) Triton X-100 in a total volume of 100 *µ*l.

The mixture is denaturated for 3 min at 95 °C. Then 30 PCR cycles for 60 sec at 94°C, 60 sec at 50 °C and 120 sec at 72 °C follow. After further incubation for 20 min at 72 °C, the mixture is cooled to 4 °C. An aliquot of 2 µl is subjected to agarose gel electrophoresis. The PCR amplificate is purified with PCR purification kit from Qiagen. 500 *µ*l of buffer PB (Qiagen) are added to 98 *µ*l of PCR reaction mixture and applied to a Quiaquick column and centrifuged for 1 min at 14,000 rpm. The flow through is discarded. 0.75 ml of buffer PE (Qiagen) are loaded on the column and centrifuged as before. The flow through is discarded and the column is centrifuged for an additional 1 min at 14,000 rpm. The column is placed in a clean 1.5 ml eppendorf tube. 50 *µ*l of H₂O (redistilled, sterile) are added to the column and it is centrifuged for 1 min at 14,000 rpm. The flow through contained 1.8 *µ*g of purified PCR product.

2.0 *µ*g of the vector pNC01 13 and 1 .8 *µ*g of the purified PCR product are digested in order to produce DNA fragments with overlapping ends. Each restriction mixture contained 7 *µ*l of *SalI* buffer from (NEB), 7 *µ*g of BSA (NEB), 40 U of *Ncol* (NEB), 30 U of *SalI* (NEB) in a total volume of 70 *µ*l and is incubated for 3 h at 37 °C. Digested vector DNA and PCR product are purified using the PCR purification kit from Qiagen.

20 ng of vector DNA and 34 ng of PCR product are ligated together with 1 U of T4-Ligase from Gibco-BRL (Eggenstein, Germany), 2 *µ*l of T4-Ligase buffer (Gibco-BRL) in a total volume of 10 *µ*l yielding the plasmid pNCODXSBACSU. The ligation mixture is incubated over night at 4 °C. With 2 *µ*l of the ligation mixture electrocompetent *E. coli* XL1-Blue cells are transformed as described in example 2. 6 *µ*g of plasmid DNA pNCODXSBACSU were isolated.

The DNA insert of the plasmid pNCODXSBACSU is sequenced as described in example 1. The sequence is identical with the sequence found in the database (accession no. dbj D84432).

### Example 28

### Production of an expression clone and construction of an expression vector for 1-deoxy-D-xylulose 5-phosphate reductoisomerase of E. coli

The *E. coli* ORF *yaeM* (accession no. gb AE000126) from bp position 9887 to 11083 is amplified by PCR using chromosomal *E. coli* DNA as template. Chromosomal DNA from Escherichia coli strain XL1-Blue is isolated according to a method described in example 2.

The reaction mixture contained 25 pmol of primer GGAGGATCCA TGAAGCAACTCACC, 25 pmol of primer GCGCGACTCTCTGCAGCCGG, 20 ng of chromosomal DNA, 2 U of Taq DNA polymerase (Eurogentec, Seraing, Belgium) and 20 nmol of dNTPs in a total volume of 100 *µ*l of 1.5 mM MgCl₂, 50 mM KCI, 10 mM Tris-hydrochloride, pH 8.8 and 0.1 % (w/w) Triton X-100.

The mixture is denaturated for 3 min at 94 °C. Then 30 PCR cycles for 45 sec at 94 °C, 45 sec at 50 °C and 75 sec at 72 °C followed. After further incubation for 7 min at 72 °C, the mixture is cooled to 4 °C. An aliquot of 2 µl is subjected to agarose gel electrophoresis.

The PCR amplificates are purified with a PCR purification kit from Qiagen as described in example 1.

2.5 *µ*g of the vector pQE30 (Quiagen), isolated as described in example 2, and 2.0 *µ*g of the purified PCR product are digested in order to produce DNA fragments with overlapping ends. Each restriction mixture contains 7 *µ*l of NEB3 buffer, 50 U of *Bam*HI (NEB), 40 U of *Pst*I (NEB) in a total volume of 70 *µ*l and is incubated for 3 h at 37 °C. Digested vector DNA and PCR product are purified using the PCR purification kit from Qiagen as described in example 1.

20 ng of vector DNA and 22 ng of PCR product are ligated together with 1 U of T4-Ligase (Gibco), 2 *µ*l of T4-Ligase buffer (Gibco) in a total volume of 10 *µ*l, yielding the plasmid pQEyaeM. The ligation mixture is incubated overnight at 4 °C. Each 2 *µ*l of the ligation mixture is used for transforming electrocompetent *E*. *coli* XL1-Blue and M15[pREP4] (Zamenhof et al., 1972) cells as described in example 1. The electrocompetent cells are prepared as described in example 1.

12 *µ*g DNA of plasmid pQEyaeM are obtained.

The DNA insert of the plasmid pQEyaeM is sequenced as described in example 2 and is identical with the sequence in the database (accession no. gb AE000126).

### Example 29

### Preparation and purification of recombinant 1-deoxy-D-xylulose 5-phosphate synthase of B. subtilis.

### E. coli XL1-Blue cells harboring the plasmid pNCODXSBACSU are grown, induced, harvested and stored as described in example 1.

2 g of the cells are thawed in 10 ml of 25 mM tris-HCl pH 8.0 containing 1 mM dithioerythritol, 10 mM EDTA and 6 mM phenylmethylsulfonyl fluoride in the presence of 1 mg lysozyme. The mixture is incubated at 37 °C for 0.5 h, cooled on ice and sonified 6 × 10 sec with a Branson Sonifier 250 (Branson SONIC Power Company, Danbury, USA), control value of 4 output. The suspension is centrifuged at 15,000 rpm at 4 °C for 30 min. The supernatant is applied on a column of Sepharose QFF (26 cm³ Amersham Pharmacia Biotech, Freiburg, Germany) previously equilibrated with 200 ml 25 mM tris-HCl pH 8.0 containing 0.5 mM MgCl₂ and 0.03 % sodium azid (buffer A). The column is washed with 60 ml buffer A monitoring the extinction at 280 nm. 1-deoxy-D-xylulose-5-phosphate synthase is eluted from the column with a gradient from 0-1 M sodium chloride in 300 ml of buffer A. The enzyme is identified by SDS-PAGE showing a band at 68 kDa. Fractions showing this protein band are collected and dialysed against buffer A overnight.

The enzyme is further purified on a column of hydroxyl apatite (Macro pep 40 *µ*m (size 2.5 6 cm, Biorad, Munich, Germany) equilibrated with buffer A. The enzyme is eluted by gradient of 01 M potassium phosphate, pH 6.5. The homogeneity of 1-deoxy-D-xylulose-5-phosphate synthase is judged by SDS-PAGE. A prominent band at 67 kDa is visible, which is in agreement to the calaculated molecular mass. The yield of pure 1-deoxy-D-xylulose-5-phosphate synthase is 44 mg.

### Example 30

### Preparation and purification of recombinant 1-deoxy-D-xylulose 5-phosphate reductoisomerase of E. coli.

Recombinant M15[pREP4] cells of *E*. *coli* containing overexpressed 1-deoxy-D-xylulose 5-phosphate reductoisomerase of *E. coli* are prepared identical to the preparation of example 1. The cells are thawed in 20 ml of 20 mM imidazole in 100 mM tris hydrochloride pH 8.0 and 0.5 M sodium chloride (standard buffer) in the presence of 1 mg/ml lysozyme and 100 µg/ml DNasel. The mixture is incubated at 37 °C for 30 min, cooled on ice and sonified 6 × 10 sec with a Branson Sonifier 250 (Branson SONIC Power Company) set to 70 % duty cycle output, control value of 4 output. The suspension is centrifuged at 1 5, 000 rpm at 4 °C for 30 min. The cell free extract of recombinant 1-deoxy-D-xylulose 5-phosphate reductoisomerase of E. coli is applied on a column of Ni^{2 +} -Chelating sepharose FF (column volume 25 ml, Amersham Pharmacia Biotech) previously equilibrated with 20 mM imidazole in standard buffer. The column is washed with 100 ml of starting buffer. 1-Deoxy-D-xylulose-5-phosphate reductoisomerase is eluted with a linear gradient of 20-500 mM imidazole in standard buffer. 1-Deoxy-D-xylulose 5-phosphate reductoisomerase containing fractions are combined according to SDS-PAGE and dialysed overnight against 100 mM tris hydrochloride pH 8.0. The dialysed 1-deoxy-D-xylulose 5-phosphate reductoisomerase is concentrated by ultrafiltration (MWCO 10 kDa, Amicon, USA.) and applied on a Superdex 75 HR 26/60 column (Amersham Pharmacia Biotech). The homogeneity of 1-deoxy-D-xylulose 5-phosphate reductoisomerase is judged by SDS-PAGE. One band at 43 kDa is visible, which is in line with the calculated molecular mass. The yield of pure 1-deoxy-D-xylulose 5-phosphate is 60 mg.

### Example 31

### Determination of 1-deoxy-D-xylulose-5-phosphate synthase activity

### 31.1. By Nuclear Magnetic resonance (NMR)

The assay mixture contains 400 mM tris hydrochloride pH 8.0, 25 mM [2-¹³C]-sodium pyruvate, 50 mM D,L-glyceraldehyde-3 phosphate, 10 mM MgCl₂, 2 mM thiamine pyrophosphate, 1 mM dithiothreitol, 0.5 mM EDTA, 10 % D₂O and 0.8 mg enzyme sample in a total volume of 0.5 ml. The mixture is incubated 3 h at 37 °C. Protein is precipitated by the addition of 0.1 ml 50 % trichloroacetic acid (TCA). After centrifugation a ¹³C-NMR-spectrum (62.9 Mhz, Bruker, Karlsruhe, Germany) is recorded. The turnover is calculated by integration of the 2C-signals of pyruvate and 1-deoxy-D-xylulose 5-phosphate. Pyruvate displays a 2C-signal at 196.5 ppm and a signal at 92.7 ppm which is assigned to the corresponding hydrate. 1-Deoxy-D-xylulose 5-phosphate displays a signal at 212.5 ppm.

### 31.2. By photometric detection (variant A)

The assay mixture contains 200 mM tris hydrochloride pH 8.0, 25 mM sodium pyruvate, 50 mM D,L-glyceraldehyde 3-phosphate (previously neutralized with NaOH), 10 mM MgCl₂, 4 mM thiamine pyrophosphate, 8 mM dithiothreitol and 0.02 mg enzyme sample in a total volume of 25 ml. The mixture is incubated 20 min at 37 °C. 25 ml of 30 % TCA are added. The supernatant is added to a buffer containing 200 mM tris hydrochloride pH 8.0, 1 mM MnSO₄, 0.5 mM NADPH in a total volume of 0.95 ml. The extinction at 340 nm is determined. A solution (50 ml, 0.1 U) of 1-deoxy-D-xylulose 5-phosphate reductoisomerase is added and the mixture is incubated 30 min at 37 °C. The extinction at 340 nm is determined again. The extinction difference is equivalent to the amount of consumed NADPH (e₃₄₀ = 6300 M⁻¹cm⁻¹) which is equivalent to the amount of produced 1-deoxy-D-xylulose-5-phosphate.

### 31 .3. By photometric detection (variant B)

The assay mixture contains 200 mM tris hydrochloride pH 8.0, 5 mM sodium pyruvate, 10 mM D,L-glyceraldehyde-3-phosphate, 1 mM MnSO₄, 1 mM thiamine pyrophosphate, 1 mM dithiothreitol, 0.5 mM NADPH and 1 U of 1-deoxy-D-xylulose-5-phosphate reductoisomerase in a total volume of 1 ml. The mixture is incubated at 37 °C in a thermostated cuvette and the extinction at 340 nm is monitored. The assay is started by the addition of 5 ml enzyme sample. The negative slope of the extinction is equivalent to the rate of the 1-deoxy-D-xylulose-5-phosphate synthase reaction.

### Example 32

### Determination of 1-deoxy-D-xylulose-5-phosphate reductoisomerase activity

Assay mixtures contain 100 mM tris hydrochloride pH 8.0, 1 mM MnCl₂, 0.5 mM NADPH and 5 µg enzyme sample in a total volume of 1 ml. The mixture is incubated at 37 °C in a thermostated cuvette and the reaction is monitored spectrophotometically at 340 nm. The assay is started by the addition of 10 ml of 50 mM 1-deoxy-D-xylulose-5 phosphate. The negative slope of the extinction is equivalent to the rate of the 1-deoxy-D-xylulose-5-phosphate reductoisomerase reaction.

### Example 33

### Comprehensive enzymatic synthesis of [2,2'-Me-¹³C₂]4-diphosphocytidyl-2C-methyl-D-erythritol.

Step a) Enzymatic synthesis of [1,2-¹³C₂]1-deoxy-D-xylulose 5-phosphate. Crude dihydroxyacetone phosphate is prepared as described by Effenberger, F., and Straub, A. (1987). A novel convinient preparation of dihydroxy acteton phosphate and its use in enzymatic aldol reactions. Tetrahedron Lett. 28, 1641-1644. 1 g of dihydroxyacetone phosphate is dissolved in 70 ml of a solution of 57 mM [2,3-¹³C₂]sodium pyruvate, 10 mM MgSO₄ and 2.5 mM thiaminepyrophosphate in 150 mM Tris hydrochloride, pH 8.0. 17000 units of triose phosphate isomerase (rabbit muscle) are added and the solution is incubated 105 min at 37°C. 0.774 ml (7.4 units) of recombinant 1-deoxyxylulose 5-phosphate synthase from B. subtilis are added. The reaction is monitored as described in example 17. After 8 h the reaction is stopped by adjusting the pH to a value of 3 by addition of 1 M HCl (11.2 ml). The reaction mixture is stored at -20°C.

Step b) Enzymatic synthesis of [2,2'-Me-¹³C₂]-2C-methyl-D-erythritol-4 phosphate.
To the reaction mixture obtained in step a, containing [1,2-¹³C₂]1-deoxy-D-xylulose 5-phosphate, 19 ml of 1 M Tris-buffer, pH 8.0, 1.1 ml of 1M MgCl₂-solution, 3 g glucose (72 mmol) and 6 ml of solution of 0.1 M MnCl₂ are added and the pH is adjusted to 8.0 with (7 ml 1 M NaOH). Precipitate is separated by centrifugation. To a final volume of 200 ml, water, 250 units of glucose dehydrogenase from B. megaterium and 56,6 mg NADP⁺ (80 *µ*mol) are added. After 5 min of preincubation at 37 °C, 2 ml (11.2 units) of recombinant 1-deoxy-D-xylulose-5 phosphate reductoisomerase from E. coli are added. After ca. 30 h the reaction is stopped by the addition of 8 ml of 2 N HCl. The reaction mixture is stored at -20°C.

Step c) Enzymatic synthesis of [2,2-Me-¹³C_{2]}-4-diphosphocytidyl-2C-methyl-D-erythritol.
The pH of the reaction mixture obtained in step b, containing [2,2-Me-¹³C₂]1-methyl-D-erythritol 4-phosphate, is adjusted to 7.0 by addition of 4 ml 2 M NaOH. 1.4 g of CTP (2,5 mmol) are added and the pH is adjusted to 8.0 with 6 ml 2 N NaOH. After 5 min of preincubation at 37°C, 1.5 ml (51.8 units) of YgbP protein from E. coli solution are added. The reaction is monitored as described in, example 7. After ca. 5 h the reaction mixture is purified and lyophylized as described in example 8. 550 mg of pure 4-diphosphocytidyl-[2,2'-¹³C₂]-methyl-D-erythritol are obtained.

### Example 34

### Enzymatic synthesis of 4-diphosphocytidyl-[2,2'-Me-¹³C₂]2C-methyl-D-erythritol in a one vial reaction

A reaction mixture containing 3 g glucose, 1 g of dihydroxyacetone phosphate (5,7 mmol), 1.4 g of CTP (2,5 mmol), 0.45 g of 2,3-¹³C₂- sodiumpyruvate (4 mmol), 56,6 mg NADP⁺ (80 *µ*mol), in 150 mM Tris hydrochloride, pH 8.0 is prepared. 17000 units of triose phosphate isomerase, 250 units of glucose dehydrogenase, 7 units of 1-deoxyxylulose 5-phosphate synthase, 13 units of 1-deoxy-D-xylulose-5 phosphate reductoisomerase and 55 units of YgbP protein are added. To a final volume of 200 ml, 10 mM MgCl₂, 10 mM MnSO₄, 2.5 mM thiamine pyrophosphate in 150 mM Tris hydrochloride, pH 8.0 are added. The pH is adjusted to 8.0 with 5 ml 1 M NaOH. The reaction mixture is incubated at 37°C. The reaction is monitored as described in example 7. After 30 h the reaction mixture is purified and lyophylized as described in example 8. 490 mg of pure 4-diphosphocytidyl-[2,2-Me-¹³C_{2]}-methyl-D-erythritol are obtained.

### Example 35

### Preparation synthesis of 2C-methyl-D-erythritol 4-phosphate (large scale up)

This preparation can be performed with any ¹³C-labeled sample of glucose or pyruvate as starting materials. In this example it is described for [U-¹³C₆]glucose and [2,3-¹³C₂]pyruvate.

Step A) preparative synthesis of [U-¹³C₅] 1-deoxy-D-xylulose 5-phosphate A reaction mixture containing 166 mg [U-¹³C₆]glucose (0.89 mmol), 44 mg thiamine pyrophosphate, 1.02 g of ATP (1.79 mmol), 200 mg of [2,3-¹³C₂]pyruvate (1.79 mmol), 6 mM MgCl₂ in 150 mM Tris hydrochloride, pH 8.0 is prepared. 410 units of triose phosphate isomerase (from rabbit muscle, Type III-S, E. C. 5.3.1.1., Sigma), 360 U hexokinase (from Bakers Yeast, Type VI, E. C. 2.7.1.1, Sigma), 50 U phosphoglucose isomerase (from Bakers Yeast, Type III, E. C. 5.3.1.9, Sigma), 20 U phosphofructokinase (from *Bacillus stearothermophilus,* Type VII, E. C. 2.7.1.11, Sigma), 35 U aldolase (from rabbit muscle, E C. 4.1.2.13, Sigma) and 2 U recombinant DXP synthase from *B*. *subtilis* are added to a final volume of 58 ml. The reaction mixture is incubated at 37°C overnight. During the reaction the pH is hold at a constant value of 8.0 by the addition of 1 M NaOH (2 ml). The reaction is stopped by adding of 3 ml of 2 N hydrochloric acid. ¹³C-NMR-spectra are recorded for monitoring the conversion (Table 9).

**Table 9. NMR data of [U-¹³C₅]1-deoxy-D-xylulose 5-phosphate**

| Position | Chemical shifts, ppm^{a} | Coupling | constants, Hz |
|---|---|---|---|
| 1 | ¹³C 25.9 | J_{PC} | J_{CC} 41.1 (2), 12.8 (3) |
| 2 | 213.1 | | 41.3 (1), 41.3 (3), 3.1 (5) |
| 3 | 77.0 | | 41.5 (2), 40.2 (4), 12.8 (1) |
| 4 | 70.7 | 6.9 | 43.2 (5), 39.6 (3) |
| 5 | 64.3 | 4.6 | 43.4 (4), 3.1 (2) |

| | | | |
|---|---|---|---|
| ^{a}Referenced to external trimethylsilylpropane sulfonate. | | | |

Step B) Preparative synthesis of [U-¹³C₅]2C-methyl-D-erythritol 4-phosphate To the solution of step A 10 U DXP reductoisomerase, 120 U glucose dehydrogenase (from *Bacillus megaterium,* E. C. 1.1.1.47, Sigma), 0.97 g glucose, 200 mM MgCl₂ and 0.3 mM NADP⁺ are added. The pH is adjusted to 8.0 with 1.5 ml of 4 N sodium hydroxide. After centrifugation the volume is 72 ml. The reaction mixture is incubated at 37 °C overnight. The conversion is monitored by recording ¹³C-NMR-spectra of the accumulating product. (Table 10). The reaction product is purified by HPLC on a column of the anionic exchanger Nucleosil 10 SB (16 × 250 mm) using 0.5 M formic acid as eluent at a flow rate of 13 ml/min. The eluent is monitored by a refractometer (GAT-LCD210 from Gamma Analyse Technik, Bremerhafen, Germany). The product is eluted at 14.5 min. The fraction containing [U-¹³C₅]2C-methyl-D-erythritol 4-phosphate is collected and lyophylized. The amount is 86 mg.

**Table 10. NMR data of [U-¹³C₅]2C-methyl-D-erythritol**

| Position | Chemical shifts, ppm^{a} |
|---|---|
| 1 | 66.5 |
| 2 | 74.1 |
| 2-Methyl | 18.5 |
| 3 | 74.1 |
| 4 | 64.6 |

| | |
|---|---|
| ^{a}Referenced to external trimethylsilylpropane sulfonate. | |

### Example 36

### Enzymatic synthesis of 4-diphosphocytidyl-2C-methyl-D-erythritol 2-phosphate

This preparation can be performed with any ¹³C-labeled sample of 2C-methyl-D-erythritol-4-phosphate as starting material. In this example it is described for [1,3,4-¹³C]2C-methyl-D-erythritol 4-phosphate.

To a reaction mixture, containing 15 mg of purified [1,3,4-¹³C]2C-methyl-D-erythritol 4-phosphate (69 *µ*mol), 34 mg CTP (69 *µ*mol), 16 mg sodium phosphoenol pyruvate (69 *µ*mol), 1,9 mg ATP (3,5 *µ*mol), 10 mg MgCl₂, 5 mM DTT, 10 mM KCI and 150 mM Tris hydrochloride, pH 8.0, 60 *µ*l of YgbP protein (2.1 mg/ml), 200 *µ*l of YchB protein (0.3 mg/ml) and 100 U of pyruvate kinase (from rabbit muscle, Type VII, E. C. 2.7.1.40, Sigma) are added. The final volume is 5 ml. The reaction mixture is incubated at 37 °C for 4 h. The reaction is monitorered as described in example 15.

4-diphosphocytidyl-methyl-D-erythritol 2-phosphate is purified by HPLC on a column of the anionic exchanger Nucleosil 5 SB (7.5 × 1 50 mm) using a gradient of 1 M Amoniumformiat (B) and 100 mM Ammoniumformiat (A) as eluent at a flow rate of 3.1 ml/min.

| t(min) | A(%) | B(%) |
|---|---|---|
| 0 | 100 | 0 |
| 5 | 100 | 0 |
| 35 | 30 | 70 |
| 40 | 30 | 70 |

The eluent is monitored by a UV-detector (Knauer) at 275 nm. 4-diphosphocytidyl-methyl-D-erythritol 2-phosphate is eluted at 26-27 min.

### Preparation Examples for labelled substrates

### Preparation Example 1

### (a) 1,2-O-Isopropylidene-(2R,3RS)-1,2,3-butanetriol (7).

1,2,5,6-Di-O-isopropylidene-D-mannitol (5) (14 g, 53.4 mmol) was dissolved in 200 ml of dry chloroform. Anhydrous potassium carbonate (50.5 g, 366 mmol), was added, and the suspension was cooled to 0 °C. Lead tetraacetate (27.1 g, 61 .1 mmol) was added in small portions under vigorous stirring. The orange coloured suspension was allowed to stand at room temperature over night. Potassium carbonate was filtered off by suction, and the filter cake was washed repeatedly with ether. The combined filtrate and washings were dried with magnesium sulphate and the solvent was removed under reduced pressure. The oil containing the isopropylidene glyceraldehyde was distilled quickly (60 °C at 30-40 mbar) affording 10,5 g (80,7 mmol, 76 %) of pure isopropylidene glyceraldehyde (6). The product was immediately dissolved in 35 ml of dry ether to avoid polymerisation. The solution of isopropylidene glyceraldehyde was added to a cooled solution of methyl magnesium iodide prepared from 5,1 g (207 mmol) of magnesium and 13,0 ml (209 mmol) of methyl iodide in 140 ml ether. After the aldehyde was added completely, the solution was stirred at room temperature over night. The solution was then slowly poured on crushed ice, and precipitated magnesium hydroxide was dissolved by the addition of saturated ammonium chloride (50 ml). The organic layer was removed, and the water phase was saturated with sodium chloride and extracted with chloroform (3 × 50 ml). The combined organic layers were dried with magnesium sulphate, and the solvent was removed under reduced pressure affording 9,9 g (67,8 mmol, 84 %) of 1,2-O-Isopropylidene-(2R,3RS)-1,2,3-butanetriol (8).

¹H NMR (360 MHZ, CDCl₃): δ (ppm) 0.96 (d, ³J = 6.5 Hz), 1,07 (d, ³J = 6.5 Hz), 1.24 (s), 1.25 (2), 1.29 (s), 1.33 (s), 3.41-3.47 (m), 3.67-3.78 (m), 3.82-3.97 (m), 4.67 (d, ³j = 4.6 Hz), 4.75 (d, ³j = 5.2 Hz) (underlined signals belong to the diastereomer which is formed predominantly); ¹³C NMR (90 MHz, CDCl₃): δ (ppm) 1.8.0, 19.8, 24.8, 26.1, 64.3, 65.9, 66.1, 66.9, 79.1, 79.3, 107.7, 107.7 (underlined signals belong to the diastereomer which is formed predominantly; anal. calcd. for C₇H₁₄O₃: C 57.5, H 9.9, O 32.6 found: C 57.2, H 9.9, O 32.8.

### (b) 3,4-O-Isopropylidene-(3R)-3,4-dihydroxy-2-butanone (8)

1:2-O-Isopropylidene-(2R,3RS)1,2,3-butanetriol (7) (9.9 g, 67.8 mmol) was dissolved in 100 ml of chloroform. Water (100 ml), 30 g of potassium carbonate (217 mmol) and 50 mg of ruthenium dioxide hydrate were added. The suspension was stirred vigorously at room temperature, and 29 g (136 mmol) of sodium periodate were added in small portions. When the pH dropped below 7 it was adjusted to pH 8-8.5 with potassium carbonate. After the addition of periodate was complete the suspension was stirred for two days at room temperature. Before work up an aliquot of the reaction mixture was controlled by ¹H NMR spectroscopy. If starting material was still present, an additional amount of periodate was added. When the oxidation was complete the suspension was filtered by suction, and the filtrate was extracted with chloroform (4 × 50 ml). The combined organic layers were dried with magnesium sulphate, and the solvent was removed under reduced pressure affording 7.2 g (50 mmol, 74 %) of 3,4-O-Isopropylidene-(3R)-3,4-dihydroxy-2- butanone (8).
¹H NMR (250 MHz, CDCl₃): δ (ppm) 1.4 (s, 3H), 1.5 (s, 3H), 2.27 (s, 3H), 4.0 (dd, ²J = 8.54Hz, ³J=5.50Hz, 1 H),4.2(dd,²J=8.54Hz,³J=7.95Hz, 1H),4.41 (dd,³J = 7.94Hz, ³J =5.5 Hz, 1H); ¹³C NMR (62 MHz, CDCl₃): δ (ppm) 25.3 (CH₃), 25.6 (CH₃), 26.3 (CH₃), 66.4 (CH₂), 80.4 (CH), 1 10.9 (C_{q}).

### (c) 1,2- O-Isopropylidene-3-O-trimethylsilyl-(2R,3RS)-1,2,3-trihydroxy-3-cyano-butane (9)

3,4-O-Isopropylidene-(3R)-3,4-dihydroxy-2-butanone (8) (7.2 g, 50 mmol) was dissolved in 50 ml of dry dichloromethane. Catalytic amounts of potassium cyanide (20 mg) and 18-crown-6 (20 mg) were added. Under cooling with ice, 9.4 ml (70 mmol) of trimethylsilyl cyanide were added within 20 minutes. The solvent and excess trimethylsilyl cyanide were removed under reduced pressure. The orange coloured oily residue (12.0 g, 49.3 mmol, 99 %) was a mixture of the erythro and threo form of 1,2-O-isopropylidene-3-O-trimethylsilyl-(2R,3RS)-1,2,3-trihydroxy-3-cyano-butane (9) in a ratio of 3: 1 which did not contain significant amounts of other products.

¹H NMR (360 MHz, CDCl₃): δ (ppm) 0.17 and 0.18 (2s,9H), 1.12 (s), 1.29 (s), 1.40 (s), 1.43 (s), 1.46 (s), 1.57 (s) (9H) 3.85-3.90 (m, 1H); 3.97-4.10,(m, 2H); ¹³C NMR (90 MHz, CDCl₃): *erythro* δ (ppm) 1.2 (TMS), 24.0 (CH₃), 25.0 and 26.0 ((CH₃)₂), 65.0 (CH₂), 80.4 (CH), 110.9 (CN); *threo* δ (ppm) -3.1 (TMS), 25.2 (CH₃), 26.2 and 26.4 ((CH₃)₂), 66.4 (CH₂), 80.8 (CH), 120.7 (CN).

### (d) 2C-Methyl-D-erythrono-1,4-lactone (11) and 2C-Methyl-D-threono-1,4-lactone (12).

1,2-O-Isopropylidene-3-O-trimethylsilyl-(2R,3RS)-1,2,3-trihydroxy-3-cyano-butane (9) (12.0 g, 49.3 mmol) was suspended in 30 ml of 25 % hydrochloric acid. Ethanol (10 ml) was added to improve the solubility of the lipophilic cyanohydrin. The reaction mixture was stirred at 45°C for 30 minutes and subsequently under reflux for three hours. The mixture became brown, and a precipitate of ammonium chloride was formed. The acid was neutralized with concentrated ammonia. The mixture was evaporated to dryness. The product mass was triturated with 50 ml of methanol. Insoluble ammonium chloride was filtered off. Methanol was removed under reduced pressure. The residual oil contained the lactones 11 and 12, and the open chain carboxylic acids (10). Lactonisation was brought to completion by boiling the residue with 50 % formic acid (30 ml) for 2 hours. When no more open chain carboxylic acids were present the reaction mixture was concentrated under reduced pressure. The residual oil was dissolved in a mixture of ethyl acetate, 2-propanol and water (5 ml, 65:23.5:11.5, v/v). The solution was placed on a column of silica gel (acidic form) and was developed with the ethyl acetate/2-propanol water mixture. Fractions were combined, and concentrated under reduced pressure. The residue was lyophilised. The residual colourless oil (5.9 g, 44,7 mmol, 91 %) contained 2C-methyl-D-erythrono-1,4-lactone and 2C-Methyl-D-threono-1,4-lactone in a ratio of about 3: 1 as determined by NMR spectroscopy.

*2C-Methyl-D-threono-1,4-lactone* ¹H NMR (250 MHz, CD₃OD): δ (ppm) 1.30 (s, 3H), 3.92 (dd, ²J = 4.27 Hz, ³J = 9.16 Hz, 1H), 4.13 (dd, ²J = 4.27 Hz, ³J = 5.50 Hz, 1 H), 4.44 (dd, ²J = 5.50 Hz, ³J= 9.15 Hz 1 H); ¹³C NMR (63 MHz, CD₃OD): δ (ppm) 17.9 (CH₃), 73.1 (CH₂), 78.8 (CH), 85.8 (Cq), 161.8 (Cq); IR (film): 1770 cm⁻¹; anal. calcd. for C₅H₈O₄: C 45.4, H 6.0, 48.3 found: C 46.2, H 6.5, O 47.3.

*2C-methyl-D-erythrono- 1,4-lactone* ¹H NMR (250 MHz, CD₃OD): δ (ppm) 1.33 (s, 3H), 4.00 (dd, ²J = 1.83 Hz, ³J = 4.27 Hz, 1 H), 4.09 (dd, ²J = 1.83 Hz, ³J = 9.77 Hz, 1 H), 4.38 (dd, ²J = 4.27 Hz, ³J= 10.38 Hz, 1 H); ¹³C NMR (63 MHz, CD₃OD): δ (ppm) 21.9 (CH₃), 73.6 (CH₂), 75.0 (CH), 75.8 (C_{q}), 164.9 (C_{q}); IR (film): 1770 cm⁻¹.

*open chain carboxylic acids (isomeric mixture 1:1)* ¹H NMR (250 MHz, D₂O): δ (ppm) 1.14 (s, 3H), 1,17 (s, 3H), 3,45 - 3,85 (m, 6H); ¹³C NMR (63 MHz, D₂O): δ (ppm) 19.8 (CH₃), 20.7 (CH₃), 64.9 (CH₂), 65.2 (CH₂), 70.1 (CH), 70.3 (CH), 77.8 (Cq), 77.9 (Cq), 182.5 (Cq), 182.8 (Cq).

### (e) 2,3-O-Isopropylidene-2C-methyl-D-erythrono-1,4-lactone (13)

Anhydrous zinc chloride (14.1 g, 103 mmol) was dissolved in 100 ml of acetone. The solution was cooled with ice, and 5.9 g of a mixture of 2C-methyl-D-erythrono-1,4-lactone (11) (33.5 mmol) and 2C-methyl-D-threono-1,4-lactone (12) (11.2 mmol) dissolved in 13 ml acetone was added. After 18 hours the solution was diluted with 150 ml of chloroform. Zinc chloride and unreacted 2C-methyl-D-threono-1,4-lactone were removed by washing with water (3 × 100 ml). The organic layer was dried with magnesium sulphate, and the solvent was removed under reduced pressure affording pure 2:3-O-isopropylidene-2C-methyl-D-erythrono-1,4-lactone (13) (4.4 g, 25.6 mmol, 76 % from 2C-methyl-D-erythrono-1,4-lactone) as a colorless, oil which crystallized at -20°C.

¹H NMR (360 MHz, CDCl₃): δ (ppm) 1.33 (s, 3H), 1.37 (s, 3H), 1.48 (s, 3H), 4.24 (dd, ³J = 3.54 Hz, ²J = 11.06 Hz, 1 H), 4.34 (dd, ²J = 1 1 .06 Hz, ³J = 0 Hz, 1 H), 4.41 (dd, ²J = 3.50 Hz, ³J = 0 Hz, 1 H); ¹³C NMR (90 MHz, CDCl₃): δ (ppm) 18.4 (CH₃), 26.5 (CH₃), 26.9 (CH₃), 68.9 (CH₂), 80.3 (CH), 81.4 (C_{q}), 113.0 (Cq), 176.7 (C_{q}).

### (f) 2,3-O-Isopropylidene-2C-methyl-D-erythrofuranose (14).

2:3-O-Isopropylidene-2Cmethyl-D-erythrono-1,4-lactone (13) (2.2 g, 12.9 mmol) was dissolved in 60 ml of dry tetrahydrofurane. The mixture was cooled to -78°C under an atmosphere of nitrogen. A solution of di-isobutylaluminum hydride (1 M in hexane, 17 ml, 17 mmol) was added slowly. The solution was allowed to stand in the cooling bath over night. Wet ether (180 ml) and wet silica gel (30 g) were added. The mixture was stirred for one hour and was allowed to warm to room temperature. The mixture was then filtered. The solution was dried with magnesium sulphate, and the solvent was removed under reduced pressure. The residual oil was purified by chromatography on silica gel with a mixture of hexane/ethyl acetate (1 :2, v/v) affording 2.0 g (11.5 mmol, 89 %) of 2:3-O-isopropylidene-2C-methyl-D-erythrofuranose (14) as an anomeric mixture (α:β = 1:1).

¹H NMR (360 MHz, CDCl₃): δ (ppm) 1.29 (s), 1.30 (s), 1.34 (s), 1 35 (s), 1.37 (s) (18 H), 3.46 (dd, ³J = 3.54 Hz, ²J = 11.06 Hz, 1 H), 3.55 (m, 1 H), 3.78 (d, ²J = 11.50 Hz, 2H), 3.84 (d, ²J = 11.06 Hz, 1 H), 3.97 (dd, ³J = 3.80 Hz, ²J = 10.40 Hz, 1H), 4.29 (dd, ³J = 3.10 Hz, ³J = 8.85 Hz, 2H), 4.52 (d, ²J = 11.06 Hz, 1 H), 5.13 (d, ³J = 2.65 Hz, 1H); ¹³C NMR (90 MHz, CDCl₃): δ (ppm) 19.4 (CH₃), 21.4 (CH₃), 26.3 (CH₃), 26.9 (CH₃), 27.2 (CH₃), 28.0 (CH₃), 67.1 (CH₂), 71 5 (CH₂), 84.9 (CH), 86.0 (C_{q}), 86.1 (CH), 91.4 (C_{q}), 101.4 (C_{q}), 103.3 (C_{q}), 112.4 (CH), 112.9 (CH).

### (g) 2:3-O-Isopropylidene-2C-methyl-D-erythrose-(O-benzyl)oxime (15).

2:3-O-Isopropylidene-2C-methyl-D-erythrofuranose (14) (0.5 g, 2.87 mmol) was dissolved in 12 ml of dry dichloromethane. Dry pyridine (1 ml) and 0.88 g (5.5 mmol) of O-benzylhydroxylamine hydrochloride were added in one portion. The hydroxylamine dissolved within 20 minutes, and the reaction mixture became turbid after 40 minutes. The mixture was stirred for 15 hours at room temperature and was evaporated to dryness under reduced pressure. The residue was suspended in a mixture of chloroform/ethyl acetate (1 :4, v:v, 1 ml). The solution was placed on a silica gel column (1 cm × 30 cm). The product was eluted with the solvent mixture. Fractions containing 2,3-O-isopropylidene-2C-methyl-D-erythrose-(O-benzyl)oxime were combined and the solvent was removed under reduced pressure affording 0.53 g (1.9 mmol, 66 %) of 2:3-O-isopropylidene-2C-methyl-D-erythrose(O-benzyl)oxime as colourless oil.

¹H NMR (250 MHz, CDCl₃): δ (ppm) 1.26 (s, 3H), 1.31 (s, 3H), 1.33 (s, 3H), 3.42-3.56 (m, 2H), 3.86 (dd, ³J = 4.89 Hz, ³J = 6.72 Hz, 1 H), 4.92 (s, 2H), 7.15-7.25 (m, 5H), 7.32 (s, 1 H); ¹³C NMR (63 MHz, CDC): δ (ppm) 22,8 (CH₃), 26.6 (CH₃), 27.9 (CH₃), 60.7 (CH₂), 76.0 (CH₂), 80.5 (CH), 84.3 (Cq), 109.4 (Cq), 127.9 (CH), 128.2 (CH), 128.3 (CH), 137.2 (Cq), 152.0 (CH).

### (h) 2,3-O-Isopropylidene-2C-methyl-D-erythrose-(O-benzyl)oxime 4-dibenzylphosphate (16).

Tribenzylphosphite (1.3 g, 3.7 mmol) was dissolved in 20 ml of dry dichloromethane. The solution was cooled to -20°C. Iodine (0.96 g, 3.8 mmol) was added in one portion. The mixture was protected from light and was allowed to come to room temperature when the violet color had disappeared. 2:3-O-Isopropylidene-2C-methyl-D-erythrose-(O-benzyl)oxime (15) (0.53 g, 1.9 mmol) was dissolved in 20 ml of dichloromethane, and 2.5 ml pyridine (31.6 mmol) was added. The solution was cooled to -20°C and the solution of dibenzyl iodophosphate was added slowly. The reaction mixture was stirred for 2 hours at room temperature and was washed subsequently with sodium hydrogen sulphate (30 %, w/v, 2 × 10 ml), a solution of sodium hydrogen carbonate (5 %, w/v, 10 ml), and water (10 ml). The organic phase was dried with magnesium sulphate. The solution was evaporated to dryness. The residue was suspended in a mixture of hexane/ethyl acetate (3: 1, v:v, 2 ml). The mixture was placed on a silica gel column (1 cm × 20 cm) which was developed with hexane/ethyl acetate (3 : 1, v/v) until benzyl iodide was completely washed out. The product was then eluted with a mixture of chloroform/ethyl acetate (1 :4, v/v). Fractions were combined. The solvent was removed under reduced pressure affording 0.73 g (1.35 mmol, 71 %) of 2,3-O-isopropylidene-2C-methyl-D-erythrose-(O-benzyl)oxime 4-dibenzylphosphate.

¹H NMR (250 MHz, CDCl₃): δ (ppm) 1.31 (s,3H), 1.37 (s, 3H), 1.39 (s, 3H), 3.90-3.99 (m, 3H), 4.94 (s, 1 H), 4.97-5.02 (m, 6H), 7.24-7.33 (m, 15H); ¹³C NMR (63 MHz, CDCl₃): δ (ppm) 22.0 (CH₃), 26.6 (CH₃), 28.0 (CH₃), 65.3 (d, ²J_{cp}= 5.5 Hz, CH₂), 69.1-69.5 (m, CH₂), 76.2 (CH₂), 80.2 (C_{q}), 82.5 (d, ³J_{CP} = 7.9 Hz, CH), 109.7 (C_{q}), 127.9-128.5 (CH), 135.6 (d, ³J_{CP} = 6.8 Hz, C_{q}), 137.9 (Cq), 150.3 (CH); ³¹P NMR (101 MHz, CDCl₃): δ (ppm) -0.8 (s).

### (i) 2,3-O-Isopropylidene-2C-methyl-D-erythrose 4-dibenzylphosphate (17).

2:3-O-Isopropylidene-2C-methyl-D-erythrose-(O-benzyl)oxime 4-dibenzylphosphate (16) (0.26 g, 0.43 mmol) was dissolved in 15 ml of dichloromethane containing 2 ml of pyridine. The solution was cooled to -78°C and was ozonized for 7 minutes with an ozone flow of about 3 g/min (0.44 mmol). Nitrogen was then bubbled through the dark blue reaction mixture. When the blue color had vanished, 2 ml of dimethylsulfide were added. The mixture was allowed to stand at -78°C for 1 hour and was then brought to room temperature. Solvent and pyridine was removed under reduced pressure, and the crude oil was purified by column chromatography (silica gel; chloroform/ethyl acetate 1/4, v/v) affording 0.17 g (0.39 mol, 81 %) of pure aldehyde.

¹H NMR (360 MHz, CDCl₃): δ (ppm) 1.24 (s, 3H), 1.36 (s, 3H), 1.46 (s, 3H), 3.93-4.02 (m, 2H), 4.05-4.13 (m, 1 H), 4.92-5.00 (m, 4H), 7.23-7.30 (m, 1 OH), 9.51 (s, 1 H); ¹³C NMR (90 MHz, CDCl₃): δ (ppm) 19.7 (CH₃), 26.5 (CH₃), 27.8 (CH₃), 64.3 (d, ²J_{CP} = 6.0 Hz, CH₂), 69.5 (m, CH₂), 82.7 (d, ³J_{CP} = 8.7 Hz, CH), 85.1 (C_{q}), 110.9 (C_{q}), 126.8 (d, ⁴J_{CP} = 14.5 Hz, CH), 127.9 (CH), 128.6 (CH), 135.6 (d, ³J_{CP} = 7.3 Hz, C_{q}), 202.0 (CH); ³¹P NMR (101 MHz, CDCl₃): δ (ppm) -1 .0 (s).

### (j) 2,3-O-Isopropylidene-2C-methyl-D-erythritol 4-dibenzylphosphate (18).

2,3-O-Isopropylidene-2C-methyl-D-erythrose 4-dibenzylphosphate (17) (85 mg, 0.2 mmol) was dissolved in 3 ml of dry methanol, and the solution was cooled to 0°C. Sodium borohydride, 20 mg, (0.5 mmol) was added in one portion. Water (5 ml) was added to destroy the excess of borohydride, and the mixture was adjusted to pH 5 with concentrated acetic acid. The suspension was extracted 4 times with 10 ml of chloroform, and the organic solution was washed with 20 ml of 5 % sodium hydrogen carbonate. The organic phase was dried with magnesium sulphate, and the solvent was removed under reduced pressure affording 85.5 mg (0.2 mmol, 100 %) pure 18.

¹H NMR (250 MHz, CDCl₃): δ (ppm) 1.20 (s, 3H), 1.30 (s, 3H), 1.36 (s, 3H), 1.89 (s, broad, 2H), 3.34 m, 2H), 3.95 (dd, J = 4.70 Hz, J = 7.10 Hz, 1H), 4.08-4.20 (m, 2H), 5.00 (dd, J = 1.83 Hz, J = 8.55 Hz, 4H), 7.29 (m, 10H); ¹³C NMR (63 MHz, CDCl₃): δ (ppm) 22.1 (CH₃), 26.4 (CH₃), 28.1 (CH₃), 65.0 (CH₂), 65.2 (d, ²J_{CP} = 5.45 Hz, CH₂), 69.4 (dd, ²J_{CP} = 2.72 Hz ²J_{CP} = 5.45 Hz, CH₂), 81.1 (d, ³J_{CP} = 8.18 Hz, CH), 81.7 (Cq), 108.5 (Cq), 126.9 (CH), 128.6 (CH), 135.6 (d, ³J_{CP} = 6.80 Hz, Cq); ³¹P NMR (101 MHz, CDCl₃):δ (ppm) 0.5 (s).

### (k) 2C-Methyl-D-erythritol 4-phosphoric acid (4).

2:3-O-Isopropylidene-2C-methyl-D-erythritol 4-dibenzylphosphate (18) (85.5 mg, 196 *µ*mol) was suspended in 8 ml of a mixture containing 4 ml of methanol and 4 ml of water. A catalytic amount of palladium on charcoal was added, and the suspension was hydrogenated for 20 hours at atmospheric pressure. The catalyst was removed by filtrating through a 0.2 *µ*m membrane filter. The acidic solution (pH 2) was heated to 70°C for 60 minutes. Methanol was removed under reduced pressure at 40°C, and the residue was lyophilized affording 35.3 mg (1 63 *µ*mol, 83 %) of the crude phosphoric acid. The phosphoric acid was dissolved in 1ml of water. The solution was placed on a Nucleosil SB₁₀ HPLC column and was eluted with 0.5 M formic acid at a flow rate of 1 ml/min. The effluent was monitored refractometrically. Fractions containing the product (retention volume 15 ml) were pooled and freeze-dried affording 18.0 mg of pure **4**.

¹H-NMR (500 MHz, D₂O): δ (ppm) 1.04 (s, 3H), 3.37 (d, ²J = 1 1 .77 Hz, 1 H), 3.50 (d, ²J = 1 1.78 Hz, 1 H), 3.64 (dd, ³j = 2.60 Hz, ³j = 8.10 Hz, 1 H), 3.77 (ddd, ³J_{HP} = 6.20 Hz, ³J = 8.10 Hz, ³J = 10.80 Hz, 1 H), 4.01 (ddd, ³J = 2.50 Hz, ³J_{HP} = 6.00 Hz, ³J = 10.80 Hz, 1 H), ¹³C NMR (125 MHz, D₂O): δ (ppm) 1 8.2 (C₃), 65.9 (d,²J_{PC} = 5.14 Hz, CH₂), 66.2 (CH₂), 73.1 (d, ³J_{CP} = 7.58 Hz, CH), 73.8 (Cq); ³¹P NMR (101 MHz, D₂O): δ (ppm) 3.7 (s).

### Preparation Example 2

### [1-²H₁]-2C-Methyl-D-erythritol 4-phosphoric acid (4)

2,3-O-Isopropylidene-2C-methyl-D-erythrose 4-dibenzylphosphate (17), 85 mg, (0.2 mmol) was dissolved in 3 ml of dry methanol, and the solution was cooled to 0°C. [²H]-NaBH₄ (20 mg, 0.5 mmol) was added in one portion. Water (5 ml) was added to destroy the excess of borohydride, and the mixture was adjusted to pH 5 with concentrated acetic acid. The suspension was extracted 4 times with 10 ml of chloroform, and the organic solution was washed with 20 ml of 5 % sodium hydrogen carbonate. The organic phase was dried with magnesium sulphate, and the solvent was removed under reduced pressure affording 85.5 mg (0.2 mmol, 100 %) pure [1-²H₁]-2,3-O-Isopropylidene-2C-methyl-D-erythritol-4-dibenzylphosphate (21).

[1-²H₁]-2,3-O-Isopropylidene-2C-methyl-D-erythritol 4-dibenzylphosphate (85.5 mg, 196 *µ*mol) was then suspended in 8 ml of a mixture containing 4 ml of methanol and 4 ml of water. A catalytic amount of palladium on charcoal was added, and the suspension was hydrogenated for 20 hours at atmospheric pressure. The catalyst was removed by filtrating through a 0.2 pm membrane filter. The acidic solution (pH 2) was heated to 70°C for 60 minutes. Methanol was removed under reduced pressure at 40°C, and the residue was lyophilized affording 35.3 mg (163 *µ*mol, 83%) of the crude phosphoric acid. The phosphoric acid was dissolved in 1 ml of water. The solution was placed on a Nucleosil SB₁₀ HPLC column and was eluted with 0.5 M formic acid at a flow rate of 1 ml/min. The effluent was monitored refractometrically. Fractions containing the product (retention volume 15 ml) were pooled and freeze-dried affording pure [1-²H₁]-4.

### Preparation Example 3

### [1-³H₁]-2C-Methyl-D-erythritol 4-phosphoric acid (4)

[³H]-NaBH₄ (8.5 *µ*mol, 100 mCi, 11.8 Ci/mmol) was suspended in 500 *µ*l of dry methanol. 170 *µ*l of a solution containing 33.3 *µ*mol of 2,3-O-Isopropylidene-2C-methyl-D-erythrose 4-dibenzylphosphate (17) in dry methanol was added in one portion to the borohydride suspension at room temperature. After 1 hour at room temperature 1 ml of water was added to destroy unreacted borohydride. The resulting suspension was extracted with chloroform (3 × 170 *µ*l), the organic phases were combined and the solvent was removed under reduced pressure without drying.

The residue was dissolved in 50 % methanol (1 ml), a catalytic amount of Pd on charcoal was added and the mixture was hydrogenated for 12 hours (room temperature, 1 atm). The catalyst was removed by filtration. Acetic acid (100 %, 1 ml) was added and the mixture was heated to 60°C for 30 minutes.

### Preparation Example 4

The repetition of preparation example 1 with [¹³C]methyl iodide in step (a) affords the ¹³C-labelled product (4).

### Preparation Example 5

The repetition of preparation example 1 with [²H₃]methyl iodide affords the deuterium labelled product (4).

### Preparation Example 6

The repition of preparation Example 1 with [³H] methyl iodide affords the tritium labelled product (4).

### Preparation Example 7

The repetition of preparation example 1 with potassium ¹⁴C-cyanide in step (c) affords ¹⁴C-labelled product (4).

### Preparation Example 8

[1,2-¹⁴C₂] 1-Deoxy-D-xylulose 5-phosphate (specific activity: 62,5 m Ci/mmol) was prepared biosynthetically by the method described in Sprenger et al Proc. Natl. Acad. Sci. USA 94 (1997) 12857 - 12862, using [U-¹⁴C]pyruvate (specific activity: 150 m Ci/mmol) and D,L-glyceraldehyde 3-phosphate.

### Preparation Example 9

[1-³H] 1-Deoxy-D-xylulose 5-phosphate (specific activity: 5 mCi/mmol) was synthesized in accordance with Preparation Example 7 by using [3-³H]pyruvate (specific activity: 72.3 Ci/mmol).

### Preparation Example 10

[1,2-¹⁴C_{2]} 1-Deoxy-D-xylulose (specific activity: 62.5 mCi/mmol) was prepared from [U-¹⁴C] pyruvate with a specific radioactivity of 150 mCi/mmol and D-glyceraldehyde by using as catalyst the pyruvate dehydrogenase complex of *E-coli* DH5α. The yield was 80%. The method of Yokota, A. and Sasajima, K. Agric. Biol. Chem. 48 149-158 (1984) and ibid 50, 2517-2524 (1986) was used.

### Annex A

Alignment of amino acid sequences orthologous to YgbP and YgbB of *E. coli*

*          20          *          40          *          60
A.T.    : MAMLQTNLGFITSPTFLCPKLKVKLNSYLWFSYRSQGNFSYSLYTFKPMNLWFVQKLDF :  59

          *          80          *          100          *          120
E.C.    :                                        MATTHLDVCAVVPAAGFGRRM---- :  21
H.I.    :                                           MARSIIAVLPAAGVGSRM---- :  18
B.S.    :                                             MSYDVVIPAAGQGKRMK--- :  17
S.S.    :                                               MHLLIPAAGSGKRM---- :  14
M.T.    :                                        MVREAGEVVAIVPAAGSGERL---- :  21
A.E.    :                                              MYTAIILAAGRGSRI---- :  15
C.T.    :                                           MNLSCSLVLLGGGKGERF---- :  18
C.P.    :                                            MIKSSLILLSGGQGTRF---- :  17
T.M.    :                                              MNVAILLAAGKGERM---- :  15
P.H.    :                                              MVTLILLAGGSGTRA---- :  15
S.T.    :                                        MAATLLDVCAVVPAAGFGRRM---- :  21
Y.P.    :                                             <EVIAVLPAAGIGSRM---- :  15
A.A.    :                                         <TSTRKIIAVVPAAGIGSRM---- :  19
V.C.    :                                             <NMTAIVPAAGVGSRM---- :  15
S.P.    :                                             <NVVAIVPAAGIGSRM---- :  15
P.M.    :                                              <IVAVVPAAGIGSRM---- :  14
P.A.    :                                                 <VIPAAGVGSRM---- :  11
N.G.    :                                                <ALIPAAGIGVRF---- :  12
B.P.    :                                                <AIVPAAGIGARASLPG :  16
N.M.SA  :                                                <ALIPAAGIGARF---- :  12
D.R.    :                                                <ALIPAAGSGTRL---- :  12
C.A.    :                                               <CAIIMAAGRGSRMK--- :  14
M.A.    :                                              <VAAVVPAAGSGERL---- :  14
M.B.    :                                             <EVVAIVPAAGSGERL---- :  15
C.T.    :                                             MKTVVIIAASGVGKRMKL-- :  18
P.G.    :                                                <ALIVAGGHGLRM---- :  12
E.F.    :                                              <ITALIIAGGVGKRM---- :  14
S.Pn.   :                                              <IYAGILAGGTGTRM---G :  15
S.A.    :                                                  <ILAGGIGSRM---G :  11
A.T.    : SKRVNRSYKRDALLLSIKCSSSTGFDNSNAVNSNVVVKEKSVSVILLAGGQGKRM---- : 114
H.P.    :        MSLIRVNGEAFKLSLESLEEDPFETKETLETLIKQTSVVLLAAGESRRF---- :  49
T.P.    :                                                    MRRGGACVQK---- :  10
R.C.    :                                             MTVAVIIVAAGRGTRA---- :  16
C.C.    :                                             MTFSVVIVAAGSGTRA---- :  16
C.J.    :                                               MSLIMLAAGNSTRF---- :  14

120 * 140 * 160 *
E.C. : QTECPKQYLSIGNQTILEHSVHALLAHPRVKRVVIAISPG-D----SRFAQLPLA---- : 71
H.I. : QADKPKQYLTLLGKTLLEHTLDVMLSYPAVSKIILAVSKD-D----PYISTLSL----- : 67
B.S. : -AGRNKLFIELKGDPVIIHTLRVFDSHRQCDKIILVINEQ-DRE-HFQQLLSDYP---- : 69
S.S. : GSGHNKLLLNVLGQPLLSWTVQAALASQSIEWIGIMGQPY-DFP-AFEALLTPLH---- : 67
M.T. : AVGVPKAFYQLDGQTLIERAVDGLLDSGVVDTVVV------AVP-ADRTDEARQI---- : 69
A.E. : --GFRKQFATLCGKPLFMHSLEKVLD--IFEEVILVLPE---------DFLDKVK---- : 57
C.T. : NSLQPKQYTHLCGEPLILHALHAYQRLPFVQEVVVVCEEQYRELFLPY----------- : 66
C.P. : GSKIPKQYLPLNGTPLVLHSLKILSSLPQIAEVIVVCDPSYQETFQEY----------- : 65
T.M. : SENVPKQFLEIEGRMLFEYPLSTFLKSEAIDGVVIVTRREWFE---VV--EKRVF---- : 65
P.H. : SLNLPKQYYRIEEKMVIEYTLENVSRVKGVDNIILVSNPRFMDT--AL--ELKES---- : 66
S.T. : QTECPXQYLSIGNKTILEHSVHALLAHPRVTRVVIAISPG-D----HRFAQLPLA---- : 71
Y.P. : LVDCPKQYLTVGGKTIIEHAIFSLLHHPRIQRVIVVIHPQ-D----TQFSRLSVA---- : 65
A.A. : QADKPKQYLHIHGQPILQHTLNVLLAYPHISRIVLAVAAD-D----PYIDQLKLS---- : 69
V.C. : QADRPKQYLTLLDKTVLEHTVEHLLEHPLIEHVVVAVSAD-D----PYFANLPLA---- : 65
S.P. : GAGKPKQYLPLLGQSILAHTLDKLLSHPLISQVIVALHPE-D----ADFYALPQA---- : 65
P.M. : QMDKPKQYLHIHGKTILEHTLSVLLGYPLIEKII*AVAAN-D----PYISTCPLL---- : 63
P.A. : RADRPKQYLDLAGRTVIERTLDCFLEHPMLRGLVVCLAED-D----PYWPGLDCA---- : 61
N.G. : GADKPKQYVEIGSKTVLEHVLGIFERHEAVDLTVVVVSPE-D----TFADKVQTA---- : 62
B.P. : EAAVPKQYRPLAGQPMLRHAVRALLADPRIVQVRVAVSAG-D----GXVEQALAG---- : 66
N.M.SA : GADKPKQYVEIGSKTVLEHTIGIFERHEAVDLTVVVVSPE-D----TFADKVQTA---- : 62
T.F. : ------------DRPVIAHTLAAFLGEPRIAGIQLVLPGE-DIATGAWRELLGPM---- : 42
D.R. : GLG-PKAFVEVAGRSLLARSVAAL--APFVDEVVV------ALP-AGMDLPA------- : 54
C.A. : -VNKNKQFILIQGKPILAYTIDKFQRSPLIDEIIIVAAES-EINFCMQEIVYKYK---- : 67
M.A. : AAGIPKAFCEIDGASMLARAVAGLLDSKVVDHVVV------AVP-ADRVDEAKRL---- : 62
M.B. : AVGVPKAFYQLDGQTLIERAVDGLLDSGVVDTVVV------AVP-ADRTDEARQI---- : 63
C.T. : DGGRSKQMLEIGGQPVIWHTMKAFQEASTVESV-YIATLP-DSIPVFKEIAKANG---- : 71
P.G. : GADRPKQFLLLAGLPVLMHTLNRF--APHVDAIVLVLP-T-DHHAYWQELCRKYD---- : 63
E.F. : GQEIPKQFIMVEEKPIIIYTLESFQKHPLIDRILVVCKKGWEQTLDAY--AKEYH---- : 67
S.Pn. : ISNLPKQFLELGDRPILIHTIEKFVLEPSIEKIVVGVHGDWVSHAEDLVDKYLPL---- : 70
S.A. : NVPLPKQFLDIDNKPILIHTIEKFILVSEFNEIIIATPAQWISHTQDILKKY-NI---- : 65
P.F. : <DEVVIVVAPGEDARAVDVLAGLSNWRS--- : 27
A.T. : -----KLCFWLRDVPKISLSLFLFCGVLEYEESIDV----------------------- : 145
H.P. : SQTIKKQWLRSNHTPLWLSVYESFKEALDFKEIILVVSELDYIYIK------------- : 95
T.P. : KEYLPLTSRQPGVCLLSEILVRALEARSFFLVVVTVPAGEVAYAESQVACDSRLSAFPS : 69
R.C. : GEGLPKQWRDLAGRPVLAQTVAAFAGLGRILVVLHPDDMGLGMDLLGGS---------- : 65
C.C. : GPGQAKQWRVLAGRPVLRWSVEAFLAAGAAEVVVVTTADGEAFLPRMLEGLQGWRS--- : 72
C.J. : NTKVKKQFLRLGNDPLWLYATKNLSSFYPFKKIVVTSSNI--TYMK------------- : 58

180 * 200 * 220 *
E.C. : -NHPQITVVDGGDERADSVLAGLKAAGD---AQ-----WVLVHDAARPCLHQDDLARLL : 121
H.I. : --DPKIQLVEGGTTRAESVLNGLNAIAE-----K--NAWVLVHDAARPCLQHADIDKLL : 117
B.S. : -FQTSIELVAGGDERQHSVYKGLKAVKQE--------KIVLVHDGARPFIKHEQIDELI : 119
S.S. : -SPKPVQLIVGGDTRQQSVFNGIQALP--PGAK-----FVLIHDGARCLATPDLFDRCT : 118
M.T. : -LGHRAMIVAGGSNRTDTVNLALTVLSGTAEPE-----FVLVHDAARALTPPALVARVV : 122
A.E. : -VHPKVKKVAGGPERQDSVFNALLQAT-G--------DIVVIHDSARPLATKKMFLE-V : 105
C.T. : ----SVKFASPGTLRQDSVFSGLQ--Q-------VSTPWVCIHDGVRPFVYADEVIEVC : 112
C.P. : ----PVSFAIPGERRQDSVFSGLQ--Q-------VSYPWVIIHDGARPFIYPDEIHDLL : 111
T.M. : -HEKVLGIVEGGDTRSQSVRSALEFLEKFS--P----SYVLVHDSARPFLRKKHVSEVL : 117
P.H. : -FPKIKDVAKGGRTRNESIYNGFMKVPQ-K--E----SKILVHDAVRPFTPRWVFERII : 117
S.T. : -NHPQITVVDGGNERADSVLAGLQAVAK---AQ-----WVLXHDAARPCLHQDDLARLL : 121
Y.P. : -QDPRISTVYGGDQRANSVMAGLQLAGQ---AE-----WVLVHDAARPCLHLDDLSRLL : 115
A.A. : -QNPKIQLVEGGETRADSVLNGLNAVQDA---GA--DVWVMVHDAARPCLTHGDLEKLL : 122
V.C. : -HHPRVIRVDGGKERADSVLSALEYVCQHRLSE-----WVLVHDAARPCVTHADITQLI : 118
S.P. : -KHPKLKTVIGGSERANSVLAALDKAPDN---S-----WALVHDAARPCLMASDIDKLL : 115
P.M. : -THPKIQLVEGGSSRADSVLNGLNAVKSAVQNSE--DFWVMVHDAARPCLTHQDLDKLV : 119
P.A. : -ASRHVQRAAGGAERAGSVLNGLLRLLE-LGAQA--DDWVLVHDAARPNLTRGDLDRLL : 116
N.G. : -FPQVRVWKNGGQTRAETVRNGVAKLLETGLAAE--TDNILVHDAARCCLPSEALARLI : 118
B.P. : -LPRTVWRPCGGPNRADTVAXALADSG----AAA--DDWIXVHDAARPGLPAAAXARLI : 118
N.M.SA : -FPQVRVWKNGGQTRAETVRNGVAKLLETGLAAE--TDNILVHDAARCCLPSEALTRLI : 118
T.F. : -PAPLLPPVVGGGLRADSVRLGLEALLRQG-AVP--SDWVLVHDAARPCLRREDLLRLL : 97
D.R. : --GVPARAIVGGETRQGSVRRLLEA----TEAG-----TVLIHDAARPFVPPPVILALL : 102
C.A. : -FNKVKNIVSGGSERQQSVMNGLKAVKSA--------NIVLIHDGARPFVDNKIIENGI : 117
M.A. : -LAAQATVVAGGADRTASVRLALAAVPG--NPA-----FVLVHDAARALTPPALIARVV : 113
M.B. : -LGHRAMIVAGGSNRTDTVNLALAVLSGTAEPE-----FVLVHDAARALTPPALVARVV : 116
C.T. : -FTKITAIIEGGKERQDSIGNCMKLIEQEIENSGVMPDAILVHDGARPFIQPEEIDDIA : 129
P.G. : -FSVSHRVVAGGNTRFASVRNGLQVVPDGV--------LVAVHDGVRPLVSAETIDACF : 113
E.F. : -IDKLQWIIPGGNSGQESINNGVNFLKEHSNPE---DTIVIHDGIRPLVDELVLSDVI : 121
S.Pn. : -YKERIIITKGGADRNTSIKNIIEAIDA--YRPLTPEDIVVTHDSVRPFITLRMIQDNI : 126
S.A. : -TDQRVKVVAGGTDRNETIMNIIDHIRN--VNGINNDDVIVTHDAVRPFLTQRIIKENI : 121
P.F. : --------VTGGDARADSVRAGLTALT-CPADQ-----PVMIHDAARPLLSQTVIE> : 69
A.T. : ----DLRFAIPGKERQDSVYSGLQEID-------VNSELVCIHDSARPLVNTEDVEKVL : 193
H.P. : RHYPEIKLVKGGASRQESVRNALKII----DSA-----YTLTSDVARGLANIEALKNLF : 145
T.P. : RTRPVILYVPGAHTRSASVRAGLDAMAT-HAPD-----VVLVHDGARPFVSVALIHSVL : 122
R.C. : -----VVLVAGGSTRSESVKNALEALEG-SDVT-----RVLIHDGARPLVPASVTAAVL : 113
C.C. : --------TLGGATRALSVQAGLAALSERPGAE-----PVMIHDAARPFVSRNVILALL : 118
C.J. : KFTKNYEFIEGGDTRAESLKKALELI----DSE-----FVMVSDVARVLVSKNLFDRLI : 108

240 * 260 * 280 *
E.C. : ALSETSRTGGILAAPVRDTMKRAEPGKNAIAHTVDRNGLWHALTPQFFPRELLHDCLTR : 180
H.I. : AIED--KQGAILAIPVTDTIKRADNQ-QCIVKTEDRSQLWQAMTPQFFPVDILRDALST : 173
B.S. : AEAEQ-TGAAILAVPVKDTIKRVQ-DLQVSE-TIERSSLWAVQTPQAFRLSLLMKAHAE : 175
S.S. : EALQH-CQGLIAAMPVKDTIKIVNADGWI-TDTPDRQGLWGAQTPQGFDVALLKACHDK : 175
M.T. : EALRDGYAAVVPVLPLSDTIKAVDANGVVLG-TPERAGLRAVQTPQGFTTDLLLRSYQR : 180
A.E. : AQLGD-YHGKVVASPARDTLKEVV-EGKVI-KTLNRSLIWHAQTPQAFRRDILLECHMR : 161
C.T. : SAARKT-GAAALASPATYTIKSCAP-----VRTLDRDALAVIHTPQCLDTEVLREGLLL : 165
C.P. : ETAEKI-GATALASPIPYTIKQRNP-----VRTLDRDNLAIIHTPQCIKTEILREGLAL : 164
T.M. : RRARETGAATLALKNSDA-LVRVENDR--IEYIPRKGVYRI-LTPQAFSYEILKKAHEN : 172
P.H. : SLLDERDVITTVNPITGN-LIELDNGK--VKRIYDRSKFAIGEAPTGYRYGALKKTLEV : 173
S.T. : AISENSRVGGILASPVRDTMKRGEPGKNAIAHTVERADLWHALTPQFFPRELLHDCLTR : 180
Y.P. : SITECSQVGGILAAPVRDTMKRAEPGIQAIAHTVDRQDLWHALTPQLFPLELLKLCLSR : 174
A.A. : EIQD--DNGAILAIPATDTIKRALPS-QQIAHTEDRSQLWLAQTPQFFRADLLRDALTR : 178
V.C. : TTALAHPIGAILASPVRDTMKRGDH-LQQIVHTVDRTALWHALTPQMFRAQSLRERLFA : 176
S.P. : TSRVQFPQGAILAMPVRDTMKRANS-LGEINSTVCRDNLWHALTPQLFPTSLLRLHLQG : 173
P.M. : QVED--QNGAILAIPATDTIKRALHN-QQIHYTEDRSQLWLAQTPQFFPIATLAQALEQ : 175
P.A. : EELAEDPVGGLLAVPARDTLKRSDRD-GRVSETIDRSVVWLAYTPQMFRLGALHRALAD : 174
N.G. : EQAGNAAEGGILAVPVADTLKRAESG--QISATVDRSGLWQAQTPQLFQAGLLHRALAA : 175
B.P. : DACXXDAVGGLLALPVADTV---XAGRQRVSRTVDRDGLWLAQTPQMFRAGLLRDALAR : 174
N.M.SA : EQAGNAAEGGILAIPVADTLKCADGG--NISATVERTSLWQAQTPQLFRAGLLHRALAA : 175
T.F. : ESLANAPQGALLAVPVADTLKRGEDGCS--SGTVDREGLWRALTPQAFPLGALLAALEA : 154
D.R. : DAIAATGAATV-ALPVADTL--VRAEGQSWGQLVPREGLWAVQTPQGFRRELLLQAHAR : 158
C.A. : KYAEK-YGGAACGVQPKDTIKIKS-EDGFSEKTIDRSKLFCVQTPQCFKYDSILKAHIN : 174
M.A. : QALRDGHRAVVPALPLHDTVKAVDANGVVLG-TPERDGLRAVQTPQGFATDLLLRAYAA : 171
M.B. : EALRDGYAAVVPVLPLSDTIKAVDANGVVLG-TPERAGLRAVQTPQGFTTDLLLRSYQR : 174
C.T. : RLSAT-HGACVPATKPKDTIKYVGCNPEIFGETLDRSRLLQVQTPQGFAPAKLIEAHRL : 187
P.G. : DLAEL-KGAVAPCRPMTESLRYYATDGNY---AVDRSRYVTVQTPQTFRSEWLREAYRQ : 168
E.F. : VKCQEYGNAVTSLPYNEQIFVKETEET--TRQYINRETLRRVSTPQAYKFEKLTWAYEK : 178
S.Pn. : QLAQNHDAVDTVVEAVDTIVE--STNGQFITDIPNRAHLYQGQTPQTFRCKDFMDLYGS : 183
S.A. : EVAAKYGAVDTVIEAIDTIVM--SKDKQNIHSIPVRNEMYQGQTPQSFNIKLLQDSYRA : 178
A.T. : KDGSAV-GAAVLGVPAKATIK-----------------------EVIKPELLKKGFEL : 227
H.P. : LTLQQTS----HYCIAPYL--PCYDTAIYYNEALDREAIKLIQTPQLSHTKALQSA--- : 195
T.P. : EATCRYGAAVPVIEATDTP--KGVAADGSIETHLIRSRVRLAQTPQGFCYASLCAAHHR : 179
R.C. : AALETTPGAAPALAVTDAL--WRGEAGLVAGTQ-DREGLYRAQTPQGFRFPEILAAHRA : 169
C.C. : GALSDADLALPALAVADTL--KRQPTGEAAQT-VSREHLWRAQTPQAARRDTLIAAYAA : 174
C.J. : ENLDKAD------CITPAL--KVADTTLFDNEALQREKIKLIQTPQISKTKLLKKA--- : 156

300 * 320 * 340 *
E.C. : ALNE--GATITDEASALE--YCGFHPQLVEGRADNIKVTRPEDLALAEFYLTRTIHQENT\\ : 235
H.I. : GIQQ--GANITDEASAIE--LAGFRPHLVAGRSDNLKVTRPEDLALAEFYLTRNKL\\ : 225
B.S. : AERK--GFLGTDDASLVEQ-MEGGSVRVVEGSYTNIKLTTPDDLTSAEAIMESESGNKH\\ : 231
S.S. : GKQE--GWEVTDDAALLE--KCGQPVKIVPGEDTNLKITTPVDLAIAEFILGQRSAKSA\\ : 230
M.T. : GSLDLPAAEYTDDASLVE--HIGGQVQVVDGDPLAFKITTKLDLLLAQAIVRG\\ : 231
A.E. : AKAE--GFVGTDDASLL-E-RYGYSVGVVEGSYWNVKITYPEDLEMVKKIMGCEED\\ : 213
C.T. : ARAM--DFSLSDDTEAAE--LLGIEPTLVFSNRVQIKVTYPEDLLFAETLLSKSSTYK\\ : 219
C.P. : AKEK--QLTLVDDIEAAE--IIGKPSQLVFNKHPQIKISYPEDLTIAQALL\\ : 211
T.M. : G-------GEWADDTEPVQKL-GVKIALVEGDPLCFKVTFKEDLELARIIAREWERIP\\ : 222
P.H. : AV-SNGTLNEIPHDIVLAMNA-GFDVYVLPCNCFNLKITFKEDIEIARTLIKMLEERE\\ : 229
S.T. : ALNE--GATITDEASALE--YCGFHPALVEGRADNIKVTRPEDLALAEFYLTRTIHQE> : 234
Y.P. : ALRE--GVAVTDEASALE--HCGYHPILVTGRSDNIKVTRPEDLALAEFYLTQ> : 223
A.A. : AKQQ--QFAVTDEASAME--LAGFRPHLVAGRSDNIKVTRPEDLALAEFYLTR> : 227
V.C. : ALQQ--QVTITDEASAFE--WRGEKPALVAGRADNLKITQPEDLALAEFYLSR> : 225
S.P. : ALNA--GAVVTDEASAME--WAGISPGLVAGRADNIKVTHPDDLELAELFLMR> : 222
P.M. : ALAQ--GLQVTDEASAME--FAGFRPHLVAGRSDNIKVTRPEDFALAEFYLSRT> : 225
P.A. : ALVA--GVAITDEASAME--WAGYAPKLVEGRADNLKITTPEDL> : 214
N.G. : ENLG--G--ITDEASAVE--KLGVRPLLIQGDARNLKLTQPQDAYIVRLLL> : 220
B.P. : ARAA--GLAVTDEASAVE--AAGHAPRLVAGALRNFKVTWPDDFELMEKWL> : 221
N.M.SA : ENLD--G--ITDEASAVE--KLGIRPLLVQGDARNLKLTQPQDAYIVRLLL> : 220
T.F. : ARAG--NRQITDEASAME--AQGWRPRLIPGHGDNIKVTLSDDLMLA> : 197
D.R. : AEAEQYAA--TDDAGLLA--RLGVQVRLVPGDARLFKVTTPGDLALAE> : 202
C.A. : AEKE--GILATDDT-MIFE-MSGNKVYLYDGSYENLKITTPDDLYAAETLLKKNS> : 225
M.A. : GA---GTAGFTDDASLVE--HVGGQVQVVDGDPLAFKITTQLDLLLAETIVRR> : 219
M.B. : GSLDLPAAEYTDDASLVE--HIGGQVQVVDGDPLAFKITTKLDLLLAQAIV> : 223
C.T. : AGEE--QWYAXDDAALVERYFPQQAIXIYETGYHNIKITTPEDVFIGEAILAGLKARK> : 243
P.G. : PYEE--YF--TDDCSVYEHHF-GRPVALIVGNIENIKLTTPLDLSLAKLLLTS> : 216
E.F. : AFRENIGISESSYTNTMMVDL-GETLHFALGSDKNIKLTTQDDLQLFKFL> : 227
S.Pn. : LSDEEKEILTDACKIFVI---KGKDVALAKGEYSNLKITTVTDLKIAKSMIEKD> : 234
S.A. : LSSEQKEILSDACKIIVE---SGHAVKLVRGELYNIKVTTPYDLKVANAIIQGD> : 229
A.T. : VKSE--GLEVTDDVSIVE--YLKHPVYVSQGSYTNIKVTTPDDLLLAERILSEDS> : 278
H.P. : LNQG-----DFKDESSAILQAFPDRVSYIEGSKDLHKLTTSGDLKHFTLFFNPAK---- : 245
T.P. : AATD----GEQYTDDSELYARYGGTVHVCAGERSNVKITYPEDLEQRASEPALTRGISV : 234
R.C. : HPGG----A---ADDVEVARHAGLSVAIVPGHEDNLKITYAPDFARAEAILRERKGLTM : 221
C.C. : WTHG----EP--TDDAQVYEHHFGRIALTAGDPLLTKLTYPEDFAMAEHLAGVARV--- : 224
C.J. : LDQN----LEFTDDSTAI-AAMGGKIWFVEGEENARKLTFKEDLKKLDL-PTPSF---- : 205
C.T. : MTEIPSSF : 8
C.P. : MDRDNEV : 7
P.F. : MFLKGYTSNVVLIILTFFILLTKEEKNIKNNISGYCFLNFGLKKNAIIKKREKQN : 55

360 * 380 * 400 *
H.P. : -------DTFIGMGFDTHAFIKD--------------------KPMVLGGVVLD-CEFGL : 277
T.P. : LPCTEEGALRVGLGTDMHALCAG--------------------RPLILAGIHIPSK-KGA : 273
R.C. : -------DVRLGNGYDVHAFCEG--------------------DHVVLCGVKVPHV-KAL : 253
C.C. : --------TRVGQGFDAHRWGPG--------------------EEVWLCGVAIKHDET-L : 255
C.J. : -------EIFTGNGFDVHEFGEN--------------------RPLLLAGVQIHPT-MGL : 237
E.C. : MRIGHGFDVHAFGGE--------------------GPIIIGGVRIPY-EKGL : 31
H.I. : MIRIGHGFDVHAFGED--------------------RPLIIGGVEVPY-HTGF : 32
B.S. : MFRIGQGFDVHQLVEG--------------------RPLIIGGIEIPY-EKGL : 32
S.S. : MTALRIGNGYDIHRLVGD--------------------RPLILGGVTIAH-HLGL : 34
M.T. : MNQLPRVGLGTDVHPIEPG--------------------RPCWLVGLLFPSAD-GC : 35
A.E. : MELRIGFGFDSHEFVEG--------------------KLLILGGVEIEK-DYGL : 33
C.T. : VLPDPEWIYRVGIGQDSHRFLPDEDP-----------------KPCILGGIIFENTP-GF : 50
C.P. : PLPKPKWIYRTGIGQDSHRFLPESST-----------------KPCILGGIIFDHCP-GF : 49
T.M. : MESDPMFIGFGYDRHPLVEG--------------------RRLVLAGVEID-APLGS : 36
H.D. : MIRIGHGFDVHAFGEK--------------------RPLIIGGVTIPY-HTGF : 32
S.T. : <IGHGFDVHAFGGE--------------------GPIIIGGVRIPY-EKGL : 29
Y.P. : <IGHGFDVHKFGENGS------------------GPLIIGGVRIPY-EKGL : 31
A.A. : <IGHGFDVHAFGTN--------------------NPLIIGGVTIPF-DKGF : 29
V.C. : <IGHGFDVHRFGGE--------------------GPIIIGGVKIPY-EQGL : 29
S.P. : <IGHGFDVHKFGEP--------------------RPLILCGVEVPY-ETGL : 29
P.M. : <IGHGFDVHAFGGE--------------------GPIIIGGVAIPY-EKGL : 29
P.A. : <IGHGYDVHRFGEG--------------------DFITLGGVRIPH-KHGL : 29
N.G. : <IGQGYDVHQLTEG--------------------RKLILGGVEIPF-EKGL : 29
B.P. : <VGQGFDVHALVEG--------------------RPLIIGGVTIAH-THGL : 29
N.M.SA : <LILGGVEIPF-EKGL : 14
N.M.MC : <IGQGYDVHQLTEG--------------------RKLILGGVEIPF-EKGL : 29
T.F. : <IGHGFDVHALVPG--------------------RALILGGVSVPY-ERGL : 29
D.R. : <IGYGEDAHRLAPG--------------------LPLVLGGVAIPHAELGA : 30
C.A. : <VGIGYDVHKLVEN--------------------RKLILGGVEIQY-SKGL : 29
M.A. : <IGLGVDVHPIQPG--------------------RPCRLLGLLFDDAD-GC : 29
M.B. : <VGLGTDVHPIEPG--------------------RPCWLVGLLFPSAD-GC : 29
C.T. : <IGIGIDVHQFAEG--------------------RKLIIGGVEVPS-PIGL : 29
P.G. : <IGFGFDVHRLSEG--------------------YPLWMGGVRLEH-SKGL : 29
E.F. : <IGQGFDVHQLVPQ--------------------RPLIIGGVTLPY-EKGL : 29
P.F. : LKLFCYNGIRIGQGYDIHKIKVLDEEYNTYANNDFNKNEQSFKTLTLGGVKIN--NVLV : 112

420 * 440 * 460 *
H.P. : KAHSDGDALLHAVIDAILGAIKGGDIGEWFPDNDPKYKNASSKELLKIVLDFSQSIGFE : 336
T.P. : QGHSDADVLAHASIDALLGAAGLGDIGTFFPSCDGRWKDAHSCALLRHTWQLVRAACWR : 332
R.C. : LGHSDADVGMHALTDAIYGALAEGDIGRHFPPSDPQWKGAASWIFLDHAAKLAKSRGFR : 312
C.C. : VGHSDADAGLHALTDAILGAIGEGDIGDHFPPTDPKWKGAASDQFLKHAVDLVTAKGGA : 314
C.J. : KAHSDGDVLAHSLTDAILGAAGLGDIGELYPDTDMKFKNANSMELLKQAYDKVREIGFE : 296
E.C. : LAHSDGDVALHALTDALLGAAALGDIGKLFPDTDPAFKGADSRELLREAWRRIQAKGYT : 90
H.I. : IAHSDGDVALHALTDAILGAAALGDIGKLFPDTDMQYKNADSRGLLREAFRQVQEKGYK : 91
B.S. : LGHSDADVLLHTVADACLGAVGEGDIGKHFPDTDPEFKDADSFKLLQHVWGIVKQKGYV : 91
S.S. : DGHSDADVLTHALMDALLGALSLGDIGHYFPPSDARWQGADSLKLLAQVHQLILERGWR : 93
M.T. : AGHSDGDVAVHALCDAVLSAAGLGDIGEVFGVDDPRWQGVSGADMLRHVVVLITQHGYR : 94
A.E. : KGHSDGDALLHAITDAILGALGERDIGEIFKDTDPRWKNAPSRIFLEKALEVMSEKGFN : 92
C.T. : EANSDGDVVFHAICNAFSSVTHKGILGGLADELLKTKGITDSVVYLQEAVASLKPTQ-R : 108
C.P. : QANSDGDIIFHAICNAISSVTNKIILGKVADELLQTRGITDSGIYLEEALKSLKPNQ-K : 107
T.M. : LGHSDGDVLSHAIIDALLGAGCLGDIGTWFPETK-EYKDANSLDLLKETVKILEERGFS : 94
H.D. : IAHSDGDVALHALTDALLGAAALGDIGKLFPDTDQQYKNIDSRKLLIEAYRQVQTKGYQ : 91
S.T. : LAHSDGDVALHALTDALLGAAALGDIGKLFPDTDPAFKGADSRELLREAWRRIQAKGYT : 88
Y.P. : LAHSDGDVALHAATDALLGAAALGDIGKLFPDTDPAFKGADSRGLLREAYRRILAKGYK : 90
A.A. : IAHSDGDVALHALTDALLGAAALGDIGKLFPDTDMQYKGADSRVLLREAYRQVQEKGYC : 88
V.C. : IAHSDGDVALHALSDALLGAIAAGDIGRHFPDTDDKWKGADSRELLKDVYRRVKAQGYV : 88
S.P. : VAHSDGDVVLHAISDAILGAMALGDIGKHFPDTDAAYKGADSRVLLRHCYALAKAKGFE : 88
P.M. : LAHSDGDVALHALTDALLGAVALGDIGKLFPDTDMQYKGADSRGLLREAYTQVQAKGYK : 88
P.A. : VAHSDGDVLLHALSDALLGAAALGDIGKHFPDTDPRFKGADSRALLRHVVAIVAEKGWK : 88
N.G. : LGHSDADALLHAVTDALLGAAGLGDIGSHFPDTAAEFKDADSRVLLRAAYQSVQAQGWQ : 88
B.P. : LGHSDADVLLHAVTDALLGGAGLGDIGRHFPDTDPAYRGADSRVLLRAAFDKVRAAGWA : 88
N.M.SA : LGHSDADALLHAVTDALLGAAGLGDIGSHFPDTAAEFKDADSRVLLRAAYQSVQAQGWQ : 73
N.M.MC : LGHSDADALLHAVTDALLGAAGLGDIGSHFPDTAAEFKDADSRVLLRAAYQSVQAQGWQ : 88
T.F. : AGHSDADVLLHSICDALLGAAALGDIGRHFPDTDARFEGADSRLLLRHCRQLVQGKGFS : 88
D.R. : VAHSDGDAVLHAVADALLSGLALGDIGQYFPDTAAEWKGMDSRRILAKALELVEERGYR : 89
C.A. : LGHSDADVLVHAIIDSILGAAGLGDIGKLFPDSDNKYKGISSLKLLKEVNALIKDKGYK : 88
M.A. : AGHSDGDVGAHALCDAVLSAAGLGDVGAVFGVDDPRWAGVSGADMLRHVADLTARHGFR : 88
M.B. : AGHSDGDVAVHALCDAVLSAAGLGDIGEVFGVDDPRWQGVSGADMLRHVVVLITQHGYR : 88
C.T. : LGHSDADVLLHAISDALLGAAALGDIGKHFPDTSPDYKDADSMELLRHVCKLLEQEGYK : 88
P.G. : EGHSDADVLIHAICDALLGAAALRDIGYHFPPSDPQYKGIDSKILLARVMELVRSQGYE : 88
E.F. : LGHSDADVLTHAIIDAILGAAGLGDIGQLFPETDPQFKNANSVNLLKKVNEKVGRSGFT : 88
P.F. : LSHSDGDIIYHSIVDSILGALGSLDIGTLFPDKDEKNKNKNSAIFLRYARLLIYKKNYD : 171

480 * 500 * 520 *
H.P. : LFEMGATIFSEIPKITPYKPAILENLSQLLGLEKSQISLKATTMEKM----GFIGKQEG : 391
T.P. : LVNLDAVVCLEQPALHPFREAMRASLAQALDTHVTRVFVKAKTAERL----GPVGSGAA : 387
R.C. : IGNADVTLICERPKVGPHAVAMAAELARIMEIEPSRVSVKATTSERL----GFTGREEG : 367
C.C. : LVNVDVTLICERPKIKPHRQAMRERLAEILSIPVDRVSVKATTTEKM----GFTGRGEG : 369
C.J. : LINIDICVMAQSPKLKDFKQAMQSNIAHTLDLDEFRINVKATTTEKL----GFIGRKEG : 351
E.C. : LGNVDVTIIAQAPKMLPHIPQMRVFIAEDLGCHMDDVNVKATTTEKL----GFTGRGEG : 145
H.I. : IGNVDITIIAQAPKMRPHIDAMRAKIAEDLQCDIEQVNVKATTTEKL----GFTGRQEG : 146
B.S. : LGNIDCTIIAQKPKMLPYIEDMRKRIAEGLEADVSQVNVKATTTEKL----GFTGRAEG : 146
S.S. : INNLDNVIVAEQPKLKPHIQAMKENLAKVLTIDPDLIGIKATTNERL----GPTGREEG : 148
M.T. : VGNAVVQVIGNRPKIGWRRLEAQAVLSRLLN---APVSVSATTTDGL----GLTGRGEG : 146
A.E. : ISNIDCVIVADRPKIAPHKERIKESLSKLLGIPKERISLKGKRREGFCEGNGLVCMCTV : 151
C.T. : VSHLAITIEGKRPKLLPQLPSMRKRIAEVLHIPLDSINITATSGEGLTA----MGQGYG : 163
C.P. : ISHVAITIEGSRPKFLCKLSALRQNIAQVMNLTPTDIGITATSGEGLSD----FGCGDG : 162
T.M. : VVNVDATVVASIVKLSPYREKIVENLKSAL--ETSRVNVKFKSGNTL----GFEGEERG : 147
H.D. : ISNIDITIIAQAPKMRPHIDNMRQLIANDLNCDIDQINIKATTTEKL----GFTGRGEG : 146
S.T. : XGNVDVTIIAQAPKMLPHIPQMRVFIAEDLGCHMDDVNVKATTTEKL----GFTGRGEG : 143
Y.P. : LGNLDITIIAQAPKMAPHIPQMRVNLAEDLQCHMDDINVKATTTEQL----GFTGRGEG : 145
A.A. : VGNVDVTIIAQAPKMRPHIDAMRALIAQDLACDIEQVNVKATTTEKL----GFTGRGEG : 143
V.C. : LGNADVTIIAQAPKMAPYIQAMCAAIAEDLETDLGNINVKATTTEKL----GFTGRKEG : 143
S.P. : LGNLDVTIIAQAPKMAPHIEDMRQVLAADLNADVADINVKATTTEKL----GFTGRKEA : 143
P.M. : VGNVDVTIIAQAPKMRPHIDAMRAAIAEDLACDIEQVNVKATTSERL----GFTGRGEG : 143
P.A. : VGNVDATIVAQAPKMAPHIETMRGLIAEDLGVAVDQVNVKATTTERL----GFTGREEG : 143
N.G. : VVNVDTTVIAQKPKLAPHIPQMRANIAADLGIDISCVNIKGKTNEKL----GYLGRMEG : 143
B.P. : PVNVDATIHAQAPKIGPHAAAMVANIAADLALDAGAVNIKAKTNEGL----GYLGRKEG : 143
N.M.SA : AVNVDTTVIAQKPTLAPHIPQMRANIAADLGIDISCVNIKGKTNEKL----GYLGRMEG : 128
N.M.MC : AVNVDTTVIAQKPKLAPHIPQMRANIAADLGIDISCVNIKGKTNEKL----GYLGRMEG : 143
T.F. : VGNVDATIVCQRPKLADHIPQMRAHIAADLAVELDAVNIKATTTEQL----GYTGRGEG : 143
D.R. : PVNVALVVTLDRPKLGPLRADIAASVAELLGLPAGEVGVSFKTSE> : 134
C.A. : IGNIDSTIIAQKPKISPYIEDIKKSLCNVLDIDLGSINIKATTEEGL----GFTGRGEG : 143
M.A. : VGNAAVQVIGNRPKVGPRRAEAQRVLSELLG---APVSVAATTTDGL----GLTGRGEG : 140
M.B. : VGNAVVQVIGNRPKIGWRRLEAQAVLSRLLN---APVSVSATTTDGL----GLTGRGEG : 140
C.T. : PVNVDTMLLLEKPKIAPYIDQMRRNIARCLGLEINAVSVKATTNEKL----GYVGRQEG> : 143
P.G. : LGNIDATIAAEQPKLNPHIPDMQRVLAEVIQVEVSDISLKATTTEKL----GFTGREEG : 143
E.F. : IGNIDCTILAEEPKMSPYLAEMKKNLAASCHLAVTQVNIKATTMETM----GFVGKKEG : 143
P.F. : IGNVDINVIAQVPKISNIRKNIIKNISTVLNIDESQISVKGKTHEKL----GVIGEKKA : 226

540
H.P.   : LLVQAHVSMRYKQKL\\     : 406
T.P.   : VTAQVVVLLKKI\\        : 399
R.C.   : IASIATVTLIGA\\        : 379
C.C.   : LAASAVVAV>            : 378
C.J.   : MAVLSSVNLKY>          : 362
E.C.   : IACEAVALLIKATK\\      : 159
H.I.   : IACEAVALLIRQ\\        : 158
B.S.   : IAAQATVLIQKG\\        : 158
S.S.   : IAAYSVALLIKEG\\       : 161
M.T.   : LAAIATALVVSLR\\       : 159
A.E.   : LLVKM\\               : 156
C.T.   : VQCFCVLTIMEYCRY\\     : 178
C.P.   : VQCFCVLTVMEYCD\\      : 176
T.M.   : ISAYAVCLVEEKGCTKST\\  : 165
H.D.   : IACEAVALLSKKTV\\      : 160
S.T.   : IACEA>                : 148
Y.P.   : IACEAVV>              : 152
A.A.   : IACEA>                : 148
V.C.   : IACEAVVLLR>           : 153
S.P.   : L>                    : 144
P.M.   : IACEA>                : 148
P.A.   : IAVHA>                : 148
N.G.   : IEAQAAV>              : 150
B.P.   : I>                    : 144
N.M.MC : IEAQAAVLLVR>          : 139
N.M.SA : IESQAAV>              : 150
T.F.   : IAAHAVVLIQH>          : 154
C.A.   : ISSQS>                : 148
M.A.   : L>                    : 141
M.B.   : L>                    : 141
P.G.   : ISAYA>                : 148
E.F.   : I>                    : 144
P.F.   : IECFANILLIPKNS>       : 240

### Annex B

Alignment of amino acid sequences orthologous to YchB of *E*. *coli*

                *        20         *        40         *        60
E.C.   : ------------------------------------------------------------ :  -
H.I.   : ------------------------------------------------------------ :  -
B.S.   : ------------------------------------------------------------ :  -
S.S.   : ------------------------------------------------------------ :  -
M.T.   : ------------------------------------------------------------ :  -
A.E.   : ------------------------------------------------------------ :  -
C.T.   : ------------------------------------------------------------ :  -
C.P.   : ------------------------------------------------------------ :  -
T.M.   : ------------------------------------------------------------ :  -
H.P.   : ------------------------------------------------------------ :  -
T.P.   : ------------------------------------------------------------ :  -
S.TL.  : ------------------------------------------------------------ :  -
Z.M.   : ------------------------------------------------------------ :  -
S.T.   : ------------------------------------------------------------ :  -
S.P.   : ------------------------------------------------------------ :  -
Y.S.   : ------------------------------------------------------------ :  -
A.A.   : ------------------------------------------------------------ :  -
V.C.   : ------------------------------------------------------------ :  -
S.PU.  : ------------------------------------------------------------ :  -
P.M.   : ------------------------------------------------------------ :  -
P.A.   : ------------------------------------------------------------ :  -
N.G.   : ------------------------------------------------------------ :  -
B.P.   : ------------------------------------------------------------ :  -
N.M.   : ------------------------------------------------------------ :  -
K.M.   : ------------------------------------------------------------ :  -
D.R.   : ------------------------------------------------------------ :  -
B.B.   : ------------------------------------------------------------ :  -
C.D.   : ------------------------------------------------------------ :  -
C.A.   : ------------------------------------------------------------ :  -
M.A.   : ------------------------------------------------------------ :  -
M.B.   : ------------------------------------------------------------ :  -
M.L.   : ------------------------------------------------------------ :  -
C.TP.  : ------------------------------------------------------------ :  -
P.G.   : ------------------------------------------------------------ :  -
E.F.   : ------------------------------------------------------------ :  -
S.M.   : ------------------------------------------------------------ :  -
S.PG.  : ------------------------------------------------------------ :  -
S.AN.  : ------------------------------------------------------------ :  -
S.A.   : ------------------------------------------------------------ :  -
A.T.   : ------MATASPPFISTLS----FTHSSFKTSSSSSFSPKLLRPLLSFS-VKAS-----R :  44
S.L.   : LWLPVIFFVVSNPKLILLKRVVFFQSWSNRPHGSSYFNKNIQFRRNSFVIVKASGSRTSK :  60
S.ML.  : ------------------------------------------------------------ :  -
C.C.   : ------------------------------------------------------------ :  -
C.J.   : ------------------------------------------------------------ :  -

*          80          *          100          *          120
E.C.   : -------------------------MRTQWPSPAKLNLFLYITGQRADGYHTLQTLFQF :  34
H.I.   : -------MKSHQFSTALCQNTTESNGQPLRFPSPAKLNLFLYINGKFPNGYHELQTLFQF :  53
B.S.   : -------------------------MRILEKAPAKINLSLDVTRKRPDGYHEVEMIMTT :  34
S.S.   : ------------------------MHSYTLHAPAKINLFLEILGDRPDGFHELVMVLQS :  35
M.T.   : ----------------------MPTGSVTVRVPGKVNLYLAVGDRREDGYHELTTVFHA :  37
A.E.   : --------------------------MIKVLSPAKINLGLWVLGRLPSGYHEILTLYQE :  33
C.T.   : ---------------------------MHFLSPAKLNLFLQILGRREDDFHEIVTRYQA :  32
C.P.   : ---------------------------MQYFSPAKLNLFLKIWGKRFDNFHELTTLYQA :  32
T.M.   : -------------------MVENIGSGSAELVSYAKLNLYLDVLGKRSDGYHEIVGLFQT :  41
H.P.   : -------------------------MTHVFEVYPKVNVFLKILHKEG-AYHKIISRMCL :  33
T.P.   : ------------------------MQSLSLRAHAKVNMHLWVGARRADGLHSIESVMQR :  35
S.TL.  : -------------------------MMTHWPSPAKLNLFLYITGQRADGYHTLQTLFQF :  34
Z.M.   : ------------------------MPKLTEIAYAKINLALHVRGKMPNGYHALETIFAF :  35
S.T.   : -------------------------MMTHWPSPAKLNLFLYITGQRADGYHTLQTLFQF :  34
S.P.   : -------------------------MMTHWPSPAKLNLFLYITGQRADGYHTLQTLFQF :  34
Y.S.   : ----------------------------<KWPSPAKLNLFLYITGQRADGYHQLQTLFQF :  31
A.A.   : ----------------------------<RFPCPAKLNLFLYINGKRATGYHELQTLFQF :  31
V.C.   : ---------------------------<TVWPSPAKLNLFLYITGRRANGYHDLQTLFQF :  32
S.PU.  : -----------------------------<WPAPAKLNLFLHINGRRSDGYHELQTLFQF :  30
P.M.   : ----------------------------<RFPCPAKLNLFLYINGKRQDGYHELQTLFQF :  31
P.A.   : -------------------------<VRLSLPAPAKLNLFLHILGRRDDGYHELQTLFQF :  34
N.G.   : --------------------------<RQAFPAPAKLNLDLRITGRREDGYHNIESIFCL :  33
B.P.   : ------------------------------<PAPAKLNLFLHVVGRRADGYHLLQTAFRF :  29
N.M.   : --------------------------<RQAFPAPAKLNLDLRITGRREDGYHNIESIFCL :  33
K.M.   : ------------------------------------------------------------ :   -
D.R.   : -------------------------------<APAKINLGLSVLGVRENGYHDLHSLMVP :  28
B.B.   : ------------------------------------------------------------ :   -
C.D.   : -------------------------------<SRAKINLSIDVLGKRQDGYHFVEMIMQT :  28
C.A.   : ---------------------------------<AKVNISLDVIGKREDGYHLLKMIMQS :  26
M.A.   : --------------------------------<PGKVNLYLAVGDRREDGYHELTTIFQA :  27
M.B.   : --------------------------------<PGKVNLYLAVGDRREDGYHELTTVFHA :  27
M.L.   : --------------------------------<PGKINLYLAVGDCCDNGYHELVTVFHA :  27
C.TP.  : ---------------------------------<AKINLGLLITSRRADGYHTLETIFAP :  26
P.G.   : ---------------------------------<AKINLGLQVVAKRADGYHNIETVFYP :  26
E.F.   : -------------------------------<APAKINLGLDVLHKRVDGYHEVESIFAS :  28
S.M.   : -------------------------------<APAKINLGLDIAGKYQDGFHELSMIMAS :  28
S.PG.  : ----------------------------------<KIKLGLDTKNKRXDGYHDLSMVMMS :  25
S.AN.  : -------------------------------<APAKINFTLDTLFKRNDGYHEIEMIMTT :  28
S.A.   : -------------------------------<APAKINFTLDTLFKRNDGYHEIEMIMTT :  28
A.T.   : KQVEIVFDPDERLNKIGDDVDKEAPLSRLKLFSPCKINVFLRITGKREDGFHDLASLFHV : 104
S.L.   : KQVEITYNPEEKFNKLADEVDREAGLSRLTLFSPCKINVFLRITSKRDDGYHDLASLFHV : 120
S.ML.  : ------------------------------------------------------------ :   -
C.C.   : -------------------------MRLSAFAPAKVNLFLHVGGPDGEGYHPISSLMVF :  34
C.J.   : ----------------------------<QMKAYAKANIFLKLTGFDSRKYHLLESRFIL :  31

*          140          *          160          *          180
E.C.   : LD-YGDTISIELRD--DGDIRLLTPVEGVE-HEDNLIVRAARLLMKTAADSGRLPTGSG- :  89
H.I.   : LD-FGDWLDISIRE-QDNQIVLTPEIPNLK-TENNLIYRAAKLLQEKAN------IQLG- : 103
B.S.   : ID-LADRIELTELA-EDEVRVS-SHNRFVPDDQRNLAYQAAKLIKDRYN------VKKG- :  84
S.S.   : IA-LGDKITVRANG-TDDIRLS-CGDSPLANDATNLAYRAAQLMINNFP------QAHDN :  86
M.T.   : VS-LVDEVTVRNAD-VLSLELVGEGADQLPTDERNLAWQAAELMAEHVG------RAPD- :  88
A.E.   : IP-FYDEIYIREG-----VLRVETNIG-IP-QEENLVYKGLREFERITG------IEIN- :  78
C.T.   : IA-FGDQLSLSIS----SRDSLQVINACHLETPSNSIWKSVALFRRYTG------ITTP- :  80
C.P.   : ID-FGDTLSLKNS----MKDSLSS-NVNELLSPSNLIWKSLEIFRRETQ------IHQP- :  79
T.M.   : IS-LHDTLTVEICD----RGFYLESSVALP--SDNTIKRAWEMFRKNTG------KEFG- :  87
H.P.   : VK-NKLKDIISVKN---ALSFSLKGDFDCP-LEENSLFKALQVLKKFLVQKNFSHSVIKS :  88
T.P.   : IT-LADSLSLSRLD-IPGRCEVCSPYMALP--RENTLTRAYARFCQVTG------VHDG- :  84
S.TL.  : LD-YGDTLHIEPRH--DGEIHLLTPVNGVE-NEDNLIVRAARLLMKVASESGRLPAGSG- :  89
Z.M.   : AK-DGDILQAEANDTEDNLTITGPFSEGLEANKDNLVLRAVTALRQACPN----KIPAG- :  89
S.T.   : LD-YGDTLHIEPRH--DGEIHLLTPVTGVE-NEDNLIVRAARLLMKVASESGRLPAGSG- :  89
S.P.   : LD-YGDTLHIEPRR--DGEIHLLTPVNGVE-NEDNLIVRAAQLLMKIASESGRLPAGSG- :  89
Y.S.   : LD-YGDQLTIEPRD--DNQIRLLTPIAGVE-NEQNLIVRAAKMLQKHP---GNTPVPRG- :  83
A.A.   : VD-FGDWLHIKVRP--DGKIRLTSVIADLK-AEDNLIYRAAKLLQQYTG------CTLG- :  80
V.C.   : LD-HGDELTITANN--SGNITLSPALADVA-LEDNLIYKAAMALKNAA-----Q-SPLG- :  81
S.PU.  : VD-CCDQLDFRVTD-TPELILHSTMSAVVA-DSDNLILRAAKSLQQATG------FNGG- :  80
P.M.   : VD-FGDWLDIEVRE--DNEICLTPELPSLK-NEDNLVYRAAKLLQQKTN------CALG- :  80
P.A.   : LD-HGDELHFEARQ--DGQVRLHTEIAGVP-HDSNLIVRAARGLQEASG------SPQG- :  83
N.G.   : ID-LQDTVYLKPRD--DGKIILHNPVDGMP-QEADLSYRAASLLQKYAR------TPTG- :  82
B.P.   : ID-LADTLHFEARA--DGAIGRAYELPGVA-ESDDLVMRAARSLQRATG------TRQG- :  78
N.M.   : ID-LQDTVYLKPRD--DGKIILHNPVDGMP-QEADLSYRAASLLQKYAR------TPTG- :  82
K.M.   : ------------------------------------------------------------ :   -
D.R.   : LT-VGDELEIRPAG---ALTLRVEGADLPT-DERNLVYRAARAYLDAAG------AAGG- :  76
B.B.   : ------TIYXKARX--DGIIARAXKLPGVP-ESTNLVVRAARSLQRATG------TAKG- :  44
C.D.   : ID-LYDIVKIKELD-EDEIKVK-STSLDIPLDEDNIVYKAAKILKNKFY------IKKG- :  78
C.A.   : IN-LYDVLDIRIID-EG-IKIT-SNRRNIPTNDKNIAYRAAKLFMDTYK------IDKG- :  75
M.A.   : VS-LLDEVTVRNAD-VLSLDIVGEGADKLPTDERNLAWQAAELMAEHVG------RAPD- :  78
M.B.   : VS-LVDEVTVRNAD-VLSLELVGEGADQLPTDERNLAWQAAELMAEHVG------RAPD- :  78
M.L.   : VS-LVDQVTVRNAD-VLSLGLVGEGANHVPTDEHNIAWRAAELMAEHVG------RAPD- :  78
C.TP.  : ID-WFDTLEFTESD---AISMECSNLDLLV-DDSNLCIRAAKALQEHTG------VKRG- :  74
P.G.   : IP-LTDALEIEVRE-DTCDRLSVHGVPIDAATEDNLVMKAVMALRRKFD-------FPP- :  76
E.F.   : VD-LADHLTFENLE-EDIIRIE-TDSSFLPVDRRNHVYQAVDLLKRTYN------IHKG- :  78
S.M.   : VD-LNDYLTITEIA-EDKIVVE-SNNCKLPLNRKNDVYKAAHLLKRRYH------ISTG- :  78
S.PG.  : ID-LCDYVTVDHID-DNKIVFA-SNCPKIPINXDNDVYKIVQLMKHRFQ------VKRG- :  75
S.AN.  : VD-LNDRLTFHKRK-DRKIVVE-IEHNYVPSNHKNLAYRAAQLFIEQYQ------LKQG- :  78
S.A.   : VD-LNDRLTFHKRK-DRKIVVE-IEHNYVPSNHKNLAYRAAQLFIEQYQ------LKQG- :  78
A.T.   : IS-LGDTIKFSLSP-SKSKDRLSTNVQGVPVDGRNLIIKALNLYRKKTG------SNRF- : 155
S.L.   : IS-LGDKIKFSLSP-SKSKDRLSTNVAGVPLDERNLIIKALNLYRKKTG------TDNY- : 171
S.ML.  : ------------------------------------------------------------ :   -
C.C.   : AD-VGDRVNLQPAD-APAFETSGPIGDQIPAGGDNLVVRAGQAFHRRLG------GPVPP :  86
C.J.   : LKDVFDELELVDKE---SDSKKEFEIISNFKCENNIIQKAYLLLSRRYNN-----ELKEL :  83

*          200          *          220          *          240
E.C.   : ---ANISIDKRLPMGGGLGGGSSNAATVLVALNHLWQC-GLSMDELAEMGLTLGADVPVF : 145
H.I.   : ---ANIHLDKILPMGGGVGGGSSNAATALVSLNYLWQA-NLSIDELAKLGLTLGADVPIF : 159
B.S.   : ---VSIMITKVIPVAAGLAGGSSDAAATLRGLNRLWNL-NLSAETLAELGAEIGSDVSFC : 140
S.S.   : YGGVDITLTKHIPMAAGLAGGSADAAAVLVGLDLLWNL-GLTRPELEQLAAQLGSDIPFC : 145
M.T.   : ---VSIMIDKSIPVAGGMAGGSADAAAVLVAMNSLWEL-NVPRRDLRMLAARLGSDVPFA : 144
A.E.   : ---YSIFIQKNIPPGAGLGGGSSNLAVVLKKVNELLGS-PLSEEELRELVGSISADAPFF : 134
C.T.   : ---VSWRVVKQIPVGAGLAGGSSNAATALFALNQIFKT-GLSDEEMRSLAEQLGVDTPFF : 136
C.P.   : ---VSWHLNKSIPLQSGLGGGSSNAATALYALNEHFQT-HIPITTLQLWAREIGSDVPFF : 135
T.M.   : ---LKVTLKKEIPVGSGLGGGSSNAAAVLRYLGEVFKI--P-LEDLLNIAAQVGSDVPFF : 141
H.P.   : LDTLAIEVEKNIPTQAGLGGGSADAGGLLYHLNQMFDW-RLSLKELYTMGSLVGADTNFF : 147
T.P.   : ---VRVRVVKRIPAGSGLGGGSADAAALLCGLDTLFGT-TLSARVLREVAYSVGSDVPFF : 140
S.TL.  : ---ADISIEKRLPMGGGLGGGSSNAATVLVALNHLWQC-GLSIDELATLGLTLGADVPVF : 145
Z.M.   : ---FSIILDKRLPVAAGIGGGSADAAAMLRMIGQHYQI--P-HELILSLANSLGADVPAC : 143
S.T.   : ---ADISIEKRLPMGGGLGGGSSNAATVLVALNHLWQC-GLSIDELATLGLTLGADVPVF : 145
S.P.   : ---ADISIEKRLPMGGGLGGGSSNAATVLVALNHLWQC-GLSIDELATLGLTLGADVPVF : 145
Y.S.   : ---ADISIDKCLPMGGGLGGGSSNAATVLVALNLLWQC-GLTDEQLADLGLTLGADVPVF : 139
A.A.   : ---TELTLDKILPIGGGVGGGSSNAATTLVALNHLWKT-GLSTGQLAELGLTLGADVPIF : 136
V.C.   : ---ADIQLHKVLPMGGGIGGGSSNAATTLVALNYLWQT-GLSDDQLAEIGLALGADVPVF : 137
S.PU.  : ---AEIWLDKRLPMGGGLGGGSSDAATTLVALNRLWNT-QLSHDELAAIGLKLGADIPVF : 136
P.M.   : ---ANLTLDKILPMGSGLGGGSSNAATALVALNYLWNT-QLSTKQLAKLGLMLGADVPIF : 136
P.A.   : ---VDIWLDKRLPMGGGIGGGSSDAATTLLALNHLWQL-GWDEDRIAALGLRLGADVPVF : 139
N.G.   : ---VEIWLDKKIPTGAGLGGGSSDAATVLLVLNRWWQC-GLTQRQLIDSGAALGADVPFF : 138
B.P.   : ---AQIGLHKRIPQGGGLGGGSSDAATTLIALNRLWGT-GLSRSQLMQLALPLGADVPVF : 134
N.M.   : ---VEIWLDKKIPTGAGLGGGSSDAATVLLVLNRWWQC-GLTQRQLIDSGAALGADVPFF : 138
K.M.   : ------------------------------------------------------------ : -
D.R.   : ---ADLVLHKRLPLASGLGGGSSDAASTLLALAELYPAPDHRPVDLPALALTLGADVPFF : 133
B.B.   : ---AQIACNKRIPQAFGLASGSRNAATTLIALTRLWGT-GLSRSQLMQLALPLGADVPVF : 100
C.D.   : ---VEIFIEKNIPVAAGMAGGSSNAAAVLVGXNHLWEL-RLSEDELKEIGLNLGADVPFC : 134
C.A.   : ---ISIHINKRIPVAAGLAGGSADGAAVLKAMRDIFKK-DVSDEELINLGVKIGADIPFC : 131
M.A.   : ---VSIMIDKSIPVAGGMAGGSADAAAVLVAMNSLWEL-NVPRRDLRMLAAQLGSDVPFA : 134
M.B.   : ---VSIMIDKSIPVAGGMAGGSADAAAVLVAMNSLWEL-NVPRRDLRMLAARLGSDVPFA : 134
M.L.   : ---VSIMIDKSIPVAGGMAGGSADAAAVLVAMNSLWEL-SLPRRDLCMLAAKLGSDVPFA : 134
C.TP.  : ---ATIKLLKRVPFGAGLGGGSSDAAATLNALCKLWQI-DVPSAELHKLAVKLGADVPYF : 130
P.G.   : ---LTIELIKHIPSGAGLGGGSSNASFMLKLVRDYFSL-PIDDEELAAIALTIGADCPFF : 132
E.F.   : ---IKIYIEKRIPVAAGLAGGSSDCAAALRGLNKLWNL-GLTMDELCEIGSQIGMDVPYC : 134
S.M.   : ---LKISLQKKIPICAGLGGGSSDAAATLRALNCLWKL-NLSPKELIDVGFEIGSDVPYC : 134
S.PG.  : ---VSVYLEKRIPMCAGMGGGSSD-AVTIRALNQMWLL-TLSRKDMMDIGIPIGSDVPYC : 130
S.AN.  : ---VTISIDKEIPVSAGLAGGSADAAATLRGLNRLFDI-GASLEELALLGSKIGTDIPFC : 134
S.A.   : ---VTISIDKEIPVSAGLAGGSADAAATLRGLNRLFDI-GASLEELALLGSKIGTDIPFC : 134
A.T.   : ---FWIHLDKKVPTGAGLGGGSSNAATALWAANELNGG-LVTENELQDWSSEIGSDIPFF : 211
S.L.   : ---FWIHLDKKVPTGAGLGGGSSNAATTLWAANQFSGC-VATEKELQEWSGEIGSDIPFF : 227
S.ML.  : ------<LEKNLPIASGMGGGSADAAATLRGLXSLWGA-TVEAASLNSPALQLGADVPMC : 52
C.C.   : ---YRLILEKHLPIAAGLGGGSSDAGAALKLMRDALAP-ALSDDDLEALAASLGADGAAC : 142
C.J.   : FSKKSLKLTKNIPVCAGLGGGSSDCASFLLLINETLNL-KLNLQELINLSIQLGSDIAFF : 142

*          260          *          280          *          300
E.C.  : VRG-HAAFAEGVGEILTPVDPPE--KWYLVAH-P-GVSIPT-------------PVIFKD : 187
H.I.  : VHG-HAAFAEGVGEKITYCEPAE--KWFVILK-P-DDSIST-------------AVIFQD : 201
B.S.  : VYG-GTALATGRGEKIKHISTPP-HCWVILAK-P-TIGVST-------------AEVYRA : 183
S.S.  : IGG-GTAIATGRGEILDPLPDGN-CFWVVLAK-HRSIEVSTPWAYQTYRQKFGKNYLNDD : 202
M.T.  : LHG-GTALGTGRGEELATVLSRN-TFHWVLAF-ADSGLLTS----------AVYNELDRL : 191
A.E.  : LLG-KSAIGRGKGEVLEPVETEISGKITLVIP---QVSSST-------------GRVYSS : 177
C.T.  : FST-GAALGVARGEKIIALEES-VSDRYVLYFSS-EGVLTS-------------RAFAAV : 180
C.P.  : FSS-GTALGKGRGEHLFSIKKLNHKHKYVLYLDH-QGIPTE-------------KAYQSL : 180
T.M.  : LYG-GTALVRGRGEIVEKLEDIE--GYSVDLFFP-GIHSST-------------KEMYLS : 184
H.P.  : ISQYKSANATSYGEVIENFEEES-LEDRLEIYAPNHVFCST-------------KAVYQA : 193
T.P.  : LAS-QAACVLGGGEQLVPLVPKT--GYLGLLVWP-GLHSGS-------------AQAYED : 183
S.TL. : VRG-HAAFAEGVGEILTPVNPPE--KWYLVAH-P-GVSIPT-------------PVIFKD : 187
Z.M.  : VDS-CLVRGEGVGEKLTQIGDRSLEEKPLLLVNP-RVSCST-------------PMIFKN : 188
S.T.  : VRG-HAAFAEGVGEILTPVNPPE--KWYLVAH-P-GVSIPT-------------PVIFKD : 187
S.P.  : VRG-HAAFAEGVGEILTPVNPPE--KWYLVAH-P-GVSIPT-------------PVIFKD : 187
Y.S.  : VRG-HAAFAEGIGEKLQPAEPVE--KWYLVIH-P-GVNIPT-------------PIIFSD : 181
A.A.  : VHG-KAAFAEGIGEKITYCEPPE--KWYLVLK-P-NVSIST-------------AVVFSD : 178
V.C.  : TRG-FAAFAEGVGEELSAVEPEE--KWYLVXXXP-AVSIAT-------------KDIFTH : 180
S.PU. : IHG-FAAFAQGVGERLQAVNPAE--LWYLVIA-P-DAHVST-------------AAVFQD : 178
P.M.  : VHG-HAAFAEGVGEKITYCEPKE--KWYVVLK-P-NVSIST-------------ATVFSD : 178
P.A.  : TRG-RAAFAEGVGEKLTPVDIPE--PWYLVVV-P-QVLVST-------------AEIFSD : 181
N.G.  : IFG-KNAFARGIGDRLDEMDIPK--QWYVIVK-P-PVHVST-------------AKIFTH : 180
B.P.  : VFG-QSAFAQGVGEDLTAVALSP--AAYLVVQ-P-DAGVPT-------------AVIFSD : 176
N.M.  : IFG-KNAFARGIGDRLDEMDIPK--QWYVIVK-P-PVHVST-------------AKIFTH : 180
K.M.  : ---------<GVGEILTPXKPEK--KWYLWPH-R-GSSIPT-------------PIIFRD : 33
D.R.  : LLG-GAALAEGVGERLTPVDDLP--PVHLVLANA-GAEV>------------------- : 168
B.B.  : VFG-QSAFAQGVGEDLTAVALPP--PAYLVVQ-P-DAGVPT-------------AAIFSD : 142
C.D.  : ISG-RPALAQGIGEKLTNIKGLPCDTNILICK-P-DLFVST-------------KEVYQG : 178
C.A.  : IVG-GTAFCEGIGEKITKLRSMN-GKIIVLVK-P-DFGVST-------------KMVYTE : 174
M.A.  : LHG-GTALGTGRGEELATV>--------------------------------------- : 152
M.B.  : LHG-GTALGTGRGEELATV>--------------------------------------- : 152
M.L.  : LHG-GTALGTGRGEELATV>--------------------------------------- : 152
C.TP. : LEMKGLAYAAGIGEELEDLNLAL--PWHVVTVFP-EVQVPT-------------AWAYKN : 174
P.G.  : VGN-RPVLATDLGQVFTPL>--------------------------------------- : 150
E.F.  : LRG-GTAFANGRGEKIEALPTMP-QCWIVLVK-P-RISVST-------------STVFND : 177
S.M.  : IEA-GCALISGKGEIVEPLATTL-STWVVLVK-P-DFGIST-------------KTIFKE : 177
S.PG. : LLS-GCAQVTGKGEVVCRILGLL-SSWVVLVK-P-DFGIST-------------XTFFLD : 173
S.AN. : IYN-KTALCTGRGEKIEFLNKPP-SAWVILAK-P-NLGISS-------------PDIFKL : 177
S.A.  : IYN-KTALCTGRGEKIEFLNKPP-SAWVILAK-P-NLGISS-------------PDIFKL : 177
A.T.  : FSH-GAAYCTGRGEIVQDLPPPFPLDLPMVLIKP-REACST-------------AEVYKR : 256
S.L.  : FSH-GAAYCTGRGEVVQDIPSPIPFDIPMVLIKP-QQACST-------------AEVYKR : 272
S.ML. : LDR-GPLVARGIGEEITPLPDLP--PXXVVLVNP-LVAVST-------------PVIFRS : 95
C.C.  : LRA-RA>---------------------------------------------------- : 147
C.J.  : LSGFHSANVSSCGEIIEEFEDDIP---NLKWTFP-QISCQT-------------KAVYDE : 185

*                320                *                340                *                360
E.C.   : PELPRNTPKRSIETLLKCEF------------------SNDCEVIARKRFREVDAVLSWL : 229
H.I.   : PNLPRNTPKKSLAQLLSEPY------------------KNDCEKVVINHYSNVEKALNWL : 243
B.S.   : LKLDGIE-HPDVQGMIEAIE------EKSFQK-MCSRLGNVLESVTLDMHPEVAMIKNQM : 235
S.S.   : QSQRARRKTIHAGPLLQGIQ------HRNPGQ-IASHIHNDLEKVVLPAHQPVAQLRQVL : 255
M.T.   : REVGDPPRLGEPGPVLAALA------AGDPDQ-LAPLLGNEMQAAAVSLDPALARALRAG : 244
A.E.   : LREEHFVTPEYAEEKIQRII------SG-----EVEEIENVLGDIARELYPEINEVYRFV : 226
C.T.   : QPSDCSS--RKNLEYTQ----------------------NDLEKPVFRLRLDLKEKKHWL : 216
C.P.   : LPQDYSTGNHNACFYGE----------------------NDLEKSVFRIRTDLKNKKHML : 218
T.M.   : LTPEMYRKGPGRVEELHRAY------LERNYEKIKELSYNVFEKVFLEKHPEVMDGLRNF : 238
H.P.   : YKPETCFFQAKEWLKKTSLEC---------LKTYDRNELNDLLKPALRTNPALKDIESQL : 244
T.P.   : LDRLRACGVHAADGEQYSLRGATALSAHYAQDCARWRFFNSLDAPVQRRYPVVALARWDL : 243
S.TL.  : PQLPRNTPKRSIDTLLKCEF------------------SNDCEVIARKRFREVDAALSWL : 229
Z.M.   : WDGVDRGALDSDGSILGAAR------SG----------RNDLEPPARKILPIIGEVVEWL : 232
S.T.   : PQLPRNTPKRSIDTLLKCEF------------------SNDCEVIARKRFREVDAALSWL : 229
S.P.   : PQLP-NTPKRSIDTLLKCEF------------------SNDCEVIARKRFREVDAALSWL : 228
Y.S.   : PELKRNTPIRPLAALLSTPY------------------ANDCEPIARKRFREVEQALSWL : 223
A.A.   : PHLPRNTPKKSLAQLLAGKY------------------ANDCEKVVRDHYSEVEESLNWL : 220
V.C.   : PQLMRNTPKRDLASLLTTPY------------------ENDCEKIVRSLYPEVDKQLSWL : 222
S.PU.  : PLLPRNTPKLGLDTLLSQPW------------------ANDCQELVVSKYPQVAKALGWL : 220
P.M.   : PDLIRNTPKQSLEQLLNQKY------------------ANDCEKVVLNHYPEVEEILHRL : 220
P.A.   : PLLTRDSPAIKVRTVLEGDS------------------RNDCQPVVERRYPEVRNALILL : 223
N.G.   : EGLTRNSASSIMPTFQNLQP------------------FRNDMQAVVFKEYPEVWKAYSEL : 223
B.P.   : PDLTRDCASVTIADFLALPT------SC---------FGRNDLEPVVLRRYPEVSGAVRWL : 222
N.M.   : EGLTRNSASSIMPTFQNLQP------------------FRNDMQAVVFKEYPEVWKAYSEL : 223
K.M.   : PELPRNTPRRSINTLLNCEF------------------SNDCELIARKRFREVDAALSWL : 75
D.R.   : ------------------------------------------------------------ : -
B.B.   : PDLTRDCASVTIADFLALPT------FC---------FGRNDLEPVVLRRYPEVSGAVRWL : 188
C.D.   : LDLNNIKKRPNNKYLIECLK------SEDIKA-VSESMVNILENVTIGKHKEISDIKQVM : 231
C.A.   : YDKCLDVKHPDSEGLVKAVN------NGHFKF-VVNNMVNVLENVTAVKYKEINEIKEKA : 227
M.A.   : ------------------------------------------------------------ : -
M.B.   : ------------------------------------------------------------ : -
M.L.   : ------------------------------------------------------------ : -
C.TP.  : FHRQFERPVPDLKTLVRRLC------HERDIS-VFGVFENDFASVVFEHYPVVREVRDAL : 227
P.G.   : ------------------------------------------------------------ : -
E.F.   : LAVDELH-HPDIAGLRIAIE------NGDYTG-MTQTVGNALESVTIARHPIVQQIKDRM : 229
S.M.   : IDMATIS-RVDIPALKEALL------ANYYED-ALQFMGNSLEDITIAKKPFIQKIKGRM : 229
S.PG.  : INCKIIS-RVSTTHLVAAIE------AGNYNDGILTEMNNLLEDIFIAKRPFIQKIKEKT : 226
S.AN.  : INLDKRY-DVHTKMCYEALE------NRDYQQ-LCQSLSNRLEPISVSKHPQIDKLKNNM : 229
S.A.   : INLDKRY-DVHTKMCYEALE------NRDYQQ-LCQSLSNRLEPISVSKHPQIDKLKNNM : 229
A.T.   : LRLDQTS-NINPLTLLENVT------SNGVSQ---SICVNDLEPPAFSVLPSLKRLKQRI : 306
S.L.   : FQLDLSS-KVDPLSLLEKIS------TSGISQ---DVCVNDLEPPAFEVLPSLKRLKQRV : 322
S.ML.  : LVRKTNPPLVLPEDARSTAE------WLT----AMAAMRNDLEPPARAHEPMIETVSNAL : 145
C.C.   : ------------------------------------------------------------ : -
C.J.   : ------------------------------------------------------------ : -

*                380                 *                400                 *                420
E.C.   : LEYAP-----SRLTGTGACVFA-------EFDTESEARQVLEQAPEWLNGFVAKGANLSP : 277
H.I.   : LQYAP-----ARLTGTGACVFA-------EFDHEAEAQAVFRQKPEAFFGFVAKGLNVSP : 291
B.S.   : KRFGAD---AVLMSGSGPTVFG-------LVQYESKVQRIYNGLRGFCDQVYAVRMIGEQ : 285
S.S.   : QSAGGL---GTMMSGSGPSVFT-------LCREQAEAEQVLAIAKEKLNDPDVDFWLTHT : 305
M.T.   : VEAGAL---AGIVSGSGPTCAF-------LCTSASSAIDVGAQLSGAGVCRTVRVATGPV : 294
A.E.   : EYLGFK----PFVSGSGSTVYFFGG----ASEELKKAAKMRGWKVVELEL>---------- : 268
C.T.   : ENLWAELPVHIGLTGSGATLF---VRYPEILEEDLSYAAQIQRAVTLSGGLLTSPIRRDP : 273
C.P.   : ERMWSPFESHVLMSGSGATLF---VCYLEELEQDSKVSSQIHSLIKQTQGIPVSRLYREP : 275
T.M.   : GDGSIV----KMMTGSGSVFFA-------LYPLDKGNYSFVGGV>--------------- : 271
H.P.   : SKEWF-------FSGSGSAFFR-------LKNTQKGANETHCQQ>--------------- : 274
T.P.   : ARAGAC---FTAMSGSGSXVFG-------LYRDEEELRRAHKLLAKRWCWCVRVRLCG>- : 291
S.TL.  : LEYAP-----SRLTGTGACVFA-------EFDTESCARQVLEQAPEWLNAFVAKGVNLSP : 277
Z.M.   : QQQKGVS--FSRMSGSGATCFA-------LFDEIEDRDTAYKKLNIDHPEWWALSSLLR> : 282
S.T.   : LEYAP-----SRLTGTGACVFA-------EFDTESCARQVLEQAPEWLNAFVAKGVNLSP : 277
S.P.   : LEYAP-----SRLTGTGACVFA-------EFDTESCARQVLEQAPEWLNAFVAKGVNLSP : 276
Y.S.   : LEYAP-----SRLTGTGACVFA-------EFDTESSARQVLSIAPEWLHGFVARGVNVSP : 271
A.A.   : VKYAP-----ARLTGTGACVFA-------EFDDKKSAQSVLQAKPKNCFGFVAKGLNHSP : 268
V.C.   : LQYAP-----SRLTGTGSCVFA-------EFSSRKDAQAVFAQLSDNVLAFVAQGRNVSP : 270
S.PU.  : LEYAP-----SRMTGTGACVFG-------EFSSQQQALAALAKLPSDMQGFVAKGMNISP : 268
P.M.   : LQYAP-----SRLTGTGACVFA-------EFNDEESAQLAFQTIPKNYFGFVAQGLNKSP : 268
P.A.   : NKFVS-----ARLTGTGGCVFG-------SFPNKAEADKVSALLPDHLQRFVAKGSNISM : 271
N.G.   : SRYGF-----ALMTGSGACVFT-------ACQDRNSAYNIYRQVSDLYEAYLAEGLSKHP : 271
B.P.   : FEHGLR----VRMSGSGACLFA-------EFPTLPEA>---------------------- : 248
N.M.   : SRYGF-----ALMTGSGACVFT-------ACQDRNSAYNIYRQVSDLYEAYLAEGLSKHP : 271
K.M.   : LEYAP-----SRLTGTGACVFA-------EFNTESAARQVLDTAPAWLNGFVARGVNLSP : 123
D.R.   : ------------------------------------------------------------ :   -
B.B.   : FEHGLR----VRMSGSGACLFA-------EFPTLPEAVLAQDP>---------------- : 220
C.D.   : MKNNAL---GSMMSGSGPTVFG-------LFKNKEDA>---------------------- : 258
C.A.   : LEYNSI---GTMMSGSGPTVFS-------FFDNTKEAEKYFYEMKKEYNKVFITR>---- : 272
M.A.   : ------------------------------------------------------------ :   -
M.B.   : ------------------------------------------------------------ :   -
M.L.   : ------------------------------------------------------------ :   -
C.TP.  : AASGAQ---FVSLSGSGSAVYA-------LYEGRADAVKAAE>----------------- : 259
P.G.   : ------------------------------------------------------------ :   -
E.F.   : LKYGAD---AALMSGSGPTVFA-------LCEKKTRAQRI>------------------- : 259
S.M.   : IKCGAD---IALMTGSGPTVFA-------LCRTEKRADRVV>------------------ : 260
S.PG.  : LQAGAA---NALMTGSGPTVFA-------LCQTEKQ>----------------------- : 252
S.AN.  : LKSGAD---GALMSGSGPTVYG-------LARKESQAKNI>------------------- : 259
S.A.   : LKSGAD---GALMNGSGPTVYG-------LARKESQAKNI>------------------- : 259
A.T.   : IASGRGEYDAVFMSGSGSTIIGIGSPDPPQFIYDDEEYKNVFLSEANFMTREANEWYKEP : 366
S.L.   : IAAGRGQYDAVFMSGSGSTIVGVGSPDPPQFVYDDEEYKDVFLSEASFITRPANEWYVEP : 382
S.ML.  : RDAGAA---LVRMSGSGATCFG-------LFTGMKSAERAAETISAGHPRW>-------- : 186
C.C.   : ------------------------------------------------------------ :   -
C.J.   : ------------------------------------------------------------ :   -

*          440
E.C.   : LHRAML>----------------- : 283
H.I.   : LHAMLKQLSSTHTHRQSKPEVL>- : 313
B.S.   : NALD>------------------- : 289
S.S.   : IGHGIQIMNN>------------- : 315
M.T.   : PGARVVSAPTEV>----------- : 306
A.E.   : ------------------------ :   -
C.T.   : TAWYSIY>---------------- : 280
C.P.   : -HWYSL>----------------- : 280
T.M.   : ------------------------ :   -
H.P.   : ------------------------ :   -
T.P.   : ------------------------ :   -
S.TL.  : LHRELL>----------------- : 283
Z.M.   : ------------------------ :   -
S.T.   : LHRELL>----------------- : 283
S.P.   : LHRELL>----------------- : 282
Y.S.   : LHR>-------------------- : 274
A.A.   : LHEML>------------------ : 273
V.C.   : LRKTL------------------- : 275
S.PU.  : L>---------------------- : 269
P.M.   : LHNML>------------------ : 273
P.A.   : LHRKL>------------------ : 276
N.G.   : L>---------------------- : 272
B.P.   : ------------------------ :   -
N.M.   : L>---------------------- : 272
K.M.   : LKQALL>----------------- : 129
D.R.   : ------------------------ :   -
B.B.   : ------------------------ :   -
C.D.   : ------------------------ :   -
C.A.   : ------------------------ :   -
M.A.   : ------------------------ :   -
M.B.   : ------------------------ :   -
M.L.   : ------------------------ :   -
C.TP.  : ------------------------ :   -
P.G.   : ------------------------ :   -
E.F.   : ------------------------ :   -
S.M.   : ------------------------ :   -
S.PG.  : ------------------------ :   -
S.AN.  : ------------------------ :   -
S.A.   : ------------------------ :   -
A.T.   : ASANATTSSAESRMDFQ>------ : 383
S.L.   : VSGSTIGDQPEFSTSFDMS>---- : 401
S.ML.  : ------------------------ :   -
C.C.   : ------------------------ :   -
C.J.   : ------------------------ :   -

### Annex C

Alignment of amino acid sequence of cloned cDNA of *ygbP* of *A. thaliana* (without leader sequence) and the amino acid sequence of the YgbP gene product found in the database

1 :                                                          :   -
2 : MAMLQTNLGFITSPTFLCPKLKVKLNSYLWFSYRSQGNFSYSLYTFKPMNLWFVQKL : 57

1 :                                          MEKSVSVILLAGGQGKRM : 18
2 : DFSKRVNRSYKRDALLLSIKCSSSTGFDNSNAVNSNVVVKEKSVSVILLAGGQGKRM : 114

1 : KMSMPKQYIPLLGQPIALYSFFTFSRMPEVKEIVVVCDPFFRDIFEEYEESIDVDLR : 75
2 : KLCF---------------------WLRDVPKI-SLSLFLFCGVL-EYEESIDVDLR : 148

1 : FAIPGKERQDSVYSGLQEIDVNSELVCIHDSARPLVNTEDVEKVLKDGSAVGAAVLG : 132
2 : FAIPGKERQDSVYSGLQEIDVNSELVCIHDSARPLVNTEDVEKVLKDGSAVGAAVLG : 205

1 : VPAKATIKEVNSDSLVVKTLDRKTLWEMQTPQVIKPELLKKGFELVKSEGLEVTDDV : 189
2 : VPAKATIKE-----------------------VIKPELLKKGFELVKSEGLEVTDDV : 239

1 : SIVEYLKHPVYVSQGSYTNIKVTTPDDLLLAERILSEDS : 228
2 : SIVEYLKHPVYVSQGSYTNIKVTTPDDLLLAERILSEDS : 278

### Annex Da

c DNA and protein sequence of ygbP from A. thaliana

10          20          30          40          50          60
|           |           |           |           |           |
GAGAAGAGTGTATCTGTGATTCTTTTAGCTGGAGGTCAAGGCAAGAGAATGAAAATGAGT

E  K  S  V  S  V  I  L  L  A  G  G  Q  G  K  R  M  K  M  S

70          80          90          100         110         120
|           |           |           |           |           |
ATGCCAAAGCAGTACATACCACTTCTTGGTCAGCCAATTGCTTTGTATAGCTTTTTCACG

M  P  K  Q  Y  I  P  L  L  G  Q  P  I  A  L  Y  S  F  F  T

130         140         150         160         170         180
|           |           |           |           |           |
TTTTCACGTATGCCTGAAGTGAAGGAAATTGTAGTTGTATGTGATCCTTTTTTCAGAGAC

F  S  R  M  P  E  V  K  E  I  V  V  V  C  D  P  F  F  R  D

190         200         210         220         230         240
|           |           |           |           |           |
ATTTTTGAAGAATACGAAGAATCAATTGATGTTGATCTTAGATTCGCTATTCCTGGCAAA

I  F  E  E  Y  E  E  S  I  D  V  D  L  R  F  A  I  P  G  K

250         260         270         280         290         300
|           |           |           |           |           |
GAAAGACAAGATTCTGTTTACAGTGGACTTCAGGAAATCGATGTGAACTCTGAGCTTGTT

E  R  Q  D  S  V  Y  S  G  L  Q  E  I  D  V  N  S  E  L  V

310         320         330         340         350         360
|           |           |           |           |           |
TGTATCCACGACTCTGCCCGACCATTGGTGAATACTGAAGATGTCGAGAAGGTCCTTAAA

C  I  H  D  S  A  R  P  L  V  N  T  E  D  V  E  K  V  L  K

370         380         390         400         410         420
|           |           |           |           |           |
GATGGTTCCGCGGTTGGAGCAGCTGTACTTGGTGTTCCTGCTAAAGCTACAATCAAAGAG

D  G  S  A  V  G  A  A  V  L  G  V  P  A  K  A  T  I  K  E

430         440         450         460         470         480
|           |           |           |           |           |
GTCAATTCTGATTCGCTTGTGGTGAAAACTCTCGACAGAAAAACCCTATGGGAAATGCAG

V  N  S  D  S  L  V  V  K  T  L  D  R  K  T  L  W  E  M  Q

490         500         510         520         530         540
|           |           |           |           |           |
ACACCACAGGTGATCAAACCAGAGCTATTGAAAAAGGGTTTCGAGCTTGTAAAAAGTGAA

T  P  Q  V  I  K  P  E  L  L  K  K  G  F  E  L  V  K  S  E

550 560 570 580 590 600
| | | | | |
GGTCTAGAGGTAACAGATGACGTTTCGATTGTTGAATACCTCAAGCATCCAGTTTATGTC

G L E V T D D V S I V E Y L K H P V Y V

610 620 630 640 650 660
| | | | | |
TCTCAAGGATCTTATACAAACATCAAGGTTACAACACCTGATGATTTACTGCTTGCTGAG

S Q G S Y T N I K V T T P D D L L L A E

670 680
| |
AGAATCTTGAGCGAGGACTCATGA

R I L S E D S -

### Annex-Db

cDNA sequence and corresponding amino acid sequence of the *ygbP* gene of A. *thaliana*

10        20        30        40        50        60        70
|         |         |         |         |         |         |
ATGGCGATGCTTCAGACGAATCTTGGCTTCATTACTTCTCCGACATTTCTGTGTCCGAAGCTTAAAGTCAAA
M  A  M  L  Q  T  N  L  G  F  I  T  S  P  T  F  L  C  P  K  L  K  V  K

80        90        100       110       120       130       140
|         |         |         |         |         |         |
TTGAACTCTTATCTGTGGTTTAGCTATCGTTCTCAAGTTCAAAAACTGGATTTTTCGAAAAGGGTTAATAGA
L  N  S  Y  L  W  F  S  Y  R  S  Q  V  Q  K  L  D  F  S  K  R  V  N  R

150       160       170       180       190       200       210
|         |         |         |         |         |         |
AGCTACAAAAGAGATGCTTTATTATTGTCAATCAAGTGTTCTTCATCGACTGGATTTGATAATAGCAATGTT
S  Y  K  R  D  A  L  L  L  S  I  K  C  S  S  S  T  G  F  D  N  S  N  V

220       230       240       250       260       270       280
|         |         |         |         |         |         |
GTTGTGAAGGAGAAGAGTGTATCTGTGATTCTTTTAGCTGGAGGTCAAGGCAAGAGAATGAAAATGAGTATG
V  V  K  E  K  S  V  S  V  I  L  L  A  G  G  Q  G  K  R  M  K  M  S  M

290       300       310       320       330       340       350       360
|         |         |         |         |         |         |         |
CCAAAGCAGTACATACCACTTCTTGGTCAGCCAATTGCTTTGTATAGCTTTTTCACGTTTTCACGTATGCCT
P  K  Q  Y  I  P  L  L  G  Q  P  I  A  L  Y  S  F  F  T  F  S  R  M  P

370       380       390       400       410       420       430
|         |         |         |         |         |         |
GAAGTGAAGGAAATTGTAGTTGTATGTGATCCTTTTTTCAGAGACATTTTTGAAGAATACGAAGAATCAATT
E  V  K  E  I  V  V  V  C  D  P  F  F  R  D  I  F  E  E  Y  E  E  S  I

440       450       460       470       480       490       500
|         |         |         |         |         |         |
GATGTTGATCTTAGATTCGCTATTCCTGGCAAAGAAAGACAAGATTCTGTTTACAGTGGACTTCAGGAAATC
D  V  D  L  R  F  A  I  P  G  K  E  R  Q  D  S  V  Y  S  G  L  Q  E  I

510       520       530       540       550       560       570
|         |         |         |         |         |         |
GATGTGAACTCTGAGCTTGTTTGTATCCACGACTCTGCCCGACCATTGGTGAATACTGAAGATGTCGAGAAG
D  V  N  S  E  L  V  C  I  H  D  S  A  R  P  L  V  N  T  E  D  V  E  K

580       590       600       610       620       630       640
|         |         |         |         |         |         |
GTCCTTAAAGATGGTTCCGCGGTTGGAGCAGCTGTACTTGGTGTTCCTGCTAAAGCTACAATCAAAGAGGTC
V  L  K  D  G  S  A  V  G  A  A  V  L  G  V  P  A  K  A  T  I  K  E  V

650       660       670       680       690       700       710       720
|         |         |         |         |         |         |         |
AATTCTGATTCGCTTGTGGTGAAAACTCTCGACAGAAAAACCCTATGGGAAATGCAGACACCACAGGTGATC
N  S  D  S  L  V  V  K  T  L  D  R  K  T  L  W  E  M  Q  T  P  Q  V  I

730       740       750       760       770       780       790
|         |         |         |         |         |         |
AAACCAGAGCTATTGAAAAAGGGTTTCGAGCTTGTAAAAAGTGAAGGTCTAGAGGTAACAGATGACGTTTCG
K  P  E  L  L  K  K  G  F  E  L  V  K  S  E  G  L  E  V  T  D  D  V  S

800       810       820       830       840       850       860
|         |         |         |         |         |         |
ATTGTTGAATACCTCAAGCATCCAGTTTATGTCTCTCAAGGATCTTATACAAACATCAAGGTTACAACACCT
I  V  E  Y  L  K  H  P  V  Y  V  S  Q  G  S  Y  T  N  I  K  V  T  T  P

870       880       890       900
|         |         |         |
GATGATTTACTGCTTGCTGAGAGAATCTTGAGCGAGGACTCATGA
D  D  L  L  L  A  E  R  I  L  S  E  D  S  -

### Annex E a

DNA sequence and corresponding protein sequence of *ychB* gene from A. *thaliana*

10        20        30        40        50        60
GCTCCTTTGTCCAGGCTTAAGCTCTTCTCACCTTGCAAGATCAATGTTTTCTTGAGGATC
A  P  L  S  R  L  K  L  F  S  P  C  K  I  N  V  F  L  R  I

70        80        90        100       110       120
ACCGGAAAGCGAGAAGATGGGTTTCATGATTTAGCCTCTTTGTTTCATGTGATTAGCTTA
T  G  K  R  E  D  G  F  H  D  L  A  S  L  F  H  V  I  S  L

130       140       150       160       170       180
GGAGGCACTATTAAATTCTCATTGTCACCATCAAAGTCTAAAGATCGTTTGTCTACTAAC
G  G  T  I  K  F  S  L  S  P  S  K  S  K  D  R  L  S  T  N

190       200       210       220       230       240
GTTCAAGGAGTCCCTGTTGATGGGAGAAATCTGATTATAAAAGCACTTAACCTTTACAGG
V  Q  G  V  P  V  D  G  R  N  L  I  I  K  A  L  N  L  Y  R

250       260       270       280       290       300
AAGAAAACTGGTAGCAACAGATTCTTCTGGATTCATTTAGATAAGAAGGTGCCTACCGGG
K  K  T  G  S  N  R  F  F  W  I  H  L  D  K  K  V  P  T  G

310       320       330       340       350       360
GCTGGACTCGGTGGTGGAAGTAGTAATGCTGCAACTGCACTCTGGGCGGCAAATGAGCTC
A  G  L  G  G  G  S  S  N  A  A  T  A  L  W  A  A  N  E  L

370       380       390       400       410       420
AATGGAGGTCTTGTCACTGAGAACGAACTCCAGGATTGGTCAAGTGAAATTGGGTCAGAT
N  G  G  L  V  T  E  N  E  L  Q  D  W  S  S  E  I  G  S  D

430       440       450       460       470       480
ATTCCTTTCTTCTTCTCGCATGGAGCTGCCTATTGTACCGGGAGAGGTGAGATTGTCCAA
I  P  F  F  F  S  H  G  A  A  Y  C  T  G  R  G  E  I  V  Q

490 500 510 520 530 540
GACCTTCCTCCACCTTTTCCTCTTGATCTTCCGATGGTGCTCATAAAGCCCCGAGAAGCA
D L P P P F P L D L P M V L I K P R E A

550 560 570 580 590 600
TGTTCCACTGCTGAAGTTTACAAACGTCTTCGTTTAGATCAGACGAGCAATATTAATCCC
C S T A E V Y K R L R L D Q T S N I N P

610 620 630 640 650 660
TTGACATTACTAAAGAATGTGACCAGCAATGGTGTGTCTCAAAGCATATGCGTAAACGAT
L T L L K N V T S N G V S Q S I C V N D

670 680 690 700 710 720
TTGGAACCGCCAGCGTTTTCAGTTCTTCCATCTCTAAAACGCTTGAAGCAACGGATAATA
L E P P A F S V L P S L K R L K Q R I I

730 740 750 760 770 780
GCATCTGGACGTGGGGAATACGATGCTGTGTTTATGTCTGGGAGTGGAAGCACTATTATC
A S G R G E Y D A V F M S G S G S T I I

790 800 810 820 830 840
GGTATTGGTTCACCAGATCCTCCTCAATTTATATATGATGATGAAGAATACAAGGACGTG
G I G S P D P P Q F I Y D D E E Y K D V

850 860 870 880 890 900
TTCTTGTCTGAAGCAAACTTTATGACGCGTGAGGCTAATGAATGGTACAAAGAACCTGCT
F L S E A N F M T R E A N E W Y K E P A

910 920 930 940 950
TCTGCAAATGCTACTACCTCATCCGCCGAATCTCGCATGGATTTCCAATGA
S A N A T T S S A E S R M D F Q STOP

### Annex Eb

cDNA sequence and corresponding amino acid sequence of the *ychB* gene of *A. thaliana*

10        20        30        40        50        60        70
|         |         |         |         |         |         |
ATGGCAACGGCTTCTCCTCCATTTATCTCAGCTCTCAGCTTCACTCACTCTTCTTTCAAAACTTCTTCTTCT
M  A  T  A  S  P  P  F  I  S  A  L  S  F  T  H  S  S  F  K  T  S  S  S

80        90        100       110       120       130       140
|         |         |         |         |         |         |
TCTTCATTTTCTCCGAAGCTTCTTCGACCCCTCTTAAGCTTTTCCGTCAAAGCTTCCAGAAAGCAAGTAGAG
S  S  F  S  P  K  L  L  R  P  L  L  S  F  S  V  K  A  S  R  K  Q  V  E

150       160       170       180       190       200       210
|         |         |         |         |         |         |
ATAGTGTTTGATCCTGATGAGAGGCTTAATAAGATAGGTGATGATGTTGACAAAGAAGCTCCTTTGTCCAGG
I  V  F  D  P  D  E  R  L  N  K  I  G  D  D  V  D  K  E  A  P  L  S  R

220       230       240       250       260       270       280
|         |         |         |         |         |         |
CTTAAGCTCTTCTCACCTTGCAAGATCAATGTTTTCTTGAGGATCACCGGAAAGCGAGAAGATGGGTTTCAT
L  K  L  F  S  P  C  K  I  N  V  F  L  R  I  T  G  K  R  E  D  G  F  H

290       300       310       320       330       340       350       360
|         |         |         |         |         |         |         |
GATTTAGCCTCTTTGTTTCATGTGATTAGCTTAGGAGGCACTATTAAATTCTCATTGTCACCATCAAAGTCT
D  L  A  S  L  F  H  V  I  S  L  G  G  T  I  K  F  S  L  S  P  S  K  S

370       380       390       400       410       420       430
|         |         |         |         |         |         |
AAAGATCGTTTGTCTACTAACGTTCAAGGAGTCCCTGTTGATGGGAGAAATCTGATTATAAAAGCACTTAAC
K  D  R  L  S  T  N  V  Q  G  V  P  V  D  G  R  N  L  I  I  K  A  L  N

440       450       460       470       480       490       500
|         |         |         |         |         |         |
CTTTACAGGAAGAAAACTGGTAGCAACAGATTCTTCTGGATTCATTTAGATAAGAAGGTGCCTACCGGGGCT
L  Y  R  K  K  T  G  S  N  R  F  F  W  I  H  L  D  K  K  V  P  T  G  A

510       520       530       540       550       560       570
|         |         |         |         |         |         |
GGACTCGGTGGTGGAAGTAGTAATGCTGCAACTGCACTCTGGGCGGCAAATGAGCTCAATGGAGGTCTTGTC
G  L  G  G  G  S  S  N  A  A  T  A  L  W  A  A  N  E  L  N  G  G  L  V

580       590       600       610       620       630       640
|         |         |         |         |         |         |
ACTGAGAACGAACTCCAGGATTGGTCAAGTGAAATTGGGTCAGATATTCCTTTCTTCTTCTCGCATGGAGCT
T  E  N  E  L  Q  D  W  S  S  E  I  G  S  D  I  P  F  F  F  S  H  G  A

650       660       670       680       690       700       710       720
|         |         |         |         |         |         |         |
GCCTATTGTACCGGGAGAGGTGAGATTGTCCAAGACCTTCCTCCACCTTTTCCTCTTGATCTTCCGATGGTG
A  Y  C  T  G  R  G  E  I  V  Q  D  L  P  P  P  F  P  L  D  L  P  M  V

730       740       750       760       770       780       790
|         |         |         |         |         |         |
CTCATAAAGCCCCGAGAAGCATGTTCCACTGCTGAAGTTTACAAACGTCTTCGTTTAGATCAGACGAGCAAT
L  I  K  P  R  E  A  C  S  T  A  E  V  Y  K  R  L  R  L  D  Q  T  S  N

800       810       820       830       840       850       860
|         |         |         |         |         |         |
ATTAATCCCTTGACATTACTAAAGAATGTGACCAGCAATGGTGTGTCTCAAAGCATATGCGTAAACGATTTG
I  N  P  L  T  L  L  K  N  V  T  S  N  G  V  S  Q  S  I  C  V  N  D  L

870       880       890       900       910       920       930
|         |         |         |         |         |         |
GAACCGCCAGCGTTTTCAGTTCTTCCATCTCTAAAACGCTTGAAGCAACGGATAATAGCATCTGGACGTGGG
E  P  P  A  F  S  V  L  P  S  L  K  R  L  K  Q  R  I  I  A  S  G  R  G

940 950 960 970 980 990 1000
GAATACGATGCTGTGTTTATGTCTGGGAGTGGAAGCACTATTATCGGTATTGGTTCACCAGATCCTCCTCAA
E Y D A V F M S G S G S T I I G I G S P D P P Q

1010 1020 1030 1040 1050 1060 1070 1080
TTTATATATGATGATGAAGAATACAAGGACGTGTTCTTGTCTGAAGCAAACTTTATGACGCGTGAGGCTAAT
F I Y D D E E Y K D V F L S E A N F M T R E A N

1090 1100 1110 1120 1130 1140 1150
GAATGGTACAAAGAACCTGCTTCTGCAAATGCTACTACCTCATCCGCCGAATCTCGCATGGATTTCCAATGA
E W Y K E P A S A N A T T S S A E S R M D F Q -

### Annex Ec

### ALIGNMENT OF TWO NUCLEOTIDE SEQUENCES

### TM_YCHBOL.

YCHB from *L. esculentum* U62773; wilde type cDNA; without putative leader sequence

### TMYBOLE1.

YCHB from *L. esculentum* sequence adapted to *E*. *coli* codon usage for highly expressed genes; without putative leader sequence

The character to show that two aligned residues are identical is '|'

TM_YCHBOL - GTGGATAGAGAAGCTGGGCTTTCAAGACTCACTCTTTTTCTCCTTGCAA -50
TMYBOLE1 - ATGGATCGTGAAGCTGGTCTTTCACGCCTCACTCTTTTTCTCCTTGCAA -50
TM-YCHB-13
TM_YCHBOL - GATAAATGTTTTCTTGAGAATAACAAGCAAGAGGGATGACGGATATCATG -100
TMYBOLE1 - GATTAATGTTTTCCTGCGCATCACAAGCAAACGTGATGACGGTTATCATG -100
TM-YCHB-11
TM_YCHBOL - ATTTGGCGTCTCTCTTTCATGTAATTAGTCTAGGAGATAAAATAAAGTTC -150
TMYBOLE1 - ATCTGGCGTCTCTCTTTCATGTAATTAGTCTTGGCGATAAAATTAAGTTC -150
TM-YCHB-9
TM_YCHBOL - TCGCTGTCACCATCGAAGTCAAAGGATCGTTTATCTACTAATGTTGCTGG -200
TMYBOLE1 - TCGCTGTCACCATCGAAATCAAAGGATCGTTTATCTACTAATGTTGCTGG -200
TM-YCHB-7
TM_YCHBOL - AGTTCCACTCGATGAGAGAAATCTGATTATAAAGGCCCTCAATCTTTATA -250
TMYBOLE1 - CGTTCCACTCGATGAGCGTAATCTGATTATCAAAGCCCTCAATCTTTATC -250
TM-YCHB-5
TM_YCHBOL - GGAAAAAGACTGGAACAGACAATTACTTTTGGATTCATCTTGATAAGAAA -300
TMYBOLE1 - GTAAAAAGACTGGTACAGACAATTACTTTTGGATTCATCTTGATAAGAAA -300
TM-YCHB-A
TM-YCHB-1
TM-YCHB-3
TM_YCHBOL - GTGCCTACTGGAGCTGGTCTTGGTGGTGGAGCAGTAATGCTGCAACAAC -350
TMYBOLE1 - GTGCCTACTGGAGCTGGTCTTGGTGGTGGAGCAGTAATGCTGCAACAAC -350
TM_YCHBOL - TCTGTGGGCAGCAAATCAATTCAGTGGTTGTGTTGCCACTGAAAAGGAGC -400

TMYBOLE1 - TCTGTGGGCAGCAAATCAATTCAGTGGTTGTGTTGCCACTGAAAAGGAGC -400

TM_YCHBOL - TCCAAGAGTGGTCTGGTGAGATTGGTTCTGATATTCCTTTCTTCTTCTCT -450
TMYBOLE1 - TCCAAGAGTGGTCTGGTGAGATTGGTTCTGATATTCCTTTCTTCTTCTCT -450

TM_YCHBOL - CATGGAGCAGCCTACTGTACGGGTAGGGGTGAGGTTGTTCAGGATATCCC -500
TMYBOLE1 - CATGGAGCAGCCTACTGTACGGGTAGGGGTGAGGTTGTTCAGGATATCCC -500

TM_YCHBOL - GTCACCCATACCATTTGACATTCCAATGGTCCTCATAAAGCCTCAACAGG -550
TMYBOLE1 - GTCACCCATACCATTTGACATTCCAATGGTCCTCATAAAGCCTCAACAGG -550

TM_YCHBOL - CATGCTCCACTGCTGAAGTTTACAAGCGTTTTCAGTTGGATCTGTCTAGT -600
TMYBOLE1 - CATGCTCCACTGCTGAAGTTTACAAGCGTTTTCAGTTGGATCTGTCTAGT -600

TM_YCHBOL - AAGGTTGATCCCTTGAGCTTACTGGAGAAAATCTCAACTAGTGGAATATC -650
TMYBOLE1 - AAGGTTGATCCCTTGAGCTTACTGGAGAAAATCTCAACTAGTGGAATATC -650

TM_YCHBOL - TCAAGATGTGTGTGTCAATGATTTAGAACCTCCTGCCTTTGAAGTTCTTC -700
TMYBOLE1 - TCAAGATGTGTGTGTCAATGATTTAGAACCTCCTGCCTTTGAAGTTCTTC -700
TM-YCHB-B
TM-YCHB-2

TM_YCHBOL - CATCTCTTAAAAGGTTAAAACAACGAGTAATTGCTGCTGGCCGAGGACAA -750
TMYBOLE1 - CATCTCTTAAACGTTTAAAACAACGTGTAATTGCTGCTGGCCGCGGTCAA -750
TM-YCHB-4

TM_YCHBOL - TATGATGCAGTCTTCATGTCTGGAAGTGGAAGCACAATAGTAGGGGTTGG -800
TMYBOLE1 - TATGATGCAGTCTTCATGTCTGGTAGTGGCAGCACAATCGTAGGTGTTGG -800
TM-YCHB-6

TM_YCHBOL - CTCTCCAGATCCACCACAATTTGTCTATGATGATGAAGAATACAAGGATG -850
TMYBOLE1 - CTCTCCAGATCCGCCACAATTTGTCTATGATGACGAAGAGTACAAAGATG -850
TM-YCHB-8 TM-YCHB-10

TM_YCHBOL - TCTTCTTGTCAGAAGCAAGTTTCATCACTCGACCAGCCAACGAGTGGTAT -900
TMYBOLE1 - TCTTCTTGTCAGAAGCAAGTTTCATCACTCGTCCAGCCAACGAGTGGTAT -900
TM-YCHB-12

TM_YCHBOL - GTTGAACCTGTTTCAGGTAGCACTATTGGTGATCAACCTGAGTTCTCTAC -950
TMYBOLE1 - GTTGAACCTGTTTCAGGTAGCACTATTGGTGATCAACCTGAGTTCTCTAC -950
TM-YCHB-14

TM_YCHBOL - ATCTTTTGACATGTCTTAA -969
TMYBOLE1 - ATCTTTTGACATGTCTTAA -969

Identity : 927 (95.67%)

### Annex F1

Nucleotide sequence of the plasmid pNCO 113

ID PNCO113 PRELIMINARY; DNA; 3420 BP.
DE EXPRESSIONSPLASMID PNCOII3 (AUS PDS56-RII,NCO)
OS ESCHERICHIA COLI
SQ SEQUENCE 3420 BP; 888 A; 816 C; 828 G; 888 T; 0 OTHER;
CTCGAGAAAT CATAAAAAAT TTATTTGCTT TGTGAGCGGA TAACAATTAT AATAGATTCA
ATTGTGAGCG GATAACAATT TCACACAGAA TTCATTAAAG AGGAGAAATT AACCATGGGA
GGATCCGTCG ACCTGCAGCC AAGCTTAATT AGCTGAGCTT GGACTCCTGT TGATAGATCC
AGTAATGACC TCAGAACTCC ATCTGGATTT GTTCAGAACG CTCGGTTGCC GCCGGGCGTT
TTTTATTGGT GAGAATCCAA GCTAGCTTGG CGAGATTTTC AGGAGCTAAG GAAGCTAAAA
TGGAGAAAAA AATCACTGGA TATACCACCG TTGATATATC CCAATGGCAT CGTAAAGAAC
ATTTTGAGGC ATTTCAGTCA GTTGCTCAAT GTACCTATAA CCAGACCGTT CAGCTGGATA
TTACGGCCTT TTTAAAGACC GTAAAGAAAA ATAAGCACAA GTTTTATCCG GCCTTTATTC
ACATTCTTGC CCGCCTGATG AATGCTCATC CGGAATTTCG TATGGCAATG AAAGACGGTG
AGCTGGTGAT ATGGGATAGT GTTCACCCTT GTTACACCGT TTTCCATGAG CAAACTGAAA
CGTTTTCATC GCTCTGGAGT GAATACCACG ACGATTTCCG GCAGTTTCTA CACATATATT
CGCAAGATGT GGCGTGTTAC GGTGAAAACC TGGCCTATTT CCCTAAAGGG TTTATTGAGA
ATATGTTTTT CGTCTCAGCC AATCCCTGGG TGAGTTTCAC CAGTTTTGAT TTAAACGTGG
CCAATATGGA CAACTTCTTC GCCCCCGTTT TCACCATGCA TGGGCAAATA TTATACGCAA
GGCGACAAGG TGCTGATGCC GCTGGCGATT CAGGTTCATC ATGCCGTCTG TGATGGCTTC
CATGTCGGCA GAATGCTTAA TGAATTACAA CAGTACTGCG ATGAGTGGCA GGGCGGGGCG
TAATTTTTTT AAGGCAGTTA TTGGTGCCCT TAAACGCCTG GGGTAATGAC TCTCTAGCTT
GAGGCATCAA ATAAAACGAA AGGCTCAGTC GAAAGACTGG GCCTTTCGTT TTATCTGTTG
TTTGTCGGTG AACGCTCTCC TGAGTAGGAC AAATCCGCCG CTCTAGAGCT GCCTCGCGCG
TTTCGGTGAT GACGGTGAAA ACCTCTGACA CATGCAGCTC CCGGAGACGG TCACAGCTTG
TCTGTAAGCG GATGCCGGGA GCAGACAAGC CCGTCAGGGC GCGTCAGCGG GTGTTGGCGG
GTGTCGGGGC GCAGCCATGA CCCAGTCACG TAGCGATAGC GGAGTGTATA CTGGCTTAAC
TATGCGGCAT CAGAGCAGAT TGTACTGAGA GTGCACCATA TGCGGTGTGA AATACCGCAC
AGATGCGTAA GGAGAAAATA CCGCATCAGG CGCTCTTCCG CTTCCTCGCT CACTGACTCG
CTGCGCTCGG TCTGTCGGCT GCGGCGAGCG GTATCAGCTC ACTCAAAGGC GGTAATACGG
TTATCCACAG AATCAGGGGA TAACGCAGGA AAGAACATGT GAGCAAAAGG CCAGCAAAAG
GCCAGGAACC GTAAAAAGGC CGCGTTGCTG GCGTTTTTCC ATAGGCTCCG CCCCCCTGAC
GAGCATCACA AAAATCGACG CTCAAGTCAG AGGTGGCGAA ACCCGACAGG ACTATAAAGA
TACCAGGCGT TTCCCCCTGG AAGCTCCCTC GTGCGCTCTC CTGTTCCGAC CCTGCCGCTT
ACCGGATACC TGTCCGCCTT TCTCCCTTCG GGAAGCGTGG CGCTTTCTCA ATGCTCACGC
TGTAGGTATC TCAGTTCGGT GTAGGTCGTT CGCTCCAAGC TGGGCTGTGT GCACGAACCC
CCCGTTCAGC CCGACCGCTG CGCCTTATCC GGTAACTATC GTCTTGAGTC CAACCCGGTA
AGACACGACT TATCGCCACT GGCAGCAGCC ACTGGTAACA GGATTAGCAG AGCGAGGTAT
GTAGGCGGTG CTACAGAGTT CTTGAAGTGG TGGCCTAACT ACGGCTACAC TAGAAGGACA
GTATTTGGTA TCTGCGCTCT GCTGAAGCCA GTTACCTTCG GAAAAAGAGT TGGTAGCTCT
TGATCCGGCA AACAAACCAC CGCTGGTAGC GGTGGTTTTT TTGTTTGCAA GCAGCAGATT
ACGCGCAGAA AAAAAGGATC TCAAGAAGAT CCTTTGATCT TTTCTACGGG GTCTGACGCT
CAGTGGAACG AAAACTCACG TTAAGGGATT TTGGTCATGA GATTATCAAA AAGGATCTTC
ACCTAGATCC TTTTAAATTA AAAATGAAGT TTTAAATCAA TCTAAAGTAT ATATGAGTAA
ACTTGGTCTG ACAGTTACCA ATGCTTAATC AGTGAGGCAC CTATCTCAGC GATCTGTCTA
TTTCGTTCAT CCATAGCTGC CTGACTCCCC GTCGTGTAGA TAACTACGAT ACGGGAGGGC
TTACCATCTG GCCCCAGTGC TGCAATGATA CCGCGAGACC CACGCTCACC GGCTCCAGAT
TTATCAGCAA TAAACCAGCC AGCCGGAAGG GCCGAGCGCA GAAGTGGTCC TGCAACTTTA
TCCGCCTCCA TCCAGTCTAT TAATTGTTGC CGGGAAGCTA GAGTAAGTAG TTCGCCAGTT
AATAGTTTGC GCAACGTTGT TGCCATTGCT ACAGGCATCG TGGTGTCACG CTCGTCGTTT
GGTATGGCTT CATTCAGCTC CGGTTCCCAA CGATCAAGGC GAGTTACATG ATCCCCCATG
TTGTGCAAAA AAGCGGTTAG CTCCTTCGGT CCTCCGATCG TTGTCAGAAG TAAGTTGGCC
GCAGTGTTAT CACTCATGGT TATGGCAGCA CTGCATAATT CTCTTACTGT CATGCCATCC

GTAAGATGCT TTTCTGTGAC TGGTGAGTAC TCAACCAAGT CATTCTGAGA ATAGTGTATG
CGGCGACCGA GTTGCTCTTG CCCGGCGTCA ATACGGGATA ATACCGCGCC ACATAGCAGA
ACTTTAAAAG TGCTCATCAT TGGAAAACGT TCTTCGGGGC GAAAACTCTC AAGGATCTTA
CCGCTGTTGA GATCCAGTTC GATGTAACCC ACTCGTGCAC CCAACTGATC TTCAGCATCT
TTTACTTTCA CCAGCGTTTC TGGGTGAGCA AAAACAGGAA GGCAAAATGC CGCAAAAAAG
GGAATAAGGG CGACACGGAA ATGTTGAATA CTCATACTCT TCCTTTTTCA ATATTATTGA
AGCATTTATC AGGGTTATTG TCTCATGAGC GGATACATAT TTGAATGTAT TTAGAAAAAT
AAACAAATAG GGGTTCCGCG CACATTTCCC CGAAAAGTGC CACCTGACGT CTAAGAAACC
ATTATTATCA TGACATTAAC CTATAAAAAT AGGCGTATCA CGAGGCCCTT TCGTCTTCAC
//

### Annex F2

Nucleotide sequence of the plasmid pNCO-SB-H6-ACYC184

ID PNCOSBH6ACYC PRELIMINARY; DNA; 4484 BP.
SQ SEQUENCE 4484 BP; 1131 A; 1127 C; 1069 G; 1157 T;
CTCGAGAAAT CATAAAAAAT TTATTTGCTT TGTGAGCGGA TAACAATTAT AATAGATTCA
ATTGTGAGCG GATAACAATT TCACACAGAA TTCATTAAAG AGGAGAAATT AACCATGCAC
CACCACCACC ACCACGCGTC CATGGCCGCG GCAAAGCCGT TTTTCCATAG GCTCCGCCCC
CCTGACAAGC ATCACGAAAT CTGACGCTCA AATCAGTGGT GGCGAAACCC GACAGGACTA
TAAAGATACC AGGCGTTTCC CCCTGGCGGC TCCCTCGTGC GCTCTCCTGT TCCTGCCTTT
CGGTTTACCG GTGTCATTCC GCTGTTATGG CCGCGTTTGT CTCATTCCAC GCCTGACACT
CAGTTCCGGG TAGGCAGTTC GCTCCAAGCT GGACTGTATG CACGAACCCC CCGTTCAGTC
CGACCGCTGC GCCTTATCCG GTAACTATCG TCTTGAGTCC AACCCGGAAA GACATGCAAA
AGCACCACTG GCAGCAGCCA CTGGTAATTG ATTTAGAGGA GTTAGTCTTG AAGTCATGCG
CCGGTTAAGG CTAAACTGAA AGGACAAGTT TTGGTGACTG CGCTCCTCCA AGCCAGTTAC
CTCGGTTCAA AGAGTTGGTA GCTCAGAGAA CCTTCGAAAA ACCGCCCTGC AAGGCGGTTT
TTTCGTTTTC AGAGCAAGAG ATTACGCGCA GACCAAAACG ATCTCAAGAA GATCATCTTA
TTAATCAGAT AAAATATTTC TAGATTTCAG TGCAATTTAT CTCTTCAAAT GTAGCACCTG
AAGTCAGCCC CATACGATAT AAGTTGTAAT TCTCATGTTT GACAGCTTAT CATCGATAAG
CTTTAATGCG GTAGTTTATC ACAGTTAAAT TGCTAACGCA GTCAGGCACC GTGTATGAAA
TCTAACAATG CGCTCATCGT CATCCTCGGC ACCGTCACCC TGGATGCTGT AGGCATAGGC
TTGGTTATGC CGGTACTGCC GGGCCTCTTG CGGGATATCG TCCATTCCGA CAGCATCGCC
AGTCACTATG GCGTGCTGCT AGCGCTATAT GCGTTGATGC AATTTCTATG CGCACCCGTT
CTCGGAGCAC TGTCCGACCG CTTTGGCCGC CGCCCAGTCC TGCTCGCTTC GCTACTTGGA
GCCACTATCG ACTACGCGAT CATGGCGACC ACACCCGTCC TGTGGGATCC GTCGACCTGC
AGCCAAGCTT AATTAGCTGA GCTTGGACTC CTGTTGATAG ATCCAGTAAT GACCTCAGAA
CTCCATCTGG ATTTGTTCAG AACGCTCGGT TGCCGCCGGG CGTTTTTTAT TGGTGAGAAT
CCAAGCTAGC TTGGCGAGAT TTTCAGGAGC TAAGGAAGCT AAAATGGAGA AAAAAATCAC
TGGATATACC ACCGTTGATA TATCCCAATG GCATCGTAAA GAACATTTTG AGGCATTTCA
GTCAGTTGCT CAATGTACCT ATAACCAGAC CGTTCAGCTG GATATTACGG CCTTTTTAAA
GACCGTAAAG AAAAATAAGC ACAAGTTTTA TCCGGCCTTT ATTCACATTC TTGCCCGCCT
GATGAATGCT CATCCGGAAT TTCGTATGGC AATGAAAGAC GGTGAGCTGG TGATATGGGA
TAGTGTTCAC CCTTGTTACA CCGTTTTCCA TGAGCAAACT GAAACGTTTT CATCGCTCTG
GAGTGAATAC CACGACGATT TCCGGCAGTT TCTACACATA TATTCGCAAG ATGTGGCGTG
TTACGGTGAA AACCTGGCCT ATTTCCCTAA AGGGTTTATT GAGAATATGT TTTTCGTCTC
AGCCAATCCC TGGGTGAGTT TCACCAGTTT TGATTTAAAC GTGGCCAATA TGGACAACTT
CTTCGCCCCC GTTTTCACCA TGCATGGGCA AATATTATAC GCAAGGCGAC AAGGTGCTGA
TGCCGCTGGC GATTCAGGTT CATCATGCCG TCTGTGATGG CTTCCATGTC GGCAGAATGC
TTAATGAATT ACAACAGTAC TGCGATGAGT GGCAGGGCGG GGCGTAATTT TTTTAAGGCA
GTTATTGGTG CCCTTAAACG CCTGGGGTAA TGACTCTCTA GCTTGAGGCA TCAAATAAAA
CGAAAGGCTC AGTCGAAAGA CTGGGCCTTT CGTTTTATCT GTTGTTTGTC GGTGAACGCT
CTCCTGAGTA GGACAAATCC GCCGCTCTAG AGCTGCCTCG CGCGTTTCGG TGATGACGGT
GAAAACCTCT GACACATGCA GCTCCCGGAG ACGGTCACAG CTTGTCTGTA AGCGGATGCC
GGGAGCAGAC AAGCCCGTCA GGGCGCGTCA GCGGGTGTTG GCGGGTGTCG GGGCGCAGCC
ATGACCCAGT CACGTAGCGA TAGCGGAGTG TATACTGGCT TAACTATGCG GCATCAGAGC
AGATTGTACT GAGAGTGCAC CATATGCGGT GTGAAATACC GCACAGATGC GTAAGGAGAA
AATACCGCAT CAGGCGCTCT TCCGCTTCCT CGCTCACTGA CTCGCTGCGC TCGGTCTGTC
GGCTGCGGCG AGCGGTATCA GCTCACTCAA AGGCGGTAAT ACGGTTATCC ACAGAATCAG
GGGATAACGC AGGAAAGAAC ATGTGAGCAA AAGGCCAGCA AAAGGCCAGG AACCGTAAAA
AGGCCGCGTT GCTGGCGTTT TTCCATAGGC TCCGCCCCCC TGACGAGCAT CACAAAAATC
GACGCTCAAG TCAGAGGTGG CGAAACCCGA CAGGACTATA AAGATACCAG GCGTTTCCCC
CTGGAAGCTC CCTCGTGCGC TCTCCTGTTC CGACCCTGCC GCTTACCGGA TACCTGTCCG
CCTTTCTCCC TTCGGGAAGC GTGGCGCTTT CTCAATGCTC ACGCTGTAGG TATCTCAGTT
CGGTGTAGGT CGTTCGCTCC AAGCTGGGCT GTGTGCACGA ACCCCCCGTT CAGCCCGACC
GCTGCGCCTT ATCCGGTAAC TATCGTCTTG AGTCCAACCC GGTAAGACAC GACTTATCGC
CACTGGCAGC AGCCACTGGT AACAGGATTA GCAGAGCGAG GTATGTAGGC GGTGCTACAG
AGTTCTTGAA GTGGTGGCCT AACTACGGCT ACACTAGAAG GACAGTATTT GGTATCTGCG
CTCTGCTGAA GCCAGTTACC TTCGGAAAAA GAGTTGGTAG CTCTTGATCC GGCAAACAAA
CCACCGCTGG TAGCGGTGGT TTTTTTGTTT GCAAGCAGCA GATTACGCGC AGAAAAAAAG
GATCTCAAGA AGATCCTTTG ATCTTTTCTA CGGGGTCTGA CGCTCAGTGG AACGAAAACT
CACGTTAAGG GATTTTGGTC ATGAGATTAT CAAAAAGGAT CTTCACCTAG ATCCTTTTAA
ATTAAAAATG AAGTTTTAAA TCAATCTAAA GTATATATGA GTAAACTTGG TCTGACAGTT
ACCAATGCTT AATCAGTGAG GCACCTATCT CAGCGATCTG TCTATTTCGT TCATCCATAG
CTGCCTGACT CCCCGTCGTG TAGATAACTA CGATACGGGA GGGCTTACCA TCTGGCCCCA
GTGCTGCAAT GATACCGCGA GACCCACGCT CACCGGCTCC AGATTTATCA GCAATAAACC
AGCCAGCCGG AAGGGCCGAG CGCAGAAGTG GTCCTGCAAC TTTATCCGCC TCCATCCAGT

CTATTAATTG TTGCCGGGAA GCTAGAGTAA GTAGTTCGCC AGTTAATAGT TTGCGCAACG
TTGTTGCCAT TGCTACAGGC ATCGTGGTGT CACGCTCGTC GTTTGGTATG GCTTCATTCA
GCTCCGGTTC CCAACGATCA AGGCGAGTTA CATGATCCCC CATGTTGTGC AAAAAAGCGG
TTAGCTCCTT CGGTCCTCCG ATCGTTGTCA GAAGTAAGTT GGCCGCAGTG TTATCACTCA
TGGTTATGGC AGCACTGCAT AATTCTCTTA CTGTCATGCC ATCCGTAAGA TGCTTTTCTG
TGACTGGTGA GTACTCAACC AAGTCATTCT GAGAATAGTG TATGCGGCGA CCGAGTTGCT
CTTGCCCGGC GTCAATACGG GATAATACCG CGCCACATAG CAGAACTTTA AAAGTGCTCA
TCATTGGAAA ACGTTCTTCG GGGCGAAAAC TCTCAAGGAT CTTACCGCTG TTGAGATCCA
GTTCGATGTA ACCCACTCGT GCACCCAACT GATCTTCAGC ATCTTTTACT TTCACCAGCG
TTTCTGGGTG AGCAAAAACA GGAAGGCAAA ATGCCGCAAA AAAGGGAATA AGGGCGACAC
GGAAATGTTG AATACTCATA CTCTTCCTTT TTCAATATTA TTGAAGCATT TATCAGGGTT
ATTGTCTCAT GAGCGGATAC ATATTTGAAT GTATTTAGAA AAATAAACAA ATAGGGGTTC
CGCGCACATT TCCCCGAAAA GTGCCACCTG ACGTCTAAGA AACCATTATT ATCATGACAT
TAACCTATAA AAATAGGCGT ATCACGAGGC CCTTTCGTCT TCAC
//

### Annex G

cDNA sequence and corresponding amino acid sequence of the *ygbB* gene of P. *falciparum*

10        20        30        40        50        60        70
|         |         |         |         |         |         |
ATGTTTTTAAAAGGATACACCTCAAATGTGGTACTAATTATATTAACATTTTTCATTCTACTAACAAAAGAA
M  F  L  K  G  Y  T  S  N  V  V  L  I  I  L  T  F  F  I  L  L  T  K  E

80        90        100       110       120       130       140
|         |         |         |         |         |         |
GAAAAAAATATAAAAAATAATATCTCTGGATATTGTTTTTTGAATTTTGGATTAAAAAAAAATGCAATAATA
E  K  N  I  K  N  N  I  S  G  Y  C  F  L  N  F  G  L  K  K  N  A  I  I

150       160       170       180       190       200       210
|         |         |         |         |         |         |
AAAAAAAGAGAAAAACAAAATTTGAAATTATTTGTTATAATGGTATAAGAATAGGTCAAGGTTATGATATC
K  K  R  E  K  Q  N  L  K  L  F  C  Y  N  G  I  R  I  G  Q  G  Y  D  I

220       230       240       250       260       270       280
|         |         |         |         |         |         |
CACAAAATAAAAGTTTTAGATGAAGAATATAATACATATGCAAATAATGATTTTAATAAAAATGAACAATCT
H  K  I  K  V  L  D  E  E  Y  N  T  Y  A  N  N  D  F  N  K  N  E  Q  S

290       300       310       320       330       340       350       360
|         |         |         |         |         |         |         |
TTTAAAACCTTAACCTTAGGAGGAGTTAAAATAAATAATGTTTTAGTTTTATCACATAGTGATGGTGATATA
F  K  T  L  T  L  G  G  V  K  I  N  N  V  L  V  L  S  H  S  D  G  D  I

370       380       390       400       410       420       430
|         |         |         |         |         |         |
ATATATCATTCGATAGTTGATTCAATTTTAGGTGCCTTAGGTTCTTTAGACATAGGAACCTTATTTCCTGAT
I  Y  H  S  I  V  D  S  I  L  G  A  L  G  S  L  D  I  G  T  L  F  P  D

440       450       460       470       480       490       500
|         |         |         |         |         |         |
AAAGATGAAAAAAATAAAAATAAAAACTCGGCTATATTCTTAAGATATGCTAGACTTTTAATATATAAAAAA
K  D  E  K  N  K  N  K  N  S  A  I  F  L  R  Y  A  R  L  L  I  Y  K  K

510       520       530       540       550       560       570
|         |         |         |         |         |         |
AATTATGATATTGGGAACGTGGATATTAATGTAATAGCACAAGTTCCCAAAATAAGCAACATCAGAAAAAAT
N  Y  D  I  G  N  V  D  I  N  V  I  A  Q  V  P  K  I  S  N  I  R  K  N

580       590       600       610       620       630       640
|         |         |         |         |         |         |
ATTATAAAAAATATATCGACAGTGTTAAATATTGACGAGTCGCAAATATCTGTTAAAGGAAAAACTCATGAG
I  I  K  N  I  S  T  V  L  N  I  D  E  S  Q  I  S  V  K  G  K  T  H  E

650       660       670       680       690       700       710       720
|         |         |         |         |         |         |         |
AAATTAGGAGTAATTGGTGAGAAAAAAGCAATAGAATGCTTTGCGAATATTTTGTTAATACCTAAAAATTCA
K  L  G  V  I  G  E  K  K  A  I  E  C  F  A  N  I  L  L  I  P  K  N  S

730       740       750
|         |         |
TAATTTCTTTTTTTTTTTTTTTTAATGTAA
-  F  L  F  F  F  F  L  M  -

## Claims

1. A protein, in a form that is enzymatically functional for the conversion of cytidine triphosphate and 2C-methyl-D-erythritol 4-phosphate into 4-diphosphocytidyl 2C-methyl-D-erythritol in the presence of magnesium ions.

2. The protein according to claim 1, wherein it has a sequence selected from the set of sequences coded by *ygbP* in the *E.coli* genome or the set of functional homologs thereof.

3. A protein in a form that is enzymatically functional for the conversion of 4-diphosphocytidyl-2C-methyl-D-erythritol in the presence of manganese ions, said protein may have a sequence selected from the set of sequences coded by *ygbB* in the *E.coli* genome or the set of functional homologs thereof.

4. A protein in a form that is enzymatically functional for the conversion of 4-diphosphocytidyl-2C-methyl-D-erythritol and adenosine triphosphate into 4-diphosphocytidyl-2C-methyl-D-erythritol 2-phosphate in the presence of a magnesium salt.

5. A protein according to claim 4, **characterized in that** it has a sequence selected from the set of sequences coded by *YchB* in the *E. coli* genome or the set of functional homologs thereof.

6. A protein in a form that is enzymatically functional for the conversion of 4-disphosphocytidyl-2C-methyl-D-erythritol 2-phosphate into 2C-methyl-D-erythritol 2,4-cyclopyrophosphate and CMP.

7. The protein according to claim 6, **characterized in that** is has a sequence selected from the set of sequences coded by the geneYgbB in the *E.coli* genome or by genes orthologous to *YgbB.*

8. A protein in a form that is enzymatically bifunctional, having an N-terminal domain with the function of synthesis of diphosphocytidyl-2C-methyl-D-erythritol from cytidine triphosphate and 2C-methyl-D-erythritol 4-phosphate and a C-terminal domain with the function of the conversion of 4-diphosphocytidyl-2C-methyl-D-erythritol.

9. The protein according to one of claims 1 to 7 whereby it is a plant enzyme.

10. The protein according to claim 9, whereby it is an *Arabidopsis thaliana* enzyme or a *Lycopersicum esculentum* enzyme.

11. The protein according to one of claims 1 to 8, whereby it is a bacterial enzyme.

12. The protein according to one of claims 1 to 7, whereby it is a protozoal enzyme.

13. The protein according to one of claims 11 or 12, whereby it is a protein of an organism selected from *Escherichia coli, Haemophilus, Bacillus subtilis, Synechocystis, Mycobacterium, Aquifex aeolicus, Chlamydia, Thermotoga maritima, Helicobacter, Treponema pallidum, Borellia burgdorfferi, Salmonella, Yersinia, Actinobacillus, Vibrio cholerae, Shewanella putrefaciens, Pasteurella multocida, Pseudomonas aeruginosa, Neisseria, Bordetella, Thiobacillus ferrooxidans, Deinococcus radiodurans, Clostridium acetobutylicum, Chlorobium tepidum, Porphyromonas gingivalis, Enterococccus faecalis, Streptococcus, Staphylococcus aureus, Rhodobacter capsulatus, Caulobacter crescentus, Campylobacter jejunis, Plasmodium falciparum.*
